# EUROPEAN PATENT SPECIFICATION

(11) **EP 3 083 587 B1**
(45) Date of publication and mention of the grant of the patent: **04.04.2018**
(21) Application number: 14859348.6
(22) Date of filing: 15.12.2014
(51) Int. Cl.: C07D 401/12, C07D 249/04, C07D 249/08, C07D 471/04, C07D 257/04, C07D 263/32, C07D 271/06, C07D 271/10, C07D 285/12, C07D 307/12, A61K 31/41, A61P 3/06

(54) **OXIMINO DERIVATIVES FOR THE TREATMENT OF DYSLIPIDEMIA**
OXIMINODERIVATE ZUR BEHANDLUNG VON DYSLIPIDÄMIE
DÉRIVÉS D'OXIMINO POUR LE TRAITEMENT DE LA DYSLIPIDÉMIE

(30) Priority: 16.12.2013 IN 3933MU2013
(43) Date of publication of application: 26.10.2016
(73) Proprietor: Cadila Healthcare Limited, Ahmedabad, Gujarat 380015 (IN)
(72) Inventor: DESAI, Ranjit C., Ahmedabad - 380 015 Gujarat (IN); PINGALI, Harikishore, Ahmedabad - 380 015 Gujarat (IN)
(74) Representative: HGF Limited
(86) International application number: PCT/IN2014/000775
(87) International publication number: WO 2015/107541

(56) References cited:
- WO-A1-99/58510
- WO-A1-2007/039555
- WO-A1-2014/002105
- WO-A1-2014/192023
- WO-A2-2008/035359
- IMOTO H ET AL: "STUDIES ON NON-THIAZOLIDINEDIONE ANTIDIABETIC AGENTS. 1. DISCOVERY OF NOVEL OXYIMINOACETIC ACID DERIVATIVES", CHEMICAL AND PHARMACEUTICAL BULLETIN, PHARMACEUTICAL SOCIETY OF JAPAN, JP, vol. 50, no. 10, 1 October 2002 (2002-10-01), pages 1349-1357, XP001181424, ISSN: 0009-2363, DOI: 10.1248/CPB.50.1349

## Description

### FIELD OF INVENTION

The present invention relates to compounds of the general formula (I), their tautomeric forms, their stereoisomers, their pharmaceutically acceptable salts, pharmaceutical compositions containing them, methods for their preparation, use of these compounds in medicine and the intermediates involved in their preparation.

The present invention is directed towards compounds which can be used to treat diseases such as hyperlipidemia and also have a beneficial effect on cholesterol.

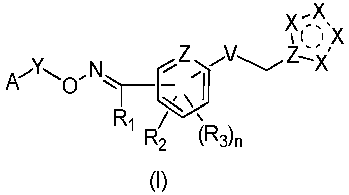

The compounds of the general formula (I) lower blood glucose, lower or modulate triglyceride levels and/or cholesterol levels and/or low-density lipoproteins (LDL) and raises the high-density lipoproteins (HDL) plasma levels and hence are useful in combating different medical conditions, where such lowering of LDL (and/or raising of HDL) is beneficial. Thus, it could be used in the treatment and/or prophylaxis of obesity, hyperlipidemia, hypercholesteremia, hypertension, atherosclerotic disease events, vascular restenosis, diabetes and many other related conditions.

The compounds of general formula (I) are useful to prevent or reduce the risk of developing atherosclerosis, which leads to diseases and conditions such as arteriosclerotic cardiovascular diseases, stroke, coronary heart diseases, cerebrovascular diseases, peripheral vessel diseases and related disorders.

These compounds of general formula (I) are useful for the treatment and/or prophylaxis of metabolic disorders loosely defined as Syndrome X. The characteristic features of Syndrome X include initial insulin resistance followed by hyperinsulinemia, dyslipidemia and impaired glucose tolerance. The glucose intolerance can lead to non-insulin dependent diabetes mellitus (NIDDM, Type 2 diabetes), which is characterized by hyperglycemia, which if not controlled may lead to diabetic complications or metabolic disorders caused by insulin resistance. Diabetes is no longer considered to be associated only with glucose metabolism, but it affects anatomical and physiological parameters, the intensity of which vary depending upon stages/duration and severity of the diabetic state. The compounds of this invention are also useful in prevention, halting or slowing progression or reducing the risk of the above mentioned disorders along with the resulting secondary diseases such as cardiovascular diseases, like arteriosclerosis, atherosclerosis; diabetic retinopathy, diabetic neuropathy and renal disease including diabetic nephropathy, glomerulonephritis, glomerular sclerosis, nephrotic syndrome, hypertensive nephrosclerosis and end stage renal diseases, like microalbuminuria and albuminuria, which may be result of hyperglycemia or hyperinsulinemia.

The compounds of the present invention can be useful as aldose reductase inhibitors; for improving cognitive functions in dementia, and in the treatment and/or prophylaxis of disorders such as psoriasis, polycystic ovarian syndrome (PCOS), cancer, osteoporosis, leptin resistance, inflammation and inflammatory bowel diseases, wound healing, xanthoma, pancreatitis, myotonic dystrophy, endothelial cell dysfunction and hyperlipidemia.

### BACKGROUND OF THE INVENTION

Higher LDL cholesterol levels in the plasma increase cardiovascular risk and reduction in the levels of LDL would decrease CVD risk by a comparable percentage (PNAS, 2009, 106, 9546-9547). Clearance of LDL cholesterol from plasma is through the action of LDL receptors in the liver and LDL receptors are cell surface glycoproteins that bind to apoliporpotein B100 (apoB100) on LDL particles with high affinity and mediate their endocytic uptake (Journal of Biological Chemistry, 2009, 284, 10561-10570). Defect in hepatic cholesterol clearance and elevated levels of plasma LDL cholesterol that result from the mutations cause familial hypercholesterolemia. Such mutations are identified in the human LDL receptor and later in apolipoprotein-B (Nature Structural and Molecular Biology, 2007, 14, 413-419). Recently, mutations within certain subtypes of the pro-protein convertase subtilisin/ gene such as the subtype nine (hereinafter "the gene") were found to represent a third class of mutations associated with autosomal dominant hypercholesterolemia (ADH). The discovery, etiology and functions of this subtype gene is discussed in details in Nature Genetics, 2003, 34, 154-156, Trends in Biochemical Sciences, 2008, 33, 426-434 etc.. Several missense mutations (S127R, D129G, F216L, D374H, D374Y) are associated with hypercholesterolemia and premature atherosclerosis (J Lipid Res. 2008, 49, 1333-1343). Loss-of-function mutations (R46L, L253F, A433T) lead to elevated receptor abundance, enhancing clearance of LDL cholesterol from the circulation and reducing cardiovascular risk (Nature Structural and Molecular Biology, 2007, 14, 413-419).

Detailed molecular mechanisms explaining the association of LDLR and the particular subtype gene and LDLR degradation is not very clear (Drug News Perspectives, 2008, 21, 323-330). Because of inhibition of LDLR recycling, number of LDL receptors on the cell surface are decreased and this increases plasma LDL levels (PNAS, 2009, 106, 9546-9547).

Various approaches for inhibiting this particular subtype gene are reported, including gene silencing by siRNA or antisense oligonucleotides, mAb disrupting protein-protein interactions or by peptides; all the above-mentioned strategies have shown lowering of LDL cholesterol which may be effective therapy for treating hypercholesterolemia (Biochemical Journal, 2009, 419, 577-584; PNAS, 2008, 105, 11915-11920; Journal of Lipid Research, 2007, 48, 763-767; PNAS, 2009, 106, 9820-9825). However, very little success has been reported in trying to inhibit this subtype gene by using small molecules. Such small molecule inhibitors have their obvious clinical and therapeutic benefits over the other known approaches as discussed above. We herein disclose novel small molecules which have shown to inhibit this particular gene in in-vitro studies and therefore provide an alternate beneficial approach for treating patients in need of such therapy.

WO2014/192023 relates to compounds used in the treatment of hyperlipidemia; WO2014/002105 relates to compounds used in the treatment of diseases such as hyperlipidemia; WO99/58510 relates to an oxyiminoalkanoic acid derivative which has hypoglycemic and hypolipidemic action; WO2008/035359 relates to oximinophenoxyalkanoic acid and phenylalkanoic acid; WO2007/039555 relates to oxime ether compounds and their use and Imoto H et al, Chemical and Pharmaceutical bulletin, Pharmaceutical Society of Japan, JP, vol. 50, no.10, 1 October 2002, pages 1349 to 1357 relates to studies of non-thiazolidenedione antidiabetic agents.

### SUMMARY OF THE INVENTION

According to a first aspect of the present invention there is provided a compound according to claim 1.

According to a second aspect of the present invention there is provided a pharmaceutical composition according to claim 3.

According to a third aspect of the present invention there is provided a compound or pharmaceutical composition for use according to claim 4.

According to a fourth aspect of the present invention there is provided a pharmaceutical composition for use according to claim 6.

The main objective of the present invention is to provide novel substituted oximino derivatives represented by the general formula (I), their tautomeric forms, their stereoisomers, their pharmaceutically acceptable salts, and pharmaceutical compositions containing them or their mixtures thereof.

Herein is described a process for the preparation of novel substituted oximino derivatives represented by the general formula (I), their tautomeric forms, their stereoisomers, their pharmaceutically acceptable salts.

Herein is described a pharmaceutical compositions containing compounds of the general formula (I), their tautomeric forms, their stereoisomers, their pharmaceutically acceptable salts, or their mixtures in combination with suitable carriers, solvents, diluents and other media normally employed in preparing such compositions.

Herein is described a process for treatment of diseases such as dyslipidemia, hyperlipidemia etc. by providing therapeutically effective amount of the compounds of formula (I) or their pharmaceutically acceptable salts or their suitable pharmaceutical compositions.

The above and other embodiments are described in details hereinafter.

### DETAILED DESCRIPTION OF THE INVENTION

Herein is described compounds of the general formula (I), 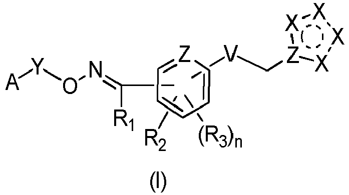 their tautomeric forms, their stereoisomers, their pharmaceutically acceptable salts, and pharmaceutical compositions containing them wherein
'A' represents an optionally substituted single or fused group selected from aryl, heterocyclyl or cycloalkyl groups;
In a preferred embodiment, 'A' is selected from optionally substituted aryl or heterocyclyl groups;
In a further preferred embodiment, when 'A' represents an aryl group, the aryl group may be selected from substituted or unsubstituted monocyclic or bicyclic aromatic groups;
In a still further preferred embodiment, the aryl group is an optionally substituted phenyl group.
In an alternate embodiment, when 'A' represents a heterocyclyl group, the heterocyclyl group may be selected from single or fused mono, bi or tricyclic aromatic or non-aromatic groups containing one or more hetero atoms selected from O, N or S;
In one preferred embodiment, 'A' represents a heteroaromatic group containing one or more hetero atoms selected from O, N or S;
In another preferred embodiment, 'A' represents a non-aromatic heterocyclic group containing one or more hetero atoms selected from O, N or S;
   In a preferred embodiment, the heterocyclyl group may be selected from pyridyl, thienyl, furyl, pyrrolyl, oxazolyl, thiazolyl, isothiazolyl, imidazolyl, isoxazolyl, oxadiazolyl, thiadiazolyl, triazolyl, tetrazolyl, benzofuranyl, benzothienyl, indolinyl, indolyl, azaindolyl, azaindolinyl, pyrazolopyrimidinyl, azaquinazolinyl, pyridofuranyl, pyridothienyl, thienopyrimidyl, quinolinyl, pyrimidinyl, pyrazolyl, quinazolinyl, pyridazinyl, triazinyl, benzimidazolyl, benzotriazolyl, phthalazynil, naphthylidinyl, purinyl, carbazolyl, phenothiazinyl, phenoxazinyl, benzoxazolyl, benzothiazolyl, thiazepinyl, oxazolidinyl, thiazolidinyl, dihydrothiophene, dihydropyran, dihydrofuran, dihydrothiazole, benzopyranyl, benzopyranonyl, benzodihydrofuranyl, benzodihydrothienyl, pyrazolopyrimidonyl, azaquinazolinoyl, thienopyrimidonyl, quinazolonyl, pyrimidonyl, benzoxazinyl, benzoxazinonyl, benzothiazinyl, benzothiazinonyl, thieno piperidinyl and the like;
'Y' represents either a bond or substituted or unsubstituted linear or branched (C₁-C₆)alkyl, (C₂-C₆)alkenyl groups or the groups represented by '-U(CH₂)ₘ-' wherein U represents O, S(O)ₒ, NR₄; 'm' represents integers from 2 to 4, 'o' represents integers from 0 to 2 and R₄ represents H, substituted or unsubstituted linear or branched (C₁₋C₆)alkyl;
'V' represents either a bond, or may be selected from O, S(O)ₒ, NR₄ or SO₂NR₄; wherein R₄ is as defined earlier;
'Z' at each occurrence independently represents C or N;
'X' at each occurrence independently represents represents C, N, O or S;
R₁ represents hydrogen, optionally substituted, (C₁-C₆)alkyl, (C₃-C₆)cycloalkyl, alkoxyalkyl, hydroxyalkyl, aminoalkyl, aryl, heterocyclyl, aralkyl, heterocyclylalkyl groups;
R₂ represents hydrogen, or the groups selected from (C₁-C₆)alkyl, aryl, heterocyclyl, aralkyl, heterocyclylalkyl, (C₁-C₆)alkoxy, hydroxy, hydroxyalkyl, thio(C₁-C₆)alkyl, amino, aminoalkyl, alkylamino, each of which may be optionally substituted;
R₃ at each occurrence independently represents hydrogen, halogen, (C₁-C₃)alkyl, halo(C₁-C₃)alkyl, (C₁-C₃)alkoxy, thio(C₁₃-C₃)alkyl, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, sulfenyl derivatives, sulfonyl derivatives;
'n' represents integers from 0-3;
Alternatively R₁ and R₂ wherever possible, together may form 4 to 7 membered saturated or partially saturated ring containing from 0-2 additional heteroatoms selected from the group consisting of N, O, and S(O)ₒ;
When A, R₁ or X are substituted, the substituents at each occurrence may be independently selected from hydroxyl, oxo, halo, thiol, nitro, amino, cyano, formyl, or substituted or unsubstituted groups selected from amidino, alkyl, haloalkyl, perhaloalkyl, alkoxy, haloalkoxy, perhaloalkoxy, alkenyl, alkynyl, cycloalkyl, cycloalkenyl, bicycloalkyl, bicycloalkenyl, alkoxy, alkenoxy, cycloalkoxy, aryl, aryloxy, aralkyl, aralkoxy, heterocylyl, heterocyclylalkyl, heterocycloxy, heterocyclylalkoxy, heterocyclylalkoxyacyl, acyl, acyloxy, acylamino, monosubstituted or disubstituted amino, arylamino, aralkylamino, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, cycloalkylthio, arylthio, heterocyclylthio, alkylsulfinyl, cycloalkylsulfinyl, arylsulfinyl, heterocyclylsulfinyl, alkylsulfonyl, cycloalkylsulfonyl, arylsulfonyl, heterocyclylsulfonyl, alkylsulfonylamino, cycloalkylsulfonylamino, arylsulfonylamino, heterocyclylsulfonylamino, alkylsulfonyloxy, cycloalkylsulfonyloxy, arylsulfonyloxy, heterocyclylsulfonyloxy, alkoxycarbonylamino, aryloxycarbonylamino, aralkyloxycarbonylamino, aminocarbonylamino, alkylaminocarbonylamino, alkoxyamino, hydroxyl amino, sulfinyl derivatives, sulfonyl derivatives, sulfonic acid and its derivatives.

When any of R₂, R₃ or R₄ are substituted the substituents at each occurrence may be independently selected from hydroxyl, oxo, halo, thiol, nitro, amino, cyano, acyl, formyl, or substituted or unsubstituted groups selected from amidino, alkyl, haloalkyl, perhaloalkyl, alkoxy, haloalkoxy, perhaloalkoxy, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, alkylsulfinyl, cycloalkylsulfinyl, alkylsulfonyl, cycloalkylsulfonyl, arylsulfonyl, alkylsulfonylamino, alkylsulfonyloxy, alkoxycarbonylamino, aminocarbonylamino, alkylaminocarbonylamino, alkoxyamino, hydroxyl amino, sulfinyl derivatives, sulfonyl derivatives, sulfonic acid and its derivatives.

When the substituents on any of A, R₁, R₂, R₃, R₄ or X are further substituted, the substituents may be selected from one or more groups described above.
The various groups, radicals and substituents used anywhere in the specification are described in the following paragraphs.
In a further preferred embodiment the groups, radicals described above may be selected from:
- the "alkyl" group used either alone or in combination with other radicals, denotes a linear or branched radical containing one to six carbons, selected from methyl, ethyl, n-propyl, *iso*-propyl, *n*-butyl, *sec*-butyl, *tert*-butyl, amyl, *t*-amyl, *n*-pentyl, *n*-hexyl, and the like;
- the "alkenyl" group used either alone or in combination with other radicals, is selected from a radical containing from two to six carbons, more preferably groups selected from vinyl, allyl, 2-butenyl, 3-butenyl, 2-pentenyl, 3-pentenyl, 4-pentenyl, 2-hexenyl, 3-hexenyl, 4-hexenyl and the like; the "alkenyl" group includes dienes and trienes of straight and branched chains;
- the "cycloalkyl", or "alicyclic" group used either alone or in combination with other radicals, is selected from a cyclic radical containing three to six carbons, more preferably cyclopropyl, cyclobutyl, cyclopentyl, cyclohexyl and the like;
- the "cycloalkenyl" group used either alone or in combination with other radicals, are preferably selected from cyclopropenyl, 1-cyclobutenyl, 2-cylobutenyl, 1-cyclopentenyl, 2-cyclopentenyl, 3-cyclopentenyl, 1-cyclohexenyl, 2-cyclohexenyl, 3-cyclohexenyl and the like; The terms "bicycloalkenyl" means more than one cycloalkenyl groups fused together;
- the "alkoxy" group used either alone or in combination with other radicals, is selected from groups containing an alkyl radical, as defined above, attached directly to an oxygen atom, more preferably groups selected from methoxy, ethoxy, n-propoxy, *iso-*propoxy, *n*-butoxy, *t*-butoxy, *iso*-butoxy, pentyloxy, hexyloxy, and the like;
- the "cycloalkoxy" group used either alone or in combination with other radicals, is selected from a cyclic radical containing three to seven carbons, more preferably cyclopropyloxy, cyclobutylxoy, cyclopentyloxy, cyclohexyloxy and the like; The terms "bicycloalkyloxy" means more than one cycloalkyl groups fused together;
- the "alkenoxy" group used either alone or in combination with other radicals, is selected from groups containing an alkenyl radical, as defined above, attached to an oxygen atom, more preferably selected from vinyloxy, allyloxy, butenoxy, pentenoxy, hexenoxy, and the like;
- the "haloalkyl" group is selected from an alkyl radical, as defined above, suitably substituted with one or more halogens; such as perhaloalkyl, more preferably, perfluoro(C₁-C₆)alkyl such as fluoromethyl, difluoromethyl, trifluoromethyl, fluoroethyl, difluoroethyl, trifluoroethyl, mono or polyhalo substituted methyl, ethyl, propyl, butyl, pentyl or hexyl groups;
- the "haloalkoxy" group is selected from suitable haloalkyl, as defined above, directly attached to an oxygen atom, more preferably groups selected from fluoromethoxy, chloromethoxy, fluoroethoxy, chloroethoxy and the like;
- the "aryl" or "aromatic" group used either alone or in combination with other radicals, is selected from a suitable aromatic system containing one, two or three rings wherein such rings may be attached together in a pendant manner or may be fused, more preferably the groups are selected from phenyl, naphthyl, tetrahydronaphthyl, indane, biphenyl, and the like;
- the "aryloxy" group used either alone or in combination with other radicals, is selected from groups containing an aryl radical, as defined above, attached directly to an oxygen atom, more preferably groups selected from phenoxy, naphthyloxy, tetrahydronaphthyloxy, biphenyloxy, and the like;
- the "heterocyclyl" or "heterocyclic" group used either alone or in combination with other radicals, is selected from suitable aromatic or non-aromatic radicals containing one or more hetero atoms selected from O, N or S. The non-aromatic radicals may be saturated, partially saturated or unsaturated mono, bi or tricyclic radicals, containing one or more heteroatoms selected from nitrogen, sulfur and oxygen, more preferably selected from aziridinyl, azetidinyl, pyrrolidinyl, imidazolidinyl, piperidinyl, piperazinyl, 2-oxopiperidinyl, 4-oxopiperidinyl, 2-oxopiperazinyl, 3-oxopiperazinyl, morpholinyl, thiomorpholinyl, 2-oxomorpholinyl, azepinyl, diazepinyl, oxapinyl, thiazepinyl, oxazolidinyl, thiazolidinyl, dihydrothiophene, dihydropyran, dihydrofuran, dihydrothiazole, benzopyranyl, benzopyranonyl, benzodihydrofuranyl, benzodihydrothienyl, pyrazolopyrimidonyl, azaquinazolinoyl, thienopyrimidonyl, quinazolonyl, pyrimidonyl, benzoxazinyl, benzoxazinonyl, benzothiazinyl, benzothiazinonyl, thieno piperidinyl, and the like; the aromatic radicals, may be selected from suitable single or fused mono, bi or tricyclic aromatic heterocyclic radicals containing one or more hetero atoms selected from O, N or S, more preferably the groups are selected from pyridyl, thienyl, furyl, pyrrolyl, oxazolyl, thiazolyl, isothiazolyl, imidazolyl, isoxazolyl, oxadiazolyl, thiadiazolyl, triazolyl, tetrazolyl, benzofuranyl, benzothienyl, indolinyl, indolyl, azaindolyl, azaindolinyl, pyrazolopyrimidinyl, azaquinazolinyl, pyridofuranyl, pyridothienyl, thienopyrimidyl, quinolinyl, pyrimidinyl, pyrazolyl, quinazolinyl, pyridazinyl, triazinyl, benzimidazolyl, benzotriazolyl, phthalazynil, naphthylidinyl, purinyl, carbazolyl, phenothiazinyl, phenoxazinyl, benzoxazolyl, benzothiazolyl and the like;
- the groups "heterocycloxy", "heterocylylalkoxy" are selected from suitable heterocyclyl, heterocylylalkyl groups respectively, as defined above, attached to an oxygen atom;
- the "acyl" group used either alone or in combination with other radicals, is selected from a radical containing one to eight carbons, more preferably selected from formyl, acetyl, propanoyl, butanoyl, *iso*-butanoyl, pentanoyl, hexanoyl, heptanoyl, benzoyl and the like, which may be substituted;
- the "acyloxy" group used either alone or in combination with other radicals, is selected from a suitable acyl group, as defined above, directly attached to an oxygen atom, more preferably such groups are selected from acetyloxy, propionyloxy, butanoyloxy, *iso-*butanoyloxy, benzoyloxy and the like;
- the "acylamino" group used either alone or in combination with other radicals, is selected from a suitable acyl group as defined earlier, attached to an amino radical, more preferably such groups are selected from CH₃CONH, C₂H₅CONH, C₃H₇CONH, C₄H₉CONH, C₆H₅CONH and the like, which may be substituted;
- the "mono-substituted amino" group used either alone or in combination with other radicals, represents an amino group substituted with one group selected from (C₁-C₆)alkyl, substituted alkyl, aryl, substituted aryl or arylalkyl groups as defined earlier, more preferably such groups are selected from methylamine, ethylamine, n-propylamine, n-butylamine, n-pentylamine and the like;
- the 'disubstituted amino" group used either alone or in combination with other radicals, represents an amino group, substituted with two radicals that may be same or different selected from (C₁-C₆)alkyl, substituted alkyl, aryl, substituted aryl, or arylalkyl groups, as defined above, more preferably the groups are selected from dimethylamino, methylethylamino, diethylamino, phenylmethyl amino and the like;
- the "arylamino" used either alone or in combination with other radicals, represents an aryl group, as defined above, linked through amino having a free valence bond from the nitrogen atom, more preferably the groups are selected from phenylamino, naphthylamino, N-methyl anilino and the like;
- the "oxo" or "carbonyl" group used either alone (-C=O-) or in combination with other radicals such as alkyl described above, for e.g. "alkylcarbonyl", denotes a carbonyl radical (-C=O-) substituted with an alkyl radical described above such as acyl or alkanoyl;
- the "carboxylic acid" group, used alone or in combination with other radicals, denotes a -COOH group, and includes derivatives of carboxylic acid such as esters and amides;
- the "ester" group used alone or in combination with other radicals, denotes -COO-group, and includes carboxylic acid derivatives, more preferably the ester moieties are selected from alkoxycarbonyl, such as methoxycarbonyl, ethoxycarbonyl, and the like, which may optionally be substituted; aryloxycarbonyl group such as phenoxycarbonyl, napthyloxycarbonyl, and the like, which may optionally be substituted; aralkoxycarbonyl group such as benzyloxycarbonyl, phenethyloxycarbonyl, napthylmethoxycarbonyl, and the like, which may optionally be substituted; heteroaryloxycarbonyl, heteroaralkoxycarbonyl, wherein the heteroaryl group, is as defined above, which may optionally be substituted; heterocyclyloxycarbonyl, where the heterocyclic group, as defined earlier, which may optionally be substituted;
- the "amide" group used alone or in combination with other radicals, represents an aminocarbonyl radical (H₂N-C=O), wherein the amino group is mono- or di-substituted or unsubstituted, more preferably the groups are selected from methyl amide, dimethyl amide, ethyl amide, diethyl amide, and the like;
- the "aminocarbonyl" group used either alone or in combination with other radicals, may be selected from 'aminocarbonyl', 'aminocarbonylalkyl", "n-alkylaminocarbonyl", "N-arylaminocarbonyl", "N,N-dialkylaminocarbonyl", "N-alkyl-N-arylaminocarbonyl", "N-alkyl-N-hydroxyaminocarbonyl", and "N-alkyl-N-hydroxyaminocarbonylalkyl", each of them being optionally substituted. The terms "N-alkylaminocabonyl" and "N,N-dialkylaminocarbonyl" denotes aminocarbonyl radicals, as defined above, which have been substituted with one alkyl radical and with two alkyl radicals, respectively. Preferred are "lower alkylaminocarbonyl" having lower alkyl radicals as described above attached to aminocarbonyl radical. The terms "N-arylaminocarbonyl" and "N-alkyl-N-arylaminocarbonyl" denote amiocarbonyl radicals substituted, respectively, with one aryl radical, or one alkyl, and one aryl radical. The term "aminocarbonylalkyl" includes alkyl radicals substituted with aminocarbonyl radicals;
- the "hydroxyalkyl" group used either alone or in combination with other radicals, is selected from an alkyl group, as defined above, substituted with one or more hydroxy radicals, more preferably the groups are selected from hydroxymethyl, hydroxyethyl, hydroxypropyl, hydroxybutyl, hydroxypentyl, hydroxyhexyl and the like;
- the "aminoalkyl" group used alone or in combination with other radicals, denotes an amino (-NH₂) moiety attached to an alkyl radical, as defined above, which may be substituted, such as mono- and di-substituted aminoalkyl. The term "alkylamino" used herein, alone or in combination with other radicals, denotes an alkyl radical, as defined above, attached to an amino group, which may be substituted, such as mono- and di-substituted alkylamino;
- the "alkoxyalkyl" group used alone or in combination with other radicals, denotes an alkoxy group, as defined above, attached to an alkyl group as defined above, more preferably the groups may be selected from methoxymethyl, ethoxymethyl, methoxyethyl, ethoxyethyl and the like;
- the "alkylthio" group used either alone or in combination with other radicals, denotes a straight or branched or cyclic monovalent substituent comprising an alkyl group as defined above, linked through a divalent sulfur atom having a free valence bond from the sulfur atom, more preferably the groups may be selected from methylthio, ethylthio, propylthio,
- the "thioalkyl" group used either alone or in combination with other radicals, denotes an alkyl group, as defined above, attached to a group of formula -SR', where R' represents hydrogen, alkyl or aryl group, e.g. thiomethyl, methylthiomethyl, phenylthiomethyl and the like, which may be optionally substituted.
- the "alkoxycarbonylamino" group used alone or in combination with other radicals, is selected from a suitable alkoxycarbonyl group, as defined above, attached to an amino group, more preferably methoxycarbonylamino, ethoxycarbonylamino, and the like;
- the "arylthio" group used either alone or in combination with other radicals, denotes a an aryl group as defined above, linked through a divalent sulfur atom having a free valence bond from the sulfur atom, more preferably the groups may be selected from phenylthio, naphthylthio, tetrahydronaphthylthio, indanethio, biphenylthio, and the like;
- the "heterocyclylthio" group used either alone or in combination with other radicals, denotes an heterocyclyl group as defined above, linked through a divalent sulfur atom having a free valence bond from the sulfur atom, more preferably the groups may be selected from aziridinylthio, azetidinylthio, pyrrolidinylthio, imidazolidinylthio, piperidinylthio, piperazinylthio, 2-oxopiperidinylthio, 4-oxopiperidinylthio, 2-oxopiperazinylthio, 3-oxopiperazinylthio, morpholinylthio, thiomorpholinylthio, 2-oxomorpholinylthio, azepinylthio, diazepinylthio, oxapinylthio, thiazepinylthio, oxazolidinylthio, thiazolidinylthio, dihydrothiophenethio, dihydropyranthio, dihydrofuranthio, dihydrothiazolethio, benzopyranylthio, benzopyranonylthio, benzodihydrofuranylthio, benzodihydrothienylthio, pyrazolopyrimidonylthio, azaquinazolinoylthio, thienopyrimidonylthio, quinazolonylthio, pyrimidonylthio, benzoxazinylthio, benzoxazinonylthio, benzothiazinylthio, benzothiazinonylthio, thieno piperidinylthio, pyridylthio, thienylthio, furylthio, pyrrolylthio, oxazolylthio, thiazolylthio, isothiazolylthio, imidazolylthio, isoxazolylthio, oxadiazolylthio, thiadiazolylthio, triazolylthio, tetrazolylthio, benzofuranylthio, benzothienylthio, indolinylthio, indolylthio, azaindolylthio, azaindolinylthio, pyrazolopyrimidinylthio, azaquinazolinylthio, pyridofuranylthio, pyridothienylthio, thienopyrimidylthio, quinolinylthio, pyrimidinylthio, pyrazolylthio, quinazolinylthio, pyridazinylthio, triazinylthio, benzimidazolylthio, benzotriazolylthio, phthalazynilthio, naphthylidinylthio, purinylthio, carbazolylthio, phenothiazinylthio, phenoxazinylthio, benzoxazolylthio, benzothiazolylthio and the like;
- the "alkoxycarbonylamino" group used alone or in combination with other radicals, is selected from a suitable alkoxycarbonyl group, as defined above, attached to an amino group, more preferably methoxycarbonylamino, ethoxycarbonylamino, and the like;
- the "aminocarbonylamino", "alkylaminocarbonylamino", "dialkylaminocarbonylamino" groups used alone or in combination with other radicals, is a carbonylamino (-CONH₂) group, attached to amino(NH₂), alkylamino group or dialkylamino group respectively, where alkyl group is as defined above;
- the "amidino" group used either alone or in combination with other radicals, represents a -C(=NH)-NH₂ radical; the "alkylamidino" group represents an alkyl radical, as described above, attached to an amidino group;
- the "alkoxyamino" group used either alone or in combination with other radicals, represents a suitable alkoxy group as defined above, attached to an amino group;
- the "hydroxyamino" group used either alone or in combination with other radicals, represents a -NHOH moiety, and may be optionally substituted with suitable groups selected from those described above;
- the "sulfenyl" group or "sulfenyl derivatives" used alone or in combination with other radicals, represents a bivalent group, -SO- or RₓSO, where Rₓ is an optionally substituted alkyl, aryl, heteroaryl, heterocyclyl, group selected from those described above;
- the "sulfonyl" group or "sulfones derivatives" used either alone or in combination with other radicals, with other terms such as alkylsulfonyl, represents a divalent radical - SO₂-, or RₓSO₂-, where Rₓ is as defined above. More preferably, the groups may be selected from "alkylsulfonyl" wherein suitable alkyl radicals, selected from those defined above, is attached to a sulfonyl radical, such as methylsulfonyl, ethylsulfonyl, propylsulfonyl and the like, "arylsulfonyl" wherein an aryl radical, as defined above, is attached to a sulfonyl radical, such as phenylsulfonyl and the like.
- The term "combination therapy" means the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the conditions or disorders described herein.
- The phrase "therapeutically effective" is intended to qualify the amount of active ingredients used in the treatment of a disease or disorder. This amount will achieve the goal of reducing or eliminating the said disease or disorder.
- The term "therapeutically acceptable" refers to those compounds (or salts, prodrugs, tautomers, zwitterionic forms, etc.) which are suitable for use in contact with the tissues of patients without undue toxicity, irritation, and allergic response, are commensurate with a reasonable benefit/risk ratio, and are effective for their intended use.
- As used herein, reference to "treatment" of a patient is intended to include prophylaxis. The term "patient" means all mammals including humans. Examples of patients include humans, cows, dogs, cats, goats, sheep, pigs, and rabbits. Preferably, the patient is a human.

Suitable groups and substituents on the groups may be selected from those described anywhere in the specification.
Particularly useful compounds may be selected from:
1-(4-((1H-tetrazol-5-yl)methoxy)phenyl)ethanone O-(4-(trifluoromethyl)benzyl) oxime;
1-(4-((2-Methyl-2H-tetrazol-5-yl)methoxy)phenyl)ethanone O-(4-(trifluoromethyl)benzyl) oxime;
1-(4-((1-Methyl-1H-tetrazol-5-yl)methoxy)phenyl)ethanone O-(4-(trifluoromethyl)benzyl) oxime;
1-(6-((1H-tetrazol-5-yl)methoxy)pyridin-3-yl)ethanone O-(4-(trifluoromethyl)benzyl) oxime;
1-(4-((1H-1,2,4-triazol-1-yl)methoxy)phenyl)ethanone O-(4-(trifluoromethyl)benzyl) oxime;
1-(4-((1H-1,2,3-triazol-5-yl)methoxy)phenyl)ethanone O-(4-(trifluoromethyl)benzyl) oxime;
1-(4-((5-Methyl-1,3,4-oxadiazol-2-yl)methoxy)phenyl)ethanone O-(4-(trifluoromethyl) enzyl)oxime;
1-(4-((5-Ethyl-1,3,4-thiadiazol-2-yl)methoxy)phenyl)ethanone O-(4-(trifluoromethyl) benzyl) oxime;
1-(4-((1,3,4-Thiadiazol-2-yl)methoxy)phenyl)ethanone O-(4-(trifluoromethyl)benzyl) oxime;
3-((4-(1-(((4-(Trifluoromethyl)benzyl)oxy)imino)ethyl)phenoxy)methyl)-1,2,4-oxadiazol-5(4H)-one;
5-((4-(1-(((4-(Trifluoromethyl)benzyl)oxy)imino)ethyl)phenoxy)methyl)oxazolidine-2,4-dione;
1-(4-((2-(tert-Butyl)-5-methyloxazol-4-yl)methoxy)phenyl)ethanone O-(4-(trifluoromethyl) benzyl) oxime;
1-(4-((5-(Hydroxymethyl)furan-2-yl)methoxy)phenyl)ethanone O-(4-(trifluoromethyl) benzyl) oxime;
5-((4-(1-(((4-(Trifluoromethyl)benzyl)oxy)imino)ethyl)phenoxy)methyl)furan-2-carboxylic acid;
1-(4-((5-Methyl-4H-1,2,4-triazol-3-yl)methoxy)phenyl)ethanone O-(4-(trifluoromethyl) benzyl) oxime;
1-(6-((5-Methyl-4H-1,2,4-triazol-3-yl)methoxy)pyridin-3-yl)ethanone O-(4-(1,1-difluoroethyl)benzyl) oxime;
1-(6-((5-Methyl-4H-1,2,4-triazol-3-yl)methoxy)pyridin-3-yl)ethanone O-(4-(trifluoromethyl)benzyl) oxime;
1-(4-((3-Amino-1H-1,2,4-triazol-5-yl)methoxy)phenyl)ethanone O-(4-(trifluoromethyl) benzyl) oxime;
1-(4-((5-Amino-1,3,4-oxadiazol-2-yl)methoxy)phenyl)ethanone O-(4-(trifluoromethyl) benzyl) oxime;
N-(5-((4-(1-(((4-(Trifluoromethyl)benzyl)oxy)imino)ethyl)phenoxy)methyl)-1,3,4-oxadiazol-2-yl)methanesulfonamide;
1-(4-((1H-tetrazol-5-yl)methoxy)phenyl)ethanone O-(4-(1,1-difluoroethyl)benzyl)oxime;
1-(2-((1H-tetrazol-5-yl)methoxy)phenyl)ethanone O-(4-(trifluoromethyl)benzyl) oxime;
1-(4-((1H-tetrazol-5-yl)methoxy)phenyl)ethanone O-((6-(trifluoromethyl)pyridin-3-yl) methyl) oxime;
1-(4-((1H-tetrazol-5-yl)methoxy)phenyl)ethanone O-(3-methoxy-4-(trifluoromethyl)benzyl) oxime;
1-(6-((1H-tetrazol-5-yl)methoxy)pyridin-3-yl)ethanone O-(3-methoxy-4-(trifluoromethyl) benzyl) oxime;
1-(4-((1H-tetrazol-5-yl) methoxy)-2-hydroxyphenyl) ethanone O-(4-(trifluoromethyl) benzyl)oxime;
1-(6-((1H-tetrazol-5-yl)methoxy)-1,3-dimethyl-1H-pyrazolo[3,4-b]pyridin-5-yl)ethanone O-(4-(trifluoromethyl)benzyl) oxime;
1-(2-Hydroxy-4-((1-methyl-1H-tetrazol-5-yl)methoxy)phenyl)ethanone O-(4-(trifluoromethyl)benzyl) oxime;
1-(4-((1H-tetrazol-5-yl)methoxy)phenyl)ethanone O-benzyl oxime;
1-(4-((1H-tetrazol-5-yl)methoxy)phenyl)ethanone O-(4-fluorobenzyl) oxime;
1-(6-((1H-tetrazol-5-yl)methoxy)pyridin-3-yl)ethanone O-(4-fluorobenzyl) oxime;
1-(6-((1H-tetrazol-5-yl)methoxy)pyridin-3-yl)ethanone O-(4-ethoxybenzyl) oxime;
1-(6-((1H-tetrazol-5-yl)methoxy)pyridin-3-yl)ethanone O-(4-propoxybenzyl) oxime;
1-(2-((1H-tetrazol-5-yl)methoxy)-6-chloropyridin-3-yl)ethanone O-(4-(trifluoromethyl) benzyl) oxime;
1-(6-((1H-tetrazol-5-yl)methoxy)-2-chloropyridin-3-yl)ethanone O-(4-(trifluoromethyl) benzyl) oxime;
1-(4-((1H-tetrazol-5-yl)methoxy)phenyl)ethanone 1-((4'-(trifluoromethyl)-[1,1'-biphenyl]-4-yl)methyl) oxime.
1-(4-((1H-tetrazol-5-yl)methoxy)phenyl)ethanone O-(2,4,6-triisopropylbenzyl) oxime.
1-(4-((1H-tetrazol-5-yl)methoxy)phenyl)ethanone O-(4-(difluoromethyl)benzyl) oxime;
1-(4-((1-Methyl-1H-tetrazol-5-yl)methoxy)phenyl)ethanone O-(2,4,6-triisopropylbenzyl) oxime;
1-(4-((2-Ethyl-2H-tetrazol-5-yl)methoxy)phenyl)ethanone O-(4-(trifluoromethyl)benzyl) oxime;
1-(4-((1-Ethyl-2H-tetrazol-5-yl)methoxy)phenyl)ethanone O-(4-(trifluoromethyl)benzyl) oxime;
1-(6-((1H-tetrazol-5-yl) methoxy) pyridin-3-yl) ethanone O-(4-(1, 1-difluoroethyl) benzyl) oxime.
1-(6-((2-Ethyl-2H-tetrazol-5-yl)methoxy)pyridin-3-yl)ethanone O-(4-(1,1-difluoroethyl) benzyl) oxime;
1-(6-((1-Ethyl-2H-tetrazol-5-yl)methoxy)pyridin-3-yl)ethanone O-(4-(1,1-difluoroethyl) benzyl) oxime;
1-(6-((2-Ethyl-2H-tetrazol-5-yl)methoxy)pyridin-3-yl)ethanone O-(4-(trifluoromethyl) benzyl) oxime;
1-(6-((1-Ethyl-2H-tetrazol-5-yl)methoxy)pyridin-3-yl)ethanone O-(4-(trifluoromethyl) benzyl) oxime;
1-(6-((2-*iso*Propyl-2H-tetrazol-5-yl)methoxy)pyridin-3-yl)ethanone O-(4-(trifluoromethyl) benzyl) oxime;
1-(6-((1-*iso*Propyl-2H-tetrazol-5-yl)methoxy)pyridin-3-yl)ethanone O-(4-(trifluoromethyl) benzyl) oxime;
1-(6-((2-(Cyclopropylmethyl)-2H-tetrazol-5-yl)methoxy)pyridin-3-yl)ethanone O-(4-(trifluoromethyl)benzyl) oxime;
1-(6-((1-(Cyclopropylmethyl)-2H-tetrazol-5-yl)methoxy)pyridin-3-yl)ethanone O-(4-(trifluoromethyl)benzyl) oxime;
1-(6-((2-(Cyclohexylmethyl)-2H-tetrazol-5-yl)methoxy)pyridin-3-yl)ethanone O-(4-(trifluoromethyl)benzyl) oxime;
1-(6-((1-(Cyclohexylmethyl)-2H-tetrazol-5-yl)methoxy)pyridin-3-yl)ethanone O-(4-(trifluoromethyl)benzyl) oxime;
1-(6-((2-(2-Hydroxyethyl)-2H-tetrazol-5-yl)methoxy)pyridin-3-yl)ethanone O-(4-(trifluoromethyl)benzyl) oxime;
2-(5-(((5-(1-(((4-(Trifluoromethyl)benzyl)oxy)imino)ethyl)pyridin-2-yl)oxy)methyl)-2H-tetrazol-2-yl)acetic acid;
2-(5-(((5-(1-(((4-(Trifluoromethyl)benzyl)oxy)imino)ethyl)pyridin-2-yl)oxy)methyl)-1H-tetrazol-1-yl)acetic acid;
3-(5-(((5-(1-(((4-(Trifluoromethyl)benzyl)oxy)imino)ethyl)pyridin-2-yl)oxy)methyl)-2H-tetrazol-2-yl)propanoic acid;
1-(6-((1H-tetrazol-5-yl)methoxy)-2-methoxypyridin-3-yl)ethanone O-(4-(trifluoromethyl) benzyl) oxime;
1-(6-((2-*iso*Propyl-2H-tetrazol-5-yl)methoxy)pyridin-3-yl)ethanone O-(4-(1,1-difluoroethyl) benzyl) oxime;
1-(6-((1-*iso*Propyl-2H-tetrazol-5-yl)methoxy)pyridin-3-yl)ethanone O-(4-(1,1-difluoroethyl) benzyl) oxime;
1-(6-((2-(Cyclopropylmethyl)-2H-tetrazol-5-yl)methoxy)pyridin-3-yl)ethanone O-(4-(1,1-difluoroethyl)benzyl) oxime;
1-(6-((1-(Cyclopropylmethyl)-2H-tetrazol-5-yl)methoxy)pyridin-3-yl)ethanone O-(4-(1,1-difluoroethyl)benzyl) oxime;
1-(6-((2-(2-Hydroxyethyl)-2H-tetrazol-5-yl)methoxy)pyridin-3-yl)ethanone O-(4-(1,1-difluoroethyl)benzyl) oxime;
1-(6-((1-(2-Hydroxyethyl)-2H-tetrazol-5-yl)methoxy)pyridin-3-yl)ethanone O-(4-(1,1-difluoroethyl)benzyl) oxime;
1-(4-((2-(2-Hydroxyethyl)-2H-tetrazol-5-yl)methoxy)phenyl)ethanone O-(4-(trifluoromethyl)benzyl) oxime;
2-(5-((4-(1-(((4-(Trifluoromethyl)benzyl)oxy)imino)ethyl)phenoxy)methyl)-2H -tetrazol-2-yl)acetonitrile;
1-(4-((2-(2-Aminoethyl)-2H-tetrazol-5-yl)methoxy)phenyl)ethanone O-(4-(trifluoromethyl)benzyl) oxime;
1-(4-((1-((Tetrahydro-2H-pyran-4-yl)methyl)-1H-tetrazol-5-yl)methoxy)phenyl)ethanone O-(4-(trifluoromethyl)benzyl) oxime;
1-(4-((2-((Tetrahydro-2H-pyran-4-yl)methyl)-2H-tetrazol-5-yl)methoxy)phenyl)ethanone O-(4-(trifluoromethyl)benzyl) oxime;
1-(6-((1-Benzyl-2H-tetrazol-5-yl)methoxy)pyridin-3-yl)ethanone O-(4-(trifluoromethyl) benzyl) oxime;
1-(6-((2-Benzyl-2H-tetrazol-5-yl)methoxy)pyridin-3-yl)ethanone O-(4-(trifluoromethyl) benzyl) oxime;
1-(4-((1,3,4-Oxadiazol-2-yl)methoxy)phenyl)ethanone O-(4-(trifluoromethyl)benzyl) oxime;
1-(6-((5-Methyl-1,3,4-oxadiazol-2-yl)methoxy)pyridin-3-yl)ethanone O-(4-(trifluoromethyl) benzyl) oxime;
1-(4-((2H-1,2,3-triazol-2-yl)methoxy)phenyl)ethanone O-(4-(trifluoromethyl)benzyl)oxime;
1-(4-((1H-1,2,3-triazol-1-yl)methoxy)phenyl)ethanone O-(4-(trifluoromethyl)benzyl)oxime;
1-(4-(Thiophen-2-ylmethoxy)phenyl)ethanone O-(4-(trifluoromethyl)benzyl)oxime;
1-(4-(Furan-2-ylmethoxy) phenyl)ethanone O-(4-(trifluoromethyl)benzyl)oxime;
1-(4-(Furan-3-ylmethoxy)phenyl)ethanone O-(4-(trifluoromethyl)benzyl)oxime;
3-((4-(1-(((4-(Trifluoromethyl) benzyl) oxy)imino)ethyl)phenoxy)methyl)-1H-1,2,4-triazol-5(4H)-one;
1-(4-((2-Methylthiazol-4-yl)methoxy)phenyl)ethanone O-(4-(trifluoromethyl)benzyl)oxime;
5-((4-(1-(((3-methoxy-4-(Trifluoromethyl)benzyl)oxy)imino)ethyl)phenoxy)methyl)oxazolidine-2,4-dione;
1-(4-((1-Trityl-1H-imidazol-4-yl)methoxy)phenyl)ethanone O-(4-(trifluoromethyl)benzyl) oxime;
1 -(4-((1H-imidazol-4-yl)methoxy)phenyl)ethanone O-(4-(1,1-difluoroethyl)benzyl)oxime;
5-((4-(1-(((4-(Trifluoromethyl)benzyl)oxy)imino)ethyl)phenoxy)methyl)isoxazol-3(2H)-one;
1-(4-((5-(Morpholinomethyl)isoxazol-3-yl)methoxy)phenyl)ethanone O-(4-(trifluoromethyl) benzyl)oxime;
1-(4-((1H-benzo[d]imidazol-2-yl)methoxy)phenyl)ethanone O-(4-(trifluoromethyl)benzyl) oxime
1-((4-(1-(((4-(trifluoromethyl)benzyl)oxy)imino)ethyl)phenoxy)methyl)pyrrolidine-2,5-dione;
1-(((5-(1-(((4-(trifluoromethyl)benzyl)oxy)imino)ethyl)pyridin-2-yl)oxy)methyl) pyrrolidine-2,5-dione;
5-((4-(1-(((4-(trifluoromethyl)benzyl)oxy)imino)ethyl)phenoxy)methyl)thiazolidine-2,4-dione;
5-(((5-(1-(((4-(trifluoromethyl)benzyl)oxy)imino)ethyl)pyridin-2yl)oxy)methyl) thiazolidine-2,4-dione;
2-((4-(1-(((4-(trifluoromethyl)benzyl)oxy)imino)ethyl)phenoxy)methyl)cyclopentane-1,3-dione;
2-(((5-(1-(((4-(trifluoromethyl)benzyl)oxy)imino)ethyl)pyridin-2 yl)oxy)methyl)cyclopentane-1,3-dione;
3-((4-(1-(((4-(trifluoromethyl)benzyl)oxy)imino)ethyl)phenoxy)methyl)pyrrolidine-2,5-dione;
3-(((5-(1-(((4-(trifluoromethyl)benzyl)oxy)imino)ethyl)pyridin-2-yl)oxy)methyl)pyrrolidine-2,5-dione;
1-(4-((4,5-dihydroisoxazol-3-yl)methoxy)phenyl)ethanone O-(4-(trifluoromethyl)benzyl) oxime;
1-(6-((4,5-dihydroisoxazol-3-yl)methoxy)pyridin-3-yl)ethanone O-(4-(trifluoromethyl) benzyl) oxime;
1-(4-(pyrrolidin-2-ylmethoxy)phenyl)ethanone O-(4-(trifluoromethyl)benzyl) oxime;
1-(6-(pyrrolidin-2-ylmethoxy)pyridin-3-yl)ethanone O-(4-(trifluoromethyl)benzyl) oxime;
4-hydroxy-5,5-dimethyl-3-((4-(1-(((4-(trifluoromethyl)benzyl)oxy)imino)ethyl)phenoxy) methyl)furan-2(5H)-one;
4-hydroxy-5,5-dimethyl-3-(((5-(1-(((4-(trifluoromethyl)benzyl)oxy)imino)ethyl)pyridin-2-yl)oxy)methyl)furan-2(5H)-one;
1-(4-(pyrrolidin-1-ylmethoxy)phenyl)ethanone O-(4-(trifluoromethyl)benzyl) oxime;
1-(6-(pyrrolidin-1-ylmethoxy)pyridin-3-yl)ethanone O-(4-(trifluoromethyl)benzyl) oxime;
5-((4-(1-(((4-(trifluoromethyl)benzyl)oxy)immo)ethyl)phenoxy)methyl)dihydrofuran-2(3H)-one;
5-(((5-(1-(((4-(trifluoromethyl)benzyl)oxy)immo)ethyl)pyridm-2-yl)oxy)methyl) dihydrofuran-2(3H)-one.

The novel compounds of this invention may be prepared using the reactions and techniques as shown in scheme below and described in this section. The reactions are performed in solvents appropriate to the reagents and materials employed and are suitable for the transformations being affected. It is understood by those skilled in the art that the nature and order of the synthetic steps presented may be varied for the purpose of optimizing the formation of the compounds of the present invention. It will also be well appreciated that one or more of the reactants may be protected and deprotected for facile synthesis by techniques known to persons skilled in the art. It will also be appreciated that one or more of the compounds of the present invention may exist in stereoisomeric and/or diastereomeric forms. Such stereoisomers and/or diastereoisomers as well as their optical antipodes are to be construed to be within the scope of the present invention. It will also be well appreciated that one or more of these compounds may be converted to their salts and other derivatives based on the specific groups present on the compounds, which can be well comprehended by persons skilled in the art. Such salts and/or other derivatives, as the case may be should also be construed to be within the scope of the present invention.

The compounds of general formula **(I)** wherein all the symbols are as defined earlier, may be prepared by reactions outlined in **Scheme 1-5** below which comprises: 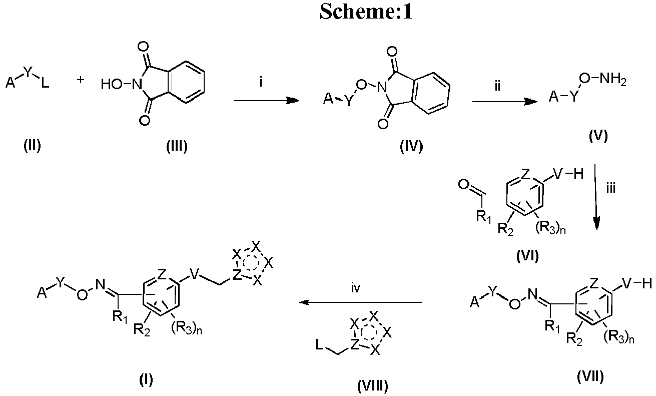
i. reacting the compounds of general formula **II** wherein 'L' represents a suitable leaving group and all other symbols are as defined earlier and N-hydroxyphthalimide **III** using suitable inorganic base(s) such as NaOH, KOH, K₂CO₃, Cs₂CO₃ and the like or organic base(s) such as pyridine, triethyl amine, diisopropyl ethylamine and the like to prepare compounds of general formula **IV** wherein the symbols are as defined earlier. The reaction may be carried out neat or in presence of suitable protic solvent(s) such as methanol, ethanol, butanol and the like or suitable aprotic solvent(s) such as dimethyl formamide, tetrahydrofuran, dichloromethane and the like or suitable mixtures thereof. The reaction may be carried out at a temperature in the range of 0 °C to reflux temperature of the solvent(s) used and the reaction time may range from 1 to 48 hours;
ii. reacting the compounds of general formula **IV** wherein all the symbols are as defined earlier with hydrazine hydrate as a deprotecting agent to obtain the compounds of general formula **V** wherein all the symbols are as defined earlier. The reaction may be carried out neat or in presence of suitable protic solvent(s) such as methanol, ethanol, butanol and the like or suitable aprotic solvent(s) such as toluene, tetrahydrofuran, dichloromethane and the like or mixtures thereof. The reaction may be carried out at a temperature in the range of 0 °C to reflux temperature of the solvent(s) used and the reaction time may range from 1 to 48 hours;
iii. reacting the compounds of general formula **V** with compounds of general formula **VI** wherein 'V' represents 'O' or 'NR₄' and all other symbols are as defined earlier in the presence of a base(s) like NaOH, NaOAc, pyridine and the like to prepare the compounds of general formula **VII** wherein 'V' represents 'O' or 'NR₄' and all other symbols are as defined earlier. The reaction may be carried out in presence of suitable solvent(s) such as methanol, ethanol, butanol, water and the like or suitable mixtures thereof. The reaction may be carried out at a temperature in the range of 0 °C to reflux temperature of the solvent(s) used and the reaction time may range from 1 to 48 hours;
iv. reacting the compounds of general formula **VII** with the compounds of formula **VIII** wherein 'L' represents a suitable leaving group and all other symbols are as defined earlier using suitable inorganic base(s) such as NaOH, KOH, K2CO3, Cs2CO3 and the like or organic base(s) such as pyridine, triethyl amine, diisopropyl ethylamine and the like to prepare compounds of general formula **I** wherein all the symbols are as defined earlier. The reaction may be carried out neat or in presence of suitable protic solvent(s) such as methanol, ethanol, butanol and the like or suitable aprotic solvent(s) such as dimethyl formamide, tetrahydrofuran, dichloromethane and the like or suitable mixtures thereof. The reaction may be carried out at a temperature in the range of 0 °C to reflux temperature of the solvent(s) used and the reaction time may range from 1 to 48 hours;

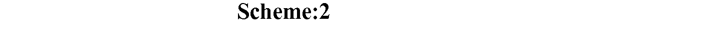

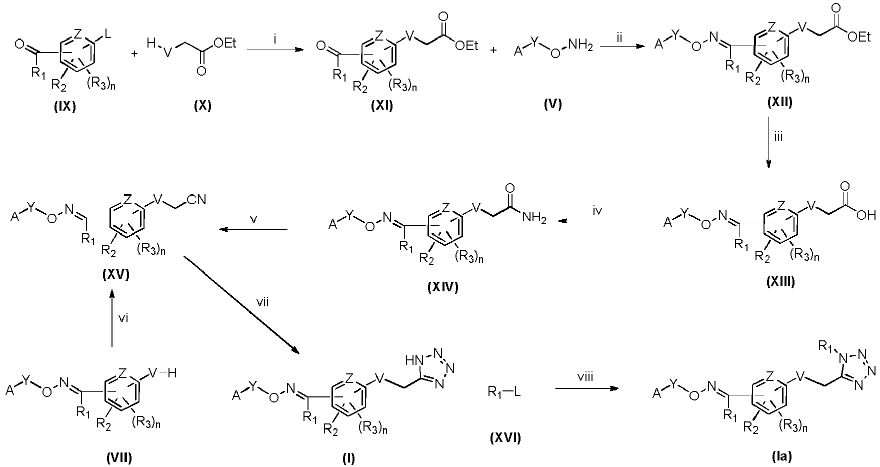

i. reacting the compounds of general formula **IX** wherein 'Z' represents N, 'L' represents a suitable leaving group and all other symbols are as defined earlier and compounds of general formula **X** wherein all other symbols are as defined earlier using suitable inorganic base(s) such as NaOH, KOH, K₂CO₃, Cs₂CO₃ and the like or organic base(s) such as pyridine, triethyl amine, diisopropyl ethylamine and the like to prepare compounds of general formula **XI** all the symbols are as defined earlier. The reaction may be carried out neat or in presence of suitable protic solvent(s) such as methanol, ethanol, butanol and the like or suitable aprotic solvent(s) such as dimethyl formamide, tetrahydrofuran, dichloromethane and the like or suitable mixtures thereof. The reaction may be carried out at a temperature in the range of 0 °C to reflux temperature of the solvent(s) used and the reaction time may range from 1 to 48 hours;
ii. reacting the compounds of general formula **XI** wherein all the symbols are as defined earlier with the compounds of general formula **V** wherein all the symbols are as defined earlier in the presence of a base(s) like NaOH, NaOAc, pyridine and the like to prepare the compounds of general formula **XII** wherein all the symbols are as defined earlier. The reaction may be carried out in presence of suitable solvent(s) such as methanol, ethanol, butanol, water and the like or suitable mixtures thereof. The reaction may be carried out at a temperature in the range of 0 °C to reflux temperature of the solvent(s) used and the reaction time may range from 1 to 48 hours;
iii. compounds of general formula **XIII** wherein all the symbols are as defined earlier may be prepared by the hydrolysis of compounds of general formula **XII** wherein all the symbols are as defined earlier using suitable base(s) e.g., NaOH, LiOH, KOH and the like. Reaction may be conducted in suitable solvent(s) such as methanol, ethanol, THF, water and the like or the mixtures thereof. The reaction may be carried out at a temperature in the range of 20 °C to reflux temperature of the solvent(s) used and the reaction time may range from 1 to 48 hours;
iv. compounds of formula **XIV** wherein all the symbols are as defined earlier may be prepared from corresponding acid of general formula **XIII** wherein all symbols are as defined earlier using suitable methods available in the literature for conversion of acid to amide;
v. reacting the compounds of general formula **XIV** wherein all the symbols are as defined earlier with oxalyl chloride and DMSO in the presence of a organic base(s) like triethyl amine, diisopropylethylamine, pyridine and the like to prepare the compounds of general formula **XV** wherein all the symbols are as defined earlier. The reaction may be carried out in presence of suitable solvent(s) such as dichloromethane, chloroform and the like or suitable mixtures thereof. The reaction may be carried out at a temperature in the range of -78 °C to reflux temperature of the solvent(s) used and the reaction time may range from 1 to 48 hours;
vi. compounds of general formula **XV** wherein all the symbols are as defined earlier may also be prepared by the coupling of compounds of general formula **VII** wherein all the symbols are as defined earlier and bromoacetonitrile using suitable inorganic base(s) such as NaOH, KOH, K₂CO₃, Cs₂CO₃ and the like or organic base(s) such as pyridine, triethyl amine, diisopropyl ethylamine and the like. The reaction may be carried out neat or in presence of suitable protic solvent(s) such as methanol, ethanol, butanol and the like or suitable aprotic solvent(s) such as dimethyl formamide, tetrahydrofuran, dichloromethane and the like or suitable mixtures thereof. The reaction may be carried out at a temperature in the range of 0 °C to reflux temperature of the solvent(s) used and the reaction time may range from 1 to 48 hours;
vii. compounds of general formula **(I)** wherein all the symbols are as defined earlier may be prepared by reacting compounds of general formula **XV** wherein all the symbols are as defined earlier and sodium azide in presence of ammonium chloride. The reaction may be carried out in presence of suitable solvent(s) such as DMF and the like. The reaction may be carried out at a temperature in the range of 0 °C to reflux temperature of the solvent(s) used and the reaction time may range from 1 to 48 hours;
viii. reacting the compounds of general formula **(I)** wherein all the symbols are as defined earlier and compounds of general formula **XVI** wherein 'L' represents a suitable leaving group and all other symbols are as defined earlier using suitable inorganic base(s) such as NaOH, KOH, K₂CO₃, Cs₂CO₃ and the like or organic base(s) such as pyridine, triethyl amine, diisopropyl ethylamine and the like to prepare compounds of general formula **(Ia)** wherein all the symbols are as defined earlier. The reaction may be carried out neat or in presence of suitable protic solvent(s) such as methanol, ethanol, butanol and the like or suitable aprotic solvent(s) such as dimethyl formamide, tetrahydrofuran, dichloromethane and the like or suitable mixtures thereof. The reaction may be carried out at a temperature in the range of 0 °C to reflux temperature of the solvent(s) used and the reaction time may range from 1 to 48 hours.

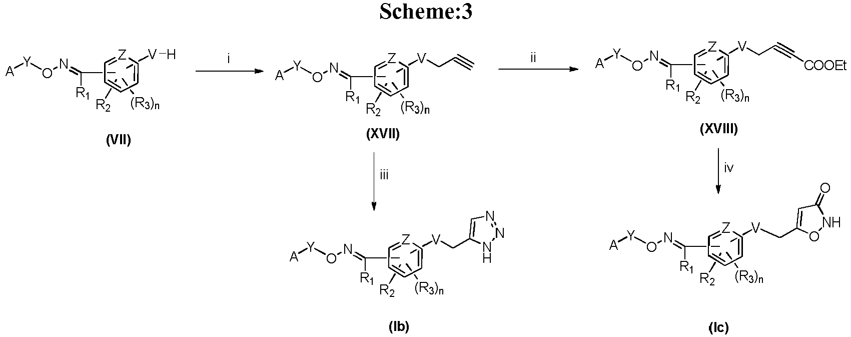

i. reacting the compounds of general formula **VII** wherein all the symbols are as defined earlier and propargyl chloride using suitable inorganic base(s) such as NaOH, KOH, K₂CO₃, Cs₂CO₃ and the like or organic base(s) such as pyridine, triethyl amine, diisopropyl ethylamine and the like to prepare compounds of general formula **XVII** wherein all the symbols are as defined earlier. The reaction may be carried out neat or in presence of suitable protic solvent(s) such as methanol, ethanol, butanol and the like or suitable aprotic solvent(s) such as dimethyl formamide, tetrahydrofuran, dichloromethane and the like or suitable mixtures thereof. The reaction may be carried out at a temperature in the range of 0 °C to reflux temperature of the solvent(s) used and the reaction time may range from 1 to 48 hours;
ii. compounds of general formula **XVIII** wherein all the symbols are as defined earlier may be prepared by reacting compounds of general formula **XVII** wherein all the symbols are as defined earlier and ethyl chloroformate in presence of organometallic reagents such as n-Butyllithium, s-Butyllithium, LDA and the like. The reaction may be carried out in presence of suitable solvent(s) such as tetrahydrofuran, diethyl ether and the like or suitable mixtures thereof. The reaction may be carried out at a temperature in the range of -78 °C to reflux temperature of the solvent(s) used and the reaction time may range from 1 to 48 hours.
iii. compounds of general formula **(Ib)** wherein all the symbols are as defined earlier may be prepared by reacting compounds of general formula **XVII** wherein all the symbols are as defined earlier and trimethyl silyl azide in presence of copper(I)iodide. The reaction may be carried out neat or in presence of suitable protic solvent(s) such as methanol, ethanol, butanol and the like or suitable aprotic solvent(s) such as dimethyl formamide, tetrahydrofuran, dichloromethane and the like or suitable mixtures thereof. The reaction may be carried out at a temperature in the range of 0 °C to reflux temperature of the solvent(s) used and the reaction time may range from 1 to 48 hours.
iv. compounds of general formula **(Ic)** wherein all the symbols are as defined earlier may be prepared by reacting compounds of general formula **XVIII** wherein all the symbols are as defined earlier and hydroxylamine hydrochloride in presence of suitable inorganic base(s) such as NaOH, KOH and the like. Reaction may be conducted in suitable solvent(s) such as methanol, ethanol, THF, water and the like or the mixtures thereof. The reaction may be carried out at a temperature in the range of 0 °C to reflux temperature of the solvent(s) used and the reaction time may range from 1 to 48 hours;

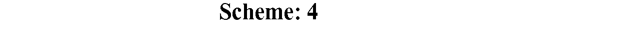

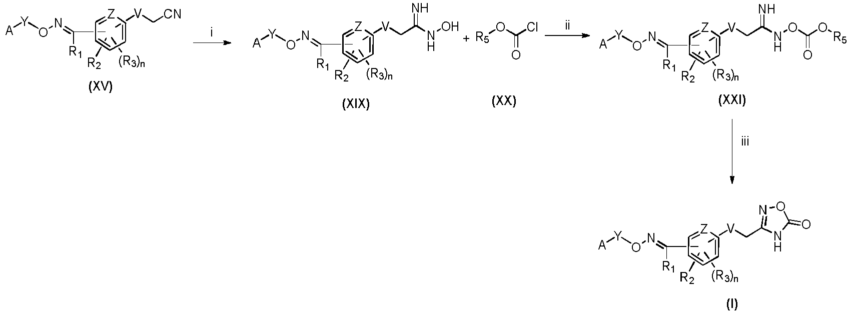

i. compounds of general formula **XIX** wherein all the symbols are as defined earlier may be prepared by reacting compounds of general formula **XV** wherein all the symbols are as defined earlier and hydroxylamine hydrochloride in presence of suitable inorganic base(s) such as NaOH, KOH, K₂CO₃, Cs₂CO₃ and the like. Reaction may be conducted in suitable solvent(s) such as methanol, ethanol, THF, water and the like or the mixtures thereof. The reaction may be carried out at a temperature in the range of 0 °C to reflux temperature of the solvent(s) used and the reaction time may range from 1 to 48 hours.
ii. compounds of general formula **XXI** wherein R₅ represents an alkyl or aryl group and all other symbols are as defined earlier may be prepared by reacting compounds of general formula **XIX** wherein all the symbols are as defined earlier and compounds of general formula **XX** wherein all the symbols are as defined earlier in presence of suitable organic base(s) such as pyridine, triethyl amine, diisopropyl ethylamine and the like. Reaction may be conducted in suitable solvent(s) such as dichloromethane, chloroform and the like or the mixtures thereof. The reaction may be carried out at a temperature in the range of 0 °C to reflux temperature of the solvent(s) used and the reaction time may range from 1 to 48 hours.
iii. cyclization of the compounds of general formula **XXI** wherein all the symbols are as defined earlier using suitable solvent(s) such as benzene, toluene, xylene and the like or suitable mixtures thereof to prepare compounds of general formula **XVII** wherein all the symbols are as defined earlier. The reaction may be carried out at a temperature in the range of 0 °C to reflux temperature of the solvent(s) used and the reaction time may range from 1 to 48 hours.

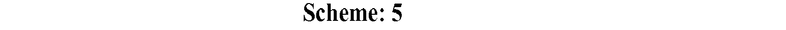

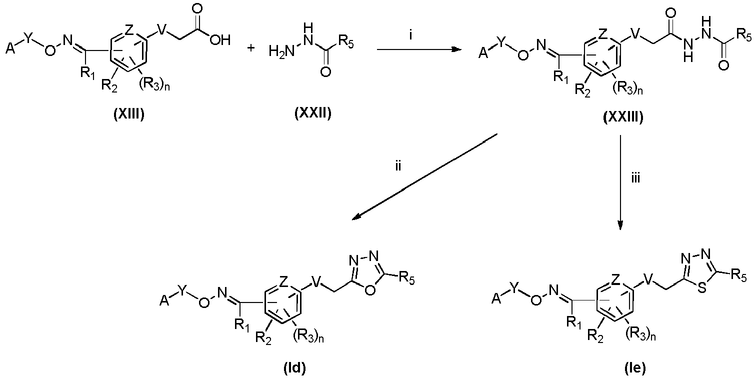

i. compounds of formula **XXIII** wherein all the symbols are as defined earlier may be prepared by coupling of compounds of formula **XIII** wherein all the symbols are as defined earlier and compounds of general formula **XXII** wherein all the symbols are as defined earlier using suitable methods available in the literature for standard peptide coupling;
ii. cyclization of the compounds of general formula **XXIII** wherein all the symbols are as defined earlier in presence of thionyl chloride, *p*-toluene sulfonyl chloride and the like using suitable solvent(s) such as benzene, toluene, xylene, acetonitrile and the like or suitable mixtures thereof to prepare compounds of general formula **(Id)** wherein all the symbols are as defined earlier. The reaction may be carried out at a temperature in the range of 0 °C to reflux temperature of the solvent(s) used and the reaction time may range from 1 to 48 hours;
iii. cyclization the compounds of general formula **XXIII** wherein all the symbols are as defined earlier in presence of phosporous pentasulfide using suitable reaction medium such as alumina, silica and the like or suitable solvent(s) or suitable mixtures thereof to prepare compounds of general formula **(Ie)** wherein all the symbols are as defined earlier. The reaction may be carried out under microwave irradiation or under heating at a temperature in the range of 30 °C to reflux temperature of the solvent(s) used and the reaction time may range from 1 to 48 hours.

The pharmaceutical composition is provided by employing conventional techniques. Preferably the composition is in unit dosage form containing an effective amount of the active component, that is, the compounds of formula (I) according to this invention.

The quantity of active component, that is, the compounds of formula (I) according to this invention, in the pharmaceutical composition and unit dosage form thereof may be varied or adjusted widely depending upon the particular application method, the potency of the particular compound and the desired concentration. Generally, the quantity of active component will range between 0.5% to 90% by weight of the composition.

The compound of the present invention may be used alone or in combination with a second medicament as may be necessary depending on the condition of the patient, the severity of the disease and such other conditions which are well known to a skilled practitioner. Such second medicament when required may be selected from a HMG-Co-A reductase inhibitor, angiotension converting enzyme (ACE) inhibitor, calcium channel blocker, aldosterone synthase inhibitor, aldoasterone antagonist, dual angiotension converting enzyme/neutral endopeptidase (ACE/NEP) inhibitor, endothelin antagonist, angiotension II receptor blocker (ARB) including their pharmaceutically acceptable salts as well as a combination of one or more medicines from any of these classes along with the compound of formula (I) of the present invention.

The invention is explained in greater detail by the examples given below, which are provided by way of illustration only and therefore should not be construed to limit the scope of the invention.

### 1H NMR spectral data given in the examples (vide infra) are recorded using a 400 MHz spectrometer (Bruker AVANCE-400) and reported in δ scale.

### Example 1

1-(4-((1H-tetrazol-5-yl)methoxy)phenyl)ethanone O-(4-(trifluoromethyl)benzyl) oxime.

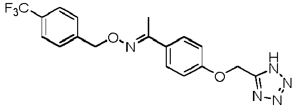

### Step 1: Preparation of 2-((4-(trifluoromethyl)benzyl)oxy)isoindoline-1,3-dione.

To a solution of 1-(bromomethyl)-4-(trifluoromethyl)benzene (10 g, 41.8 mmol) in DMF (50 ml) K₂CO₃ (11.56 g, 84 mmol) was added followed by 2-hydroxyisoindoline-1,3-dione (6.82 g, 41.8 mmol) under nitrogen atmosphere at 25 °C. The reaction mixture was stirred under nitrogen atmosphere at 25 °C for 18 hr. The reaction mixture was poured into ice cold water (200 mL). Off white solid separated was filtered, washed with water and dried over P₂O₅ under vacuum to yield 5.1 g (37.9 %) of title product as off white solid.
¹H NMR: CDCl₃, *δ* 5.27 (s, 2H), 7.64-7.69 (m, 4H), 7.73-7.77 (m, 2H), 7.80-7.85 (m, 2H).

### Step 2: Preparation of O-(4-(trifluoromethyl)benzyl)hydroxylamine hydrochloride.

To a solution of 2-((4-(trifluoromethyl)benzyl)oxy)isoindoline-1,3-dione (5.1 g, 15.88 mmol) in THF (40 ml) and EtOH (40.0 ml) was added hydrazine monohydrate (1.557 ml, 31.8 mmol) and the reaction mixture was stirred for 1 hour at 25 °C. The reaction mixture was poured into K₂CO₃ solution (100 mL) and extracted with ethyl acetate. The combined organic layer was washed with water & brine solution, dried over Na₂SO₄ and evaporated under vacuum to yield crude product as brown thick liquid. The crude product was added to ethereal HCl. The solid separated was filtered, washed with diethyl ether and dried over CaCl₂ under vacuum to yield 2 g, (55.3 %) of product as off white solid.
¹H NMR: MeOD, *δ* 5.15 (s, 2H), 7.64 (d, *J* = 8.0 Hz, 2H), 7.74 (d, *J* = 8.0 Hz, 2H).

### Step 3: Preparation of 1-(4-hydroxyphenyl)ethanone O-(4-(trifluoromethyl)benzyl) oxime.

To a solution of 1-(4-hydroxyphenyl)ethanone (5.98 g, 43.9 mmol) in EtOH (100 ml) was added O-(4-(trifluoromethyl)benzyl)hydroxylamine hydrochloride (10 g, 43.9 mmol) followed by acetic acid (7.55 ml, 132 mmol) and sodium acetate (7.21 g, 88 mmol) at 25 °C and the reaction mixture was refluxed for 2 hr. The reaction mixture was cooled to room temperature and solvent was evaporated under vacuum. The residue was poured into water. White solid separated was filtered, washed with water and dried over P₂O₅ under vacuum to yield 12.0 g (88 %) of product as white solid.
¹H NMR: CDCl₃, *δ* 2.25 (s, 3H), 5.22 (s, 2H), 6.76-6.79 (m, 2H), 7.49-7.53 (m, 4H), 7.59 (d, *J* = 8.4 Hz, 2H).

### Step 4: Preparation of 2-(4-(1-(((4-(trifluoromethyl)benzyl)oxy)imino)ethyl)phenoxy) acetonitrile.

To a solution of 1-(4-hydroxyphenyl)ethanone O-(4-(trifluoromethyl)benzyl) oxime (5g, 16.2 mmol) in DMF (40 mL) was added cesiumcarbonate (10.5 g, 32.3 mmol) followed by 2-bromoacetonitrile (1.35 mL, 19.4 mmol) and the reaction mixture was stirred at ambient temperature for 6 hours. Water was added to the reaction mixture and extracted with ethylacetate (3X100 mL). Combined organic extract was washed with water & brine solution, dried over sodium sulphate and evaporated on rotavopor under reduced pressure to yield 4.5 g (80 %) of product as white solid.

### Step 5: Preparation of 1-(4-((1H-tetrazol-5-yl)methoxy)phenyl)ethanone O-(4-(trifluoromethyl)benzyl) oxime.

To a solution of 2-(4-(1-(((4-(trifluoromethyl)benzyl)oxy)imino)ethyl)phenoxy)acetonitrile (4.5 g, 12.9 mmol) in DMF (40 mL) were added sodium azide (1.68 g, 25.8 mmol) and ammonium chloride (1.38 g, 25.8 mmol) and the reaction mixture was stirred at 120° C for 3 hour. Water was added to the reaction mixture, solid separated was filtered under suction and dried under vacuum to yield 3.5 g (68.5 %) of product as white solid.
¹H NMR: DMSO-*d*₆, *δ* 2.22 (s, 3H), 5.27 (s, 2H), 5.37 (s, 2H), 7.06 (d, *J* = 8.8 Hz, 2H), 7.58-7.62 (m, 4H), 7.73 (d, *J=* 8.0 Hz, 2H).

### Example 2

### 1-(4-((2-Methyl-2H-tetrazol-5-yl)methoxy)phenyl)ethanone O-(4-(trifluoromethyl) benzyl) oxime.

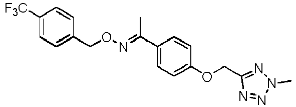

A solution of 1-(4-((1H-tetrazol-5-yl)methoxy)phenyl)ethanone O-(4-(trifluoromethyl)benzyl) oxime (0.5g, 1.27 mmol) in DMF (5 ml) was placed into round-bottomed flask. Iodomethane (0.088 mL, 1.4 mmol) was added to the reaction mixture followed by addition of Cs₂CO₃ (265 mg, 1.916 mmol) under nitrogen atmosphere. The reaction mixture was stirred under nitrogen atmosphere at 27 °C for 3 hours. The reaction mixture was poured into H₂O (25 mL) and extracted by ethyl acetate. The combined organic layers was washed with H₂O (25 mL) & brine solution (20 mL), dried over Na₂SO₄ and evaporated on rotavapor under vacuum to yield crude product as mixture of 1-(4-((2-methyl-2H-tetrazol-5-yl)methoxy)phenyl)ethanone O-(4-(trifluoromethyl)benzyl) oxime and 1-(4-((1-methyl-2H-tetrazol-5-yl)methoxy)phenyl)ethanone O-(4-(trifluoromethyl)benzyl) oxime which were separated by column chromatography. Initial fractions contained 225 mg (49 %) of pure title compound as white solid.
¹H NMR: DMSO-*d*₆, *δ* 2.22 (s, 3H), 4.38 (s, 3H), 5.27 (s, 2H), 5.39 (s, 2H), 7.06 (d, *J* = 6.8 Hz, 2H), 7.57-7.61 (m, 4H), 7.73 (*d, J* = 8.0 Hz, 2H).

### Example 3

### 1-(4-((1-Methyl-1H-tetrazol-5-yl)methoxy)phenyl)ethanone O-(4-(trifluoromethyl) benzyl) oxime.

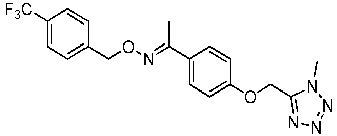

Later fractions from column chromatography in example 2 gave 140 mg (30%) of title compound as white solid.
¹H NMR: CDCl₃, *δ* 2.25 (s, 3H), 4.16 (s, 3H), 5.26 (s, 2H), 5.44 (s, 2H), 6.96 (d, *J* = 10.0Hz, 2H), 7.50 (d, *J* = 8.0 Hz, 2H), 7.58-7.62 (m, 4H).

### Example 4

### 1-(6-((1H-tetrazol-5-yl)methoxy)pyridin-3-yl)ethanone O-(4-(trifluoromethyl)benzyl)oxime.

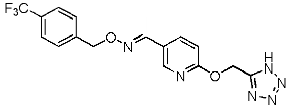

### Step 1: Preparation of Ethyl 2-((5-acetylpyridin-2-yl)oxy)acetate.

To a solution of 1-(6-chloropyridin-3-yl)ethanone (4.0 gm, 0.0257 mole) in DMF (15 mL), cesium carbonate (16.8 gm, 0.051 mole) was added followed by addition of methyl 2-hydroxyacetate (8.0 ml, 0.103 mmoles) at 25 °C under nitrogen atmosphere and the reaction mixture was stirred at 80-90 °C for 18 h. The reaction mixture was poured into ice cold water and extracted with ethyl acetate. The combined ethyl acetate extract was washed with water & brine, dried over sodium sulphate and evapourated under reduced pressure. The crude product was purified by colunm chromatography (Eluent: 16% ethyl acetate in hexane) to yield 1.25 gm (23%) of product as off white solid.
¹H NMR: DMSO-*d*₆, *δ* 2.55 (s, 3H), 3.67 (s, 3H), 5.02 (s, 2H), 7.04 (dd, *J* = 8.8 & 0.4 Hz, 1H), 8.20 (dd, *J* = 8.8 & 2.4 Hz, 1H), 8.78 (d, *J* = 2.0 Hz, 1H).

### Step 2: Preparation of Methyl 2-((5-(1-(hydroxyimino)ethyl)pyridin-2-yl)oxy)acetate.

To a solution of methyl 2-((5-acetylpyridin-2-yl)oxy)acetate (1.23 gm, 0.059 mole) in ethanol (10 mL), a solution of sodium acetate (0.942 gm, 0.0117 mole) and hydroxylammonium chloride (1.2 gm, 0.0117 mole) in water (5 ml) was added and the reaction mixture was refluxed for 1 hour. The reaction mixture was cooled to room temperature and solvent was evapourated in vacuum. The residue was diluted with ice cold water (100 mL) and solid seperated was filtered, washed with water and dried over P₂O₅ under vacuum to yield 1.15 gm (88%) of title product as off white solid.
¹H NMR: DMSO-*d*₆, *δ* 2.12 (s, 3H), 3.66 (s, 3H), 4.94 (s, 2H), 6.95 (d, *J* = 8.8 Hz, 1H), 8.02 (dd, *J* = 8.4 & 2.4 Hz, 1H), 8.34 (d, *J* = 2.0 Hz, 1H).

### Step 3: Preparation of Methyl 2-((5-(1-(((4-(trifluoromethyl)benzyl)oxy)imino)ethyl) pyridin-2-yl)oxy)acetate.

To a solution of 1-(bromomethyl)-4-(trifluoromethyl)benzene (0.87 ml, 5.6 mmoles) in DMF (10 mL), cesium carbonate (3.35 gm, 10.2 mmoles) was added followed by addition of methyl 2-((5-(1-(hydroxyimino)ethyl)pyridin-2-yl)oxy)acetate (1.15 gm, 5.1 mmoles) at 25 °C under nitrogen atmosphere and the reaction mixture was stirred at the same temperature for 12 h. The reaction mixture was poured into ice cold water and extracted with ethyl acetate. The combined ethyl acetate extract was washed with water & brine, dried over sodium sulphate and evaporated under reduced pressure to yield 2.0 gm (94%) of product as thick liquid.
¹H NMR: DMSO-*d*₆, *δ* 2.24 (s, 3H), 3.65 (s, 3H), 4.94 (s, 2H), 5.29 (s, 2H), 6.94 (d, *J* = 8.8 Hz, 1H), 7.60 (d, *J* = 8.0 Hz, 2H), 7.73 (d, *J* = 8.0 Hz, 2H), 8.0 (dd, *J* = 8.8 & 2.4 Hz, 1H), 8.36 (d, *J* = 2.4 Hz, 1H).

### Step 4: Preparation of 2-((5-(1-(((4-(trifluoromethyl)benzyl)oxy)imino)ethyl)pyridin-2-yl)oxy)acetic acid.

To a solution of the product of step 3 (2.0 gm, 5.2 mmoles) in a mixture of THF (12 mL), methanol (4 mL) and water (4 mL), lithium hydroxide (440 mg, 10.5 mmoles) was added and the reaction mixture was stirred at ambient temperature for 4 hours. The solvents were evaporated under reduced pressure. The residue was dissolved in water, acidified with 1N HCl and extracted with ethyl acetate. The combined ethyl acetate extract was washed with water & brine, dried over sodium sulphate and evapourated under reduced pressure to yield 1.7 gm (88%) of title product as white solid.
¹H NMR: DMSO-*d*₆, *δ* 2.24 (s, 3H), 4.84 (s, 2H), 5.29 (s, 2H), 6.93 (d, *J* = 8.8 Hz, 1H), 7.60 (d, *J* = 8.0 Hz, 2H), 7.73 (d, *J* = 8.0 Hz, 2H), 7.98 (dd, *J* = 8.8 & 2.8 Hz, 1H), 8.36 (d, *J* = 2.4 Hz, 1H).

### Step 5: Preparation of 2-((5-(1-(((4-(trifluoromethyl)benzyl)oxy)imino)ethyl)pyridin-2-yl)oxy)acetamide.

A solution of 2-((5-(1-(((4-(trifluoromethyl)benzyl)oxy)imino)ethyl)pyridin-2-yl)oxy)acetic acid (1.5 g, 4.07 mmol) in DMF (10 ml) was placed into round-bottomed flask. EDC (0.937g, 4.89 mmol) and HOBT (0.936 g, 6.11 mmol) was added to the reaction mixture followed by addition of N-ethylmorpholine (1.251 ml, 9.88 mmol) and ammonia gas was passed into the reaction mixture at 10 °C. Then the reaction mixture was stirred at 27 °C for 2 hours. The reaction mixture was poured into H₂O (100 mL) and extracted with ethyl acetate (2 x 50 mL). The combined organic layers was washed with H₂O (50 mL) & brine solution (75 mL), dried over Na2SO4 and evaporated on rotavapor under vacuum to yield 750 mg (50 %) of product as white solid.

### Step 6: Preparation of 2-((5-(1-(((4-(trifluoromethyl)benzyl)oxy)imino)ethyl)pyridin-2-yl) oxy)acetonitrile.

A solution of 2-((5-(1-(((4-(trifluoromethyl)benzyl)oxy)imino)ethyl)pyridin-2-yl)oxy) acetamide (750 mg, 2.04 mmol) in dichloromethane (10 mL) and DMSO (0.435 mL) was placed into round-bottomed flask. Oxalyl chloride (0.357 mL, 4.08 mmol) was added to the reaction mixture followed by triethylamine (0.996 mL, 7.15 mmol) under nitrogen atmosphere at -78 °C. The reaction mixture was stirred under nitrogen atmosphere at 25 °C for 2 hours. The reaction mixture was poured into H₂O (150 mL) and extracted with ethyl acetate (2 x 50 mL). The combined organic layer was washed with H₂O (50 mL) & brine solution (50 mL), dried over Na₂SO₄ and evaporated on rotavapor under vacuum. The crude product was purified by column chromatography (230-400 silica gel column, Elution: - 8% EtOAc in Hexane) to yield 230 mg (32%) of product as pale yellow solid.

### Step 7: Preparation of 1-(6-((1H-tetrazol-5-yl)methoxy)pyridin-3-yl)ethanone O-(4-(trifluoromethyl)benzyl) oxime.

Title compound was prepared from 2-((5-(1-(((4-(trifluoromethyl)benzyl)oxy)imino)ethyl)pyridin-2-yl)oxy)acetonitrile by a similar procedure described in example 1 step 5.
¹H NMR: DMSO-*d*₆, *δ* 2.25 (s, 3H), 5.29 (s, 2H), 5.69 (s, 2H), 6.97 (d, *J* = 8.4 Hz, 1H), 7.61 (d, *J* = 8.0 Hz, 2H), 7.74 (d, *J* = 8.0 Hz, 2H), 8.02 (d, *J* = 8.8 Hz, 1H), 8.40 (d, *J* = 2.0 Hz, 1H).

### Example 5

### 1-(4-((1H-1,2,4-triazol-1-yl)methoxy)phenyl)ethanone O-(4-(trifluoromethyl)benzyl)oxime.

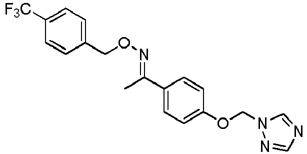

### Step 1.Preparation of (1H-1,2,4-triazol-1-yl)methanol.

A mixture of 1H-1,2,4-triazole (7.04 g, 102 mmol), paraformaldehyde (3.06 g, 102 mmol) and catalytic amount of triethylamine was heated to molten condition and stirred at the same temperature (140 °C) for 0.5 hour. Reaction mixture was then cooled to 30°C to obtain the product (9.0 g, 89 %) as white solid.

### Step 2. Preparation of 1-(chloromethyl)-1H-1,2,4-triazole.

To a solution of (1H-1,2,4-triazol-1-yl)methanol (1.0 g, 10.09 mmol) in dichloroethane (20 ml) thionyl chloride (1.105 ml, 15.14 mmol) was added and the reaction mixture was stirred at 100 °C for 4 hours. The reaction mixture was cooled to 30°C, poured into H₂O (25 mL) and extracted with dichloromethane (3 x 20 mL). The combined organic layers was washed with H₂O (50 mL) & brine solution (50 mL), dried over Na₂SO₄ and evaporated in rotavapor under vacuum to yield 1-(chloromethyl)-1H-1,2,4-triazole (0.570 g, 48 %) as brown liquid. **Step 3:** Preparation of 1-(4-((1H-1,2,4-triazol-1-yl)methoxy)phenyl)ethanone O-(4-(trifluoromethyl)benzyl) oxime.

To a solution of 1-(4-hydroxyphenyl)ethanone O-(4-(trifluoromethyl)benzyl) oxime (300 mg, 0.970 mmol) and 1-(chloromethyl)-1H-1,2,4-triazole (171 mg, 1.455 mmol) in DMF (5.0 ml) cesium carbonate (632 mg, 1.940 mmol) was added and the reaction mixture was stirred at 80 °C for 2 hours. The reaction mixture was poured into H₂O (50 mL) and extracted with ethyl acetate (3 x 25 mL). The combined organic layers were washed with H₂O (50 mL) & brine solution (50 mL), dried over Na₂SO₄ and evaporated on rotavapor under vacuum. The crude product was purified by flash chromatography using EtOAc in Hexane as eluent to yield the title compound (297 mg, 77 %) as yellow liquid.
¹H NMR: CDCl₃, *δ* 2.24 (s, 3H), 5.26 (s, 2H), 6.06 (s, 2H), 6.99-7.03 (m, 2H), 7.50 (d, *J* = 11.6 Hz, 2H), 7.57-7.62 (m, 4H), 7.99 (s, 1H), 8.27 (s, 1H).

### Example 6

### 1-(4-((1H-1,2,3-triazol-5-yl)methoxy)phenyl)ethanone O-(4-(trifluoromethyl)benzyl) oxime.

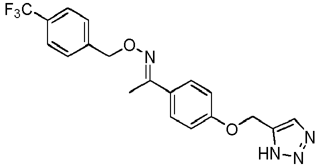

### Step 1. Preparation of 1-(4-(prop-2-yn-1-yloxy)phenyl)ethanone O-(4-(trifluoromethyl) benzyl) oxime.

To a solution of 1-(4-hydroxyphenyl)ethanone O-(4-(trifluoromethyl)benzyl) oxime (3.75 g, 12.12 mmol) and propargylchloride (1.861 ml, 18.19 mmol) in DMF (5 mL) cesium carbonate (7.90 g, 24.25 mmol) was added and the reaction mixture was stirred at 30 °C for 1hour. The reaction mixture was poured into H₂O (100 mL) and extracted with ethyl acetate (3 x 50 mL). The combined organic layers was washed with H₂O (75 mL) & brine solution (100 mL), dried over Na₂SO₄ and evaporated on rotavapor under vacuum to yield 3.47 g, (82 %) of product as pale yellow solid.

### Step 2. Preparation of 1-(4-((1H-1,2,3-triazol-5-yl)methoxy)phenyl)ethanone O-(4-(trifluoromethyl)benzyl) oxime.

To a solution of 1-(4-(prop-2-yn-1-yloxy)phenyl)ethanone O-(4-(trifluoromethyl)benzyl) oxime (0.400 g, 1.152 mmol) in a mixture of DMF (10 mL) & methanol (1 mL) copper(I) iodide (10.97 mg, 0.058 mmol) was added followed by trimethyl silylazide (0.229 ml, 1.727 mmol) and the reaction mixture was stirred at 100 °C for 4 hours. The reaction mixture was poured into H₂O (50 mL) and extracted with ethyl acetate (3 x 50 mL). The combined organic layers were washed with H₂O (50 mL) & brine solution (50 mL), dried over Na₂SO₄ and evaporated on rotavapor under vacuum. The crude product obtained was purified by flash chromatography using 20% EtOAc in Hexane as eluent to yield 0.4 g, (88 %) of pure product as white solid.
¹H NMR: MeOD, *δ* 2.26 (s, 3H), 5.22 (s, 2H), 5.26 (s, 2H), 7.00 (dd, *J* = 8.8 & 3.2 Hz, 2H), 7.56-7.62 (m, 4H), 7.65 (d, *J=* 8.4 Hz, 2H), 7.88 (s, 1H).

### Example 7

### 1-(4-((5-Methyl-1,3,4-oxadiazol-2-yl)methoxy)phenyl)ethanone O-(4-(trifluoromethyl) benzyl)oxime.

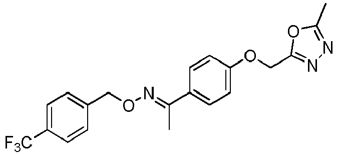

### Step 1: Preparation of 2-(4-(1-(((4-(trifluoromethyl)benzyl)oxy)imino)ethyl)phenoxy)acetic acid.

To a solution of 1-(4-hydroxyphenyl)ethanone O-(4-(trifluoromethyl)benzyl) oxime (2.77 g, 8.96 mmol) in DMF was added cesium carbonate (5.84 g, 17.92 mmol) followed by ethyl-2-chloroacetate (1.32 g, 10.75 mmol) and the reaction mixture was stirred at ambient temperature for 15 hours. The reaction mixture was poured into water (100 mL) and extracted with ethyl acetate (2 X 50 mL). The combined organic layer was washed with water (50 mL) & brine solution (50 mL), dried over sodium sulfate and evaporated on rotavapor under vacuum to yield ethyl-2-(4-(1-(((4-(trifluoromethyl)benzyl)oxy)imino) ethyl)phenoxy)acetate as brown liquid. Which was hydrolysed by adding a solution of lithium hydroxide hydrate (3.61 g, 86 mmol) in water (20 ml) to the solution of this ester (17 g, 43 mmol) in THF (60 mL). The reaction mixture was stirred for 30 °C for 3 hours. The solvent was evaporated and the residue was diluted with water (100 mL) and acidified by dil HCl (pH 2-3). Solid product was filtered washed with water and dried over P₂O₅ under vacuum to yield 2.83 g (80 %) of 2-(4-(1-(((4-(trifluoromethyl)benzyl) oxy)imino)ethyl)phenoxy)acetic acid as white solid.

### Step 2: Preparation of N'-acetyl-2-(4-(1-(((4-(trifluoromethyl)benzyl)oxy)imino)ethyl) phenoxy)acetohydrazide.

To a solution of 2-(4-(1-(((4-(trifluoromethyl)benzyl)oxy)imino)ethyl)phenoxy)acetic acid (1.0 g, 2.72 mmol), in DMF (5 ml), acetohydrazide (0.222 g, 2.99 mmol), 1H-benzo[d][1,2,3]triazol-7-ol (0.552 g, 4.08 mmol), N1-((ethylimino)methylene)-N3,N3-dimethylpropane-1,3-diamine hydrochloride (0.626 g, 3.27 mmol) and 4-ethylmorpholine (1.034 ml, 8.17 mmol) were added and the reaction mixture was stirred at 30 °C for 3 hours. The reaction mixture was poured into water (100 mL), solid separated was filtered, washed with water and dried over P₂O₅ under vacuum to yield N'-acetyl-2-(4-(1-(((4-(trifluoromethyl)benzyl)oxy)imino)ethyl)phenoxy)acetohydrazide (1.1 g, 95 %) as white solid.

### Step 3. Preparation of 1-(4-((5-methyl-1,3,4-oxadiazol-2-yl)methoxy)phenyl)ethanone O-(4-(trifluoromethyl)benzyl) oxime.

To a solution of N'-acetyl-2-(4-(1-(((4-(trifluoromethyl)benzyl)oxy)imino)ethyl) phenoxy)acetohydrazide (0.500 g, 1.181 mmol) in Toluene (5 ml), thionyl chloride (0.190 ml, 2.60 mmol) was added. The reaction mixture was stirred at 100 °C for 4 hours. The reaction mixture was poured into water (50 mL) and extracted by ethyl acetate. The combined organic layers was washed with water (3 x 25 mL) & brine (25 mL), dried over sodium sulfate and evaporated in rotavapor. The crude product was purified by column chromatography (230-400 silica gel column, Elution: - 22 % EtOAc in Hexane) to yield pure 1-(4-((5-methyl-1,3,4-oxadiazol-2-yl)methoxy)phenyl)ethanone O-(4-(trifluoromethyl) benzyl) oxime (0.250 g, 48.9 %) as off white solid.
¹H NMR: CDCl₃, *δ* 2.25 (s, 3H), 2.55 (s, 3H), 5.23 (s, 2H), 5.26 (s, 2H), 6.97-7.00 (m, 2H), 7.50 (d, *J=* 8.0 Hz, 2H), 7.57-7.62 (m, 4H).

### Example 8

### 1-(4-((5-Ethyl-1,3,4-thiadiazol-2-yl)methoxy)phenyl)ethanone O-(4-(trifluoromethyl) benzyl) oxime.

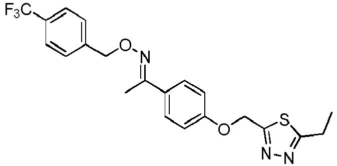

### Step 1: Preparation of 2-(4-(1-(((4-(trifluoromethyl)benzyl)oxy)imino)ethyl)phenoxy) acetohydrazide

To a solution of ethyl 2-(4-(1-(((4-(trifluoromethyl)benzyl)oxy)imino)ethyl)phenoxy)acetate (0.500 g, 1.265 mmol) in Ethanol (5 mL), hydrazine hydrate (0.615 ml, 12.65 mmol) was added and the reaction mixture was stirred at 30 °C for 5 minutes. Solid separated was filtered and dried to Yield 2-(4-(1-(((4-(trifluoromethyl)benzyl)oxy)imino)ethyl)phenoxy) acetohydrazide (0.400 g, 83 %) as white solid.

### Step 2: Preparation of N-ethyl-2-(2-(4-(1-(((4-(trifluoromethyl)benzyl)oxy)imino)ethyl) phenoxy)acetyl)hydrazinecarbothioamide

To a solution of 2-(4-(1-(((4-(trifluoromethyl)benzyl)oxy)imino)ethyl)phenoxy) acetohydrazide (0.500 g, 1.311 mmol) in EtOH (15 mL), ethyl isothiocyanate (0.121 ml, 1.377 mmol) was added and the reaction mixture was stirred for 100°C for 3 hours. The reaction mixture was cooled to 30°C. White solid separated was filtered, washed with ethanol to yield N-ethyl-2-(2-(4-(1-(((4-(trifluoromethyl)benzyl)oxy)imino)ethyl)phenoxy) acetyl)hydrazinecarbothioamide (0.480 g, 78 %) as white solid.

### Step 3. Preparation of 1-(4-((5-ethyl-1, 3, 4-thiadiazol-2-yl) methoxy)phenyl)ethanone O-(4-(trifluoromethyl)benzyl) oxime,

To ice cold Sulfuric acid (5.0 ml, 94 mmol), N-ethyl-2-(2-(4-(1-(((4-(trifluoromethyl) benzyl) oxy)imino)ethyl)phenoxy)acetyl)hydrazine carbothioamide (0.480 g, 1.025 mmol) was added portion wise. The reaction mixture was stirred at 0°C for 2 hours. The reaction mixture was poured into water (50 mL), extracted with ethyl acetate (3 x 25 mL). The combined organic layer was washed with sat NaHCO₃ & water (50 mL), dried over sodium sulfate and evaporated on rotavapor under vacuum. The crude product was purified by column chromatography (230-400 silica gel column, Eluent: - 32 % EtOAc in Hexane) to yield pure 1-(4-((5-ethyl-1,3,4-thiadiazol-2-yl)methoxy)phenyl)ethanone O-(4-(trifluoromethyl)benzyl) oxime (240 mg, 52.0 %) as white solid.
¹H NMR: CDCl₃, *δ* 1.30 (t, *J=* 7.2 Hz, 3H), 2.24 (s, 3H), 3.36 (q, *J* = 6.8 Hz, 2H), 5.25 (s, 2H), 5.31 (s, 2H), 6.97 (d, *J* = 8.8 Hz, 2H), 7.50 (d, *J* = 8.0 Hz, 2H), 7.52-7.62 (m, 4H).

### Example 9

### 1-(4-((1,3,4-Thiadiazol-2-yl)methoxy)phenyl)ethanone O-(4-(trifluoromethyl)benzyl)oxime.

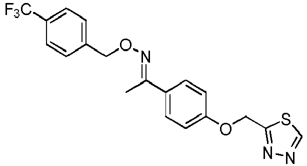

Placed 2-(4-(1-(((4-(trifluoromethyl)benzyl)oxy)imino)ethyl)phenoxy)acetohydrazide (0.600 g, 1.573 mmol), Triethyl orthoformate (0.288 ml, 1.731 mmol) in the vessel. Then in this Phosphorus pentasulfide (0.839 g, 1.888 mmol)/alumuna (Neutral, 839 mg) was added and the reaction mixture was stirred at 40 °C for 30 min in microwave. Ethyl acetate was added to the reaction mixture and solid inorganic material was filtered off. The filtrate was evaporated to yield crude product as yellow liquid. The crude product was purified by column chromatography (230-400 silica gel column, Eluent: - 20% EtOAc in Hexane) to yield pure 1-(4-((1,3,4-thiadiazol-2-yl)methoxy)phenyl)ethanone O-(4-(trifluoromethyl)benzyl) oxime (0.300 g, 45.6 %) as off white solid.
¹H NMR: CDCl₃, *δ* 2.25 (s, 3H), 5.26 (s, 2H), 5.56 (s, 2H), 6.96-6.99 (m, 2H), 7.50 (d, *J* = 8.0 Hz, 2H), 7.58-7.62 (m, 4H), 9.16 (s, 1H).

### Example 10

### 3-((4-(1-(((4-(Trifluoromethyl)benzyl)oxy)imino)ethyl)phenoxy)methyl)-1,2,4-oxadiazol-5(4H)-one.

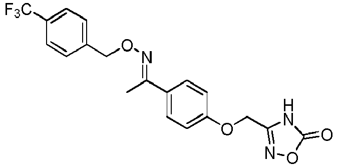

### Step 1: Preparation of N-hydroxy-2-(4-(1-(((4-(trifluoromethyl)benzyl)oxy)imino)ethyl) phenoxy)acetimidamide.

To a solution of 2-(4-(1-(((4-(trifluoromethyl)benzyl)oxy)imino)ethyl)phenoxy)acetonitrile (1.0 g, 2.87 mmol) in ethanol (20 mL), hydroxyl amine hydrochloride (0.299 g, 4.31 mmol) and potassium carbonate (2.381 g, 17.23 mmol) were added and the reaction mixture was stirred at 100 °C for 4 hours. The reaction mixture was cooled to 30°C and the ethanol was evaporated. Water (100 mL) was added to the residue and extracted with ethyl acetate (3 x 30 mL). Combined organic layer was washed with water (50 mL), brine solution (50 mL), dried over sodium sulphate and evaporated under vacuum. The crude product was purified by column chromatography (230-400 silica gel column, Eluent: - 35% EtOAc in Hexane) to yield pure N-hydroxy-2-(4-(1-(((4-(trifluoromethyl)benzyl)oxy)imino)ethyl)phenoxy) acetimidamide (650 mg, 59.4 %) as white solid.

### Step 2: Preparation of phenyl (1-(hydroxyimino)-2-(4-(1-(((4-(trifluoromethyl)benzyl)oxy) imino)ethyl)phenoxy)ethyl)carbamate.

To a solution of N-hydroxy-2-(4-(1-(((4-(trifluoromethyl)benzyl)oxy)imino)ethyl) phenoxy)acetimidamide (0.150 g, 0.393 mmol) in dichloromethane (10 mL), triethylamine (0.082 ml, 0.590 mmol) was added followed by Phenyl chloroformate (0.054 ml, 0.433 mmol). The reaction mixture was stirred at 30 °C for 30 minutes. The reaction mixture was poured into water (25 mL) and extracted with dichloromethane (3 x 20 mL). The combined organic layer was washed with water () & brine (25 mL), dried over sodium sulfate and evaporated in rotavapor to yield phenyl (-1-(hydroxyimino)-2-(4-(1-(((4-(trifluoromethyl)benzyl)oxy)imino)ethyl)phenoxy)ethyl)carbamate (190 mg, 96 %) as white solid.

### Step 3: Preparation of 3-((4-(1-(((4-(trifluoromethyl)benzyl)oxy)imino)ethyl)phenoxy) methyl)-1,2,4-oxadiazol-5(4H)-one.

A solution of N-((phenoxycarbonyl)oxy)-2-(4-(1-(((4-(trifluoromethyl)benzyl)oxy)imino) ethyl)phenoxy)acetimidamide (720 mg, 1.436 mmol) in Toluene (7 mL) was refluxed for 30 hours. The reaction mixture was cooled to 30°C and the toluene was evaporated to yield crude product as liquid. The crude product was purified by column chromatography (230-400 silica gel column, Eluent: - 30% EtOAc in Hexane) to yield pure 3-((4-(1-(((4-(trifluoromethyl)benzyl)oxy)imino)ethyl)phenoxy)methyl)-1,2,4-oxadiazol-5(4H)-one (0.250 g, 40.4 %) as yellow solid.
¹H NMR: CDCl₃, *δ* 2.27 (s, 3H), 5.03 (s, 2H), 5.28 (s, 2H), 6.95 (d, *J* = 8.8 Hz, 2H), 7.52 (d, J = 8.0 Hz, 2H), 7.62-7.64 (m, 4H).

### Example 11

### 5-((4-(1-(((4-(Trifluoromethyl)benzyl)oxy)imino)ethyl)phenoxy)methyl)oxazolidine-2,4-dione.

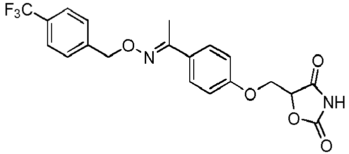

### Step 1: Preparation of methyl 2-hydroxy-3-(4-(1-(((4-(trifluoromethyl)benzyl)oxy)immo) ethyl)phenoxy)propanoate.

A mixture of 1-(4-hydroxyphenyl)ethanone O-(4-(trifluoromethyl)benzyl) oxime (5.0 g, 16.17 mmol), methyl oxirane-2-carboxylate (3.30 g, 32.3 mmol) and dimethylaminopyridine (0.198 g, 1.617 mmol) was stirred at 120 °C for 8 hours. The reaction mixture was purified by column chromatography (230-400 silica gel column, Eluent: - 15% EtOAc in Hexane) to yield pure methyl 2-hydroxy-3-(4-(1-(((4-(trifluoromethyl)benzyl)oxy)imino) ethyl)phenoxy)propanoate (3.5 g, 52.6 %) as white solid.

### Step 2: Preparation of 2-hydroxy-3-(4-(1-(((4-(trifluoromethyl)benzyl)oxy)imino) ethyl)phenoxy)propanoic acid.

To a solution of methyl 2-methyl-3-(4-(1-(((4-(trifluoromethyl)benzyl)oxy)imino) ethyl)phenoxy)propanoate (1.0 g, 2.443 mmol) in MeOH (15 ml), solution of NaOH (0.195 g, 4.89 mmol) in water (5 mL) was added. The reaction mixture was stirred at 27 °C for 2 hours. Volatile material was evaporated and the residue was poured into water (50 mL), solid separated was filtered, washed with water and dried over P₂O₅ to yield 2-hydroxy-3-(4-(1-(((4-(trifluoromethyl)benzyl)oxy)imino)ethyl)phenoxy)propanoic acid (850mg, 88 %) as white solid.

### Step 3: Preparation of 2-hydroxy-3-(4-(1-(((4-(trifluoromethyl)benzyl)oxy)imino) ethyl)phenoxy)propanamide.

To a solution of 2-hydroxy-3-(4-(1-(((4-(trifluoromethyl)benzyl)oxy)imino) ethyl)phenoxy)propanoic acid (850 mg, 2.139 mmol) in DMF (10 mL), N1-((ethylimino)methylene)-N3,N3-dimethylpropane-1,3-diamine hydrochloride (492 mg, 2.57 mmol), 1H-benzo[d][1,2,3]triazol-7-ol (491 mg, 3.21 mmol) and N-ethylmorpholine (0.812 ml, 6.42 mmol) were added and ammonia gas was bubbled through reaction mixture at 0 °C for 10 minutes. Then reaction mixture was stirred at 27 °C for 2 hours. The reaction mixture was then poured into water (50 mL) and extracted with ethyl acetate (2 x 25 mL). The combined organic layer was washed with H₂O (50 mL) & brine solution (25 mL), dried over Na₂SO₄ and evaporated on rotavapor under vacuum. The crude product obtained was purified by column chromatography (230-400 silica column, Eluent: - 60% EtOAc in Hexane) to yield 300 mg, (35%) of pure product as white solid.

### Step 4: Preparation of 5-((4-(1-(((4-(trifluoromethyl)benzyl)oxy)imino)ethyl)phenoxy) methyl)oxazolidine-2,4-dione.

To a solution of 2-hydroxy-3-(4-(1-(((4-(trifluoromethyl)benzyl)oxy)imino)ethyl) phenoxy)propanamide (160mg, 0.404 mmol) in THF (5 mL), 1,1'-Carbonyldiimidazole (98 mg, 0.606 mmol) was added. The reaction mixture was stirred at 70 °C for 15 hours. The reaction mixture was poured into water (25 mL) and extracted with ethyl acetate (2 x 15 mL). The combined organic layer was washed with H₂O (25 mL) & brine solution (15 mL), dried over Na₂SO₄ and evaporated on rotavapor. The crude product was purified by column chromatography (230-400 silica gel column, Eluent: -15% EtOAc in Hexane) to yield 90 mg, (52%) pure 5-((4-(1-(((4-(trifluoromethyl)benzyl)oxy)imino)ethyl)phenoxy)methyl) oxazolidine-2,4-dione as white solid.
¹H NMR: DMSO-*d*₆, *δ* 2.22 (s, 3H), 4.41 (s, 2H), 5.27 (s, 2H), 5.38 (s, 1H), 6.95 (d, *J* = 8.8 Hz, 2H), 7.58-7.61 (m, 4H), 7.73 (d, *J=* 8.0 Hz, 2H).

### Example 12

### 3-(5-(((5-(1-(((4-(Trifluoromethyl)benzyl)oxy)imino)ethyl)pyridin-2-yl)oxy)methyl)-2H-tetrazol-2-yl)propanoic acid.

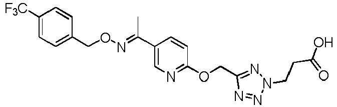

### Step 1: Preparation of 3-(5-(((5-(1-(((4-(Trifluoromethyl)benzyl)oxy)imino)ethyl)pyridin-2-yl)oxy)methyl)-2H-tetrazol-2-yl)propanoic acid ethyl ester.

To a solution of (E)-1-(4-((1H-tetrazol-5-yl)methoxy)phenyl)ethanone O-(4-(trifluoromethyl)benzyl) oxime (1.5 g, 3.83 mmol) in DMF (8 mL) was added cesiumcarbonate (2.5 g, 7.67 mmol) followed by ethyl 3-bromopropionate (0.74 mL, 5.75 mmol) and the reaction mixture was stirred at ambient temperature for 6 hours. Water was added to the reaction mixture and extracted with ethyl acetate (3 X 50 mL). Combined organic extract was washed with water & brine solution, dried over sodium sulphate and evaporated on rotavopor under reduced pressure to yield thick brown liquid. The crude product was purified by column chromatography (Eluent: 17% ethyl acetate in hexane) to yield 0.5 g (26 %) of product as thick liquid.

### Step 2: Preparation of 3-(5-(((5-(1-(((4-(Trifluoromethyl)benzyl)oxy)imino)ethyl)pyridin-2-yl)oxy)methyl)-2H-tetrazol-2-yl)propanoic acid.

To a solution of 3-(5-(((5-(1-(((4-(Trifluoromethyl)benzyl)oxy)imino)ethyl)pyridin-2-yl)oxy)methyl)-2H-tetrazol-2-yl)propanoic acid ethyl ester (0.5 g, 1.01 mmol) in ethanol (5 mL) were added a solution of sodium hydroxide (51 mg, 2.02 mmol) and the reaction mixture was stirred at 25 °C for 18 hours. Water was added to the reaction mixture, and acidified by the addition of dilute HCl. The solid separated was filtered under suction and dried under vacuum to yield 0.1 g (22 %) of product as white solid.
¹H NMR: DMSO-*d*₆, *δ* 2.25 (s, 3H), 2.99 (t, *J* = 6.4 Hz, 2H), 4.82 (t, *J* = 6.4 Hz, 2H), 5.28 (s, 2H), 5.61 (s, 2H), 6.91 (dd, *J* = 8.8 & 0.8 Hz, 1H), 7.60 (d, *J* = 8.0 Hz, 2H), 7.72 (d, *J =* 8.0 Hz, 2H), 7.99 (dd, *J* = 8.6 & 2.6 Hz, 1H), 8.41 (d, *J* = 2.0 Hz, 1H).

### Example 13

### 1-(4-((5-(Hydroxymethyl)furan-2-yl)methoxy)phenyl)ethanone O-(4-(trifluoromethyl) benzyl) oxime.

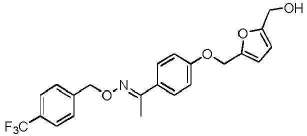

### Step1. Preparation of 5-(chloromethyl)furan-2-carbaldehyde.

To a solution of D-(-)-Fructose (3.6 g, 19.98 mmol) in Toluene (30 ml) and Water (0.240 ml), Magnesium chloride (1.903 g, 19.98 mmol) was added and the reaction mixture was stirred at 75 °C for 30 min. Concentrated HCl (6.28 gm) was added and the mixture was continued to stirr for 1 hour. The solid residue was filteered, washed with toluene (100 ml) and the organic layer was washed with brine (2 X 100 ml) and dried over sodium sulfate. The solvent was removed under reduced pressure to yield 5-(chloromethyl)furan-2-carbaldehyde (1.5 g, 51.9 %) as Brown oil.
¹H NMR: CDCl₃, *δ* 4.61 (s, 2H), 6.58 (d, *J=* 3.6 Hz, 1H), 7.20 (d, *J=* 3.6 Hz, 1H)

### Step2. Preparation of 5-((4-(1-(((4-(trifluoromethyl)benzyl)oxy)imino)ethyl)phenoxy) methyl)furan-2-carbaldehyde.

To a solution of 1-(4-hydroxyphenyl)ethanone O-(4-(trifluoromethyl)benzyl) oxime (0.750 g, 2.425 mmol) and 5-(chloromethyl)furan-2-carbaldehyde (0.456 g, 3.15 mmol) in DMF (10 ml), Cesium carbonate (1.580 g, 4.85 mmol) was added and the reaction mixture was stirred at 30 °C for 15 hours. The reaction mixture was poured into water (50 mL) and extracted with ethyl acetate. The combined organic layers was washed with water & brine, dried over sodium sulfate and evaporated in rotavapor under vacuum. The crude product was purified by column chromatography (230-400 silica gel column, Elution: - 10% EtOAc in Hexane) to yield pure 5-((4-(1-(((4-(trifluoromethyl)benzyl)oxy)imino) ethyl)phenoxy)methyl)furan-2-carbaldehyde (0.250 g, 24.70 %) as yellow solid.
¹H NMR: CDCl₃, *δ*, 2.27 (s, 3H), 5.13 (s, 2H), 5.27 (s, 2H), 6.62 (d, *J* = 3.6 Hz, 1H), 6.94-6.97 (m, 2H), 7.23 (d, *J* = 3.6 Hz, 1H), 7.52 (d, *J* = 8.0 Hz, 2H), 7.58-7.63 (m, 4H), 9.66 (s, 1H).

### Step3. Preparation of 1-(4-((5-(hydroxymethyl)furan-2-yl)methoxy)phenyl)ethanone O-(4-(trifluoromethyl)benzyl) oxime.

To a solution of 5-((4-(1-(((4-(trifluoromethyl)benzyl)oxy)imino)ethyl)phenoxy)methyl) furan-2-carbaldehyde (0.250 g, 0.599 mmol) in MeOH (10 ml), Sodium borohydride (0.023 g, 0.599 mmol) was added at 0°C and the reaction mixture was stirred at 30 °C for 30 min. Methanol from the reaction mixture was evaporated under reduced pressure and the residue was extracted with ethyl acetate (2 x 20 mL). The combined organic layers was washed with water (2 x 20 mL) & brine (25 mL), dried over sodium sulfate and evaporated in rotavapor under vacuum to yield 1-(4-((5-(hydroxymethyl)furan-2-yl)methoxy)phenyl)ethanone O-(4-(trifluoromethyl)benzyl) oxime (0.225 g, 88 %) as white solid.
¹H NMR: DMSO-*d*₆, *δ* 1.74 (t, *J* = 6 Hz, 1H), 2.27 (s, 3H), 4.63 (d, *J* = 6.0 Hz, 2H), 5.00 (s, 2H), 5.27 (s, 2H), 6.29 (d, J = 3.2 Hz, 1H), 6.39 (d, *J* = 3.2 Hz, 1H), 6.94-3.98 (m, 2H), 7.52 (d, *J* = 8.0 Hz, 2H), 7.57-7.63 (m, 4H).

### Example 14

### 5-((4-(1-(((4-(Trifluoromethyl)benzyl)oxy)imino)ethyl)phenoxy)methyl)furan-2-carboxylic acid.

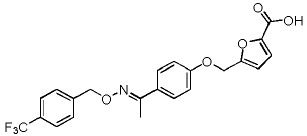

To a solution of 5-((4-(1-(((4-(trifluoromethyl)benzyl)oxy)imino)ethyl)phenoxy)methyl) furan-2-carbaldehyde (0.500 g, 1.198 mmol) in Acetone (10 ml), another solution of sodium bicarbonate (0.101 g, 1.198 mmol) in water (10.00 ml) was added followed by addition of a solution of KMnO₄ (0.038 g, 0.240 mmol) in water (2.00 ml) at 30°C. Then reaction mixture was stirred at 30 °C for 4 hours. Inorganic materials were filtered and the filtrate was concentrated under reduced pressure and aqueous layer was acidified and extracted by ethyl acetate (3 x 20 mL). The combined organic layers was washed with water (2 x 25 mL) & brine (25 mL), dried over sodium sulfate and evaporated in rotavapor under vacuum. The crude product was purified by column chromatography (230-400 silica gel column, Elution: - 1% MeOH in CHCl3) to yield pure 5-((4-(1-(((4-(trifluoromethyl)benzyl)oxy)imino)ethyl)phenoxy)methyl)furan-2-carboxylic acid (0.160 g, 29.7 %) as white solid.
¹H NMR: DMSO-*d*₆, *δ* 2.23 (s, 3H), 5.15 (s, 2H), 5.27 (s, 2H), 6.74 (d, *J* = 3.2 Hz, 1H), 7.05 (dd, *J* = 6.8 & 2.0 Hz, 2H), 7.16 (d, *J=* 3.2 Hz, 1H), 7.58-7.62 (m, 4H), 7.73 (d, *J* = 8.0 Hz, 2H).

The following examples were prepared following the general procedures given in Examples **1-14,** with suitable modifications, alterations and other process variations which are within the scope of a person skilled in the art.

### Example 15

### 1-(4-((2-(tert-Butyl)-5-methyloxazol-4-yl)methoxy)phenyl)ethanone O-(4-(trifluoromethyl) benzyl) oxime.

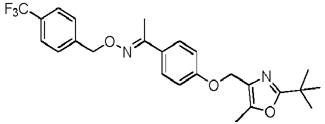

¹H NMR: CDCl₃, *δ* 1.36 (s, 9H), 2.26 (s, 3H), 2.31 (s, 3H), 4.89 (s, 2H), 5.26 (s, 2H), 6.95 (d, *J* = 6.8 Hz, 2H), 7.50 (d, *J* = 8.0 Hz, 2H), 7.56-7.62 (m, 4H).

### Example 16

### 1-(4-((5-Methyl-4H-1,2,4-triazol-3-yl)methoxy)phenyl)ethanone O-(4-(trifluoromethyl) benzyl) oxime.

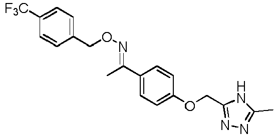

¹H NMR: CDCl₃, *δ* 2.25 (S, 3H), 2.49 (s, 3H), 5.16 (s, 2H), 5.25 (s, 2H), 6.99 (d, *J* = 8.8 Hz, 2H), 7.50 (d, *J* = 8.0 Hz, 2H), 7.56-7.62 (m, 4H).

### Example 17

### 1-(6-((5-Methyl-4H-1,2,4-triazol-3-yl)methoxy)pyridin-3-yl)ethanone O-(4-(1,1-difluoroethyl) benzyl)oxime.

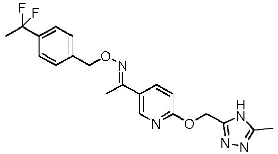

¹H NMR: CDCl₃, *δ* 1.93 (t, *J* = 18.0 Hz, 3H), 2.26 (s, 3H), 2.48 (s, 3H), 5.25 (s, 2H), 5.48 (s, 2H), 6.84 (d, J = 8.8 Hz, 1H), 7.46 (d, *J* = 8.0 Hz, 2H), 7.52 (d, *J* = 8.4 Hz, 2H), 7.96 (dd, *J* = 8.8 & 2.4 Hz, 1H), 8.40 (d, *J* = 2.4 Hz, 1H).

### Example 18

### 1-(6-((5-Methyl-4H-1,2,4-triazol-3-yl)methoxy)pyridin-3-yl)ethanone O-(4-(trifluoromethyl) benzyl)oxime.

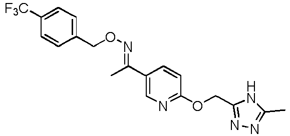

¹H NMR: CDCl₃, *δ* 2.28 (s, 3H), 2.48 (s, 3H), 5.27 (s, 2H), 5.48 (s, 2H), 6.84 (d, *J* = 8.8 Hz, 1H), 7.51 (d, *J* = 8.4 Hz, 2H), 7.63 (d, J = 8.0 Hz, 2H), 7.95 (dd, *J=* 8.4 & 2.4 Hz, 1H), 8.41 (d, *J* = 2.0 Hz, 1H).

### Example 19

### 1-(4-((3-Amino-1H-1,2,4-triazol-5-yl)methoxy)phenyl)ethanone O-(4-(trifluoromethyl) benzyl)oxime.

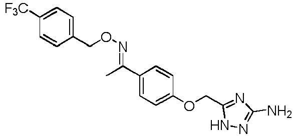

¹H NMR: DMSO-*d*₆, *δ* 2.22 (s, 3H), 4.84 (s, 2H), 5.26 (s, 2H), 5.96 (s, 2H), 7.02 (d, *J* = 8.8 Hz, 2H), 7.58 (dd, *J=* 18.8 & 8.0 Hz, 4H), 7.73 (d, *J=* 8.0 Hz, 2H), 11.95 (s, 1H).

### Example 20

### 1-(4-((5-Amino-1,3,4-oxadiazol-2-yl)methoxy)phenyl)ethanone O-(4-(trifluoromethyl) benzyl)oxime.

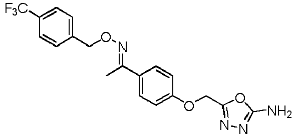

¹H NMR: MeOD, *δ* 2.26 (s, 3H), 5.13 (s, 2H), 5.27 (s, 2H), 7.09-7.04 (m, 2H), 7.57-7.66 (m, 6H).

### Example 21

### N-(5-((4-(1-(((4-(Trifluoromethyl)benzyl)oxy)imino)ethyl)phenoxy)methyl)-1,3,4-oxadiazol-2-yl)methanesulfonamide.

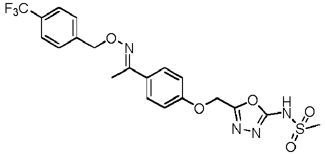

¹H NMR: CDCl₃, *δ* 2.26 (s, 3H), 3,09 (s, 3H), 5.06 (s, 2H), 5.26 (s, 2H), 6.94-6.99 (m, 2H), 7.50 (d, *J* = 8.0 Hz, 2H), 7.59-7.62 (m, 4H).

### Example 22

### 1-(4-((1H-tetrazol-5-yl)methoxy)phenyl)ethanone O-(4-(1,1-difluoroethyl)benzyl)oxime.

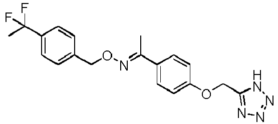

¹H NMR: DMSO-*d*₆, *δ* 1.92 (t, *J* = 18.2 Hz, 3H), 2.25 (s, 3H), 5.24 (s, 2H), 5.48 (s, 2H), 7.06 (dd, *J=* 7.2 & 2.0 Hz, 2H), 7.49 (d, *J=* 8.4 Hz, 2H), 7.55 (d, *J=* 8.4 Hz, 2H), 7.59-7.63 (m, 2H).

### Example 23

### 1-(2-((1H-tetrazol-5-yl)methoxy)phenyl)ethanone O-(4-(trifluoromethyl)benzyl)oxime.

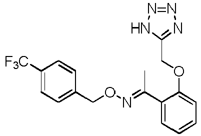

¹H NMR: DMSO-*d*₆, *δ* 2.13 (s, 3H), 5.22 (s, 2H), 5.42 (s, 2H), 6.95-6.99 (m, 1H), 7.18 (dd, *J* = 7.6 & 1.6 Hz, 1H), 7.24 (d, *J* = 8.0 Hz, 1H), 7.35-7.39 (m, 1H), 7.57 (d, *J*= 9.2 Hz, 2H), 7.71 (d, *J* = 8.0 Hz, 2H)

### Example 24

### 1-(4-((1H-tetrazol-5-yl)methoxy)phenyl)ethanone O-((6-(trifluoromethyl)pyridin-3-yl)methyl)oxime.

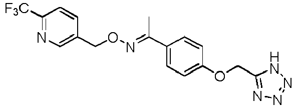

¹H NMR: DMSO-*d*₆, *δ* 2.21 (s, 3H), 5.12 (s, 2H), 5.31 (s, 2H), 7.05 (d, *J* = 6.8 Hz, 2H), 7.55 (d, *J* = 6.8 Hz, 2H), 7.91 (d, *J =* 8.0 Hz, 1H), 8.09 (d, *J* = 9.2 Hz, 1H), 8.80 (s, 1H).

### Example 25

### 1-(4-((1H-tetrazol-5-yl)methoxy)phenyl)ethanone O-(3-methoxy-4-(trifluoromethyl)benzyl) oxime.

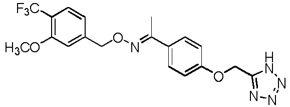

¹H NMR: DMSO-*d*₆, *δ* 2.22 (s, 3H), 3.90 (s, 3H), 5.22 (s, 2H), 5.35 (s, 2H), 7.06 (d, *J* = 8.8 Hz, 2H), 7.28-7.32 (m, 2H), 7.50 (d, *J* = 7.6 Hz, 1H), 7.58 (d, *J* = 8.8 Hz, 2H).

### Example 26

### 1-(6-((1H-tetrazol-5-yl)methoxy)pyridin-3-yl)ethanone O-(3-methoxy-4-(trifluoromethyl) benzyl) oxime.

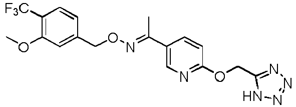

¹H NMR: DMSO-*d*₆, *δ* 2.25 (s, 3H), 3.90 (s, 3H), 5.24 (s, 2H), 5.71 (s, 2H), 6.96 (d, *J* = 8.4 Hz, 1H), 7.30 (d, *J* = 8.0 Hz, 2H), 7.51 (d, *J* = 8.0 Hz, 1H), 8.02 (dd, *J* = 8.8 & 2.4 Hz, 1H), 8.39 (d, *J* = 2.0 Hz, 1H).

### Example 27

### 1-(4-((1H-tetrazol-5-yl) methoxy)-2-hydroxyphenyl) ethanone O-(4-(trifluoromethyl) benzyl)oxime.

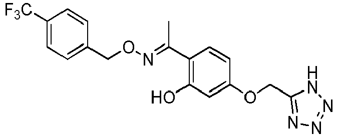

¹H NMR: DMSO-*d*₆, *δ* 2.29 (s, 3H), 5.30 (s, 2H), 5.46 (s, 2H), 6.55 (s, 1H), 6.58 (d, *J* = 8.8 Hz, 1H), 7.41 (d, *J* = 8.4 Hz, 1H), 7.62 (d, *J* = 8.0 Hz, 2H), 7.76 (d, *J* = 8.4 Hz, 2H), 10.84 (s, 1H).

### Example 28 (Does not form part of the invention)

### 1-(6-((1H-tetrazol-5-yl)methoxy)-1,3-dimethyl-1H-pyrazolo[3,4-b]pyridm-5-yl)ethanone O-(4-(trifluoromethyl)benzyl)oxime.

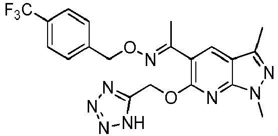

¹H NMR: DMSO-*d*₆, *δ* 2.18 (s, 3H), 2.41 (s, 3H), 3.85 (s, 3H), 5.27 (s, 2H), 5.76 (s, 2H), 7.60 (d, *J=* 8.0 Hz, 2H), 7.73 (d, *J=* 8.4 Hz, 2H), 7.99 (s, 1H).

### Example 29

### 1-(2-Hydroxy-4-((1-methyl-1H-tetrazol-5-yl)methoxy)phenyl)ethanone O-(4-(trifluoromethyl)benzyl) oxime.

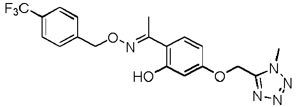

¹H NMR: DMSO-*d*₆, *δ* 2.29 (s, 3H), 4.37 (s, 3H), 5.30 (s, 2H), 5.35 (s, 2H), 6.53-6.59 (m, 2H), 7.40 (d, *J* = 8.4 Hz, 1H), 7.62 (d, *J* = 8.0 Hz, 2H), 7.76 (d, *J* = 8.0 Hz, 2H), 10.84(s, 1H).

### Example 30

### 1-(4-((1H-tetrazol-5-yl)methoxy)phenyl)ethanone O-benzyl oxime.

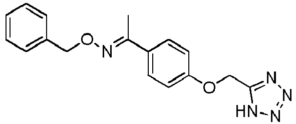

¹H NMR: DMSO-*d*₆, *δ* 2.18 (s, 3H), 5.16 (s, 2H), 5.21 (s, 2H), 7.05 (d, *J* = 8.4 Hz, 2H), 7.30-7.41 (m, 5H), 7.57 (d, *J* = 8.4 Hz, 2H).

### Example 31

### 1-(4-((1H-tetrazol-5-yl)methoxy)phenyl)ethanone O-(4-fluorobenzyl) oxime.

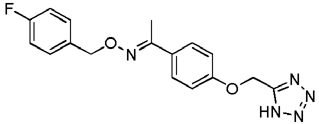

¹H NMR: DMSO-*d*₆, *δ* 2.17 (s, 3H), 5.14 (s, 4H), 7.06 (d, *J* = 8.4 Hz, 2H), 7.19 (t, *J* = 8.8 Hz, 2H), 7.45 (t, J = 6.4 Hz, 2H), 7.55 (d, *J* = 8.8 Hz, 2H).

### Example 32

### 1-(6-((1H-tetrazol-5-yl)methoxy)pyridin-3-yl)ethanone O-(4-fluorobenzyl) oxime.

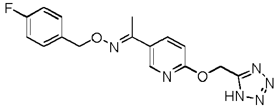

¹H NMR: DMSO-*d*₆, *δ* 2.20 (s, 3H), 5.16 (s, 2H), 5.36 (s, 2H), 6.80 (d, *J* = 8.8 Hz, 1H), 7.16-7.21 (m, 2H), 7.44-7.48 (m, 2H), 7.95 (dd, *J* = 8.8 Hz, 2H), 8.42 (d, *J* = 2.4 Hz, 1H).

### Example 33

### 1-(6-((1H-tetrazol-5-yl)methoxy)pyridin-3-yl)ethanone O-(4-ethoxybenzyl) oxime.

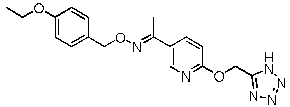

¹H NMR: DMSO-*d*₆, *δ* 1.31 (t, *J* = 6.8 Hz, 3H), 2.17 (s, 3H), 4.00 (d, *J* = 7.2 Hz, 2H), 5.08 (s, 2H), 5.68 (s, 2H), 6.90 (d, *J=* 8.8 Hz, 2H), 6.96 (d, *J=* 8.8 Hz, 1H), 7.32 (d, *J=* 8.4 Hz, 2H), 8.03 (d, *J* = 8.8 Hz, 1H), 8.39 (d, *J* = 2.4 Hz, 1H).

### Example 34

### 1-(6-((1H-tetrazol-5-yl)methoxy)pyridin-3-yl)ethanone O-(4-propoxybenzyl) oxime.

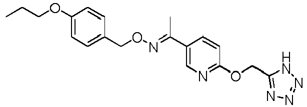

¹H NMR: DMSO-*d*₆, *δ* 0.96 (t, *J* = 7.4 Hz, 3H), 1.70 (q, *J* = 14.0 Hz, 2H), 2.17 (s, 3H), 3.91 (t, *J* = 6.6 Hz, 2H), 5.08 (s, 2H), 5.68 (s, 2H), 6.89 (d, *J* = 9.6 Hz, 2H), 6.96 (d, *J* = 8.0 Hz, 1H), 7.32 (d, *J* = 8.4 Hz, 2H), 8.03 (d, *J* = 8.8 Hz, 1H), 8.39 (d, *J* = 2.0 Hz, 1H).

### Example 35

### 1-(2-((1H-tetrazol-5-yl)methoxy)-6-chloropyridin-3-yl)ethanone O-(4-(trifluoromethyl) benzyl) oxime.

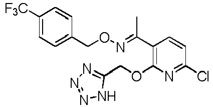

¹H NMR: DMSO-*d*₆, *δ* 2.17 (s, 3H), 5.27 (s, 2H), 5.71 (s, 2H), 7.20 (d, *J=* 8.0 Hz, 1H), 7.58 (d, *J* = 8.0 Hz, 2H), 7.71-7.74 (m, 3H).

### Example 36

### 1-(6-((1H-tetrazol-5-yl)methoxy)-2-chloropyridin-3-yl)ethanone O-(4-(trifluoromethyl) benzyl) oxime.

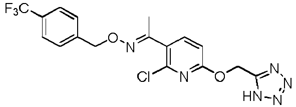

¹H NMR: DMSO-*d*₆, *δ* 2.29 (S, 3H), 5.28 (s, 2H), 7.70 (s 2H), 6.81 (d, *J* = 8.4 Hz, 1H), 7.40 (t, *J* = 5.2 Hz, 1H), 7.47-7.53 (m, 2H), 7.59-7.65 (m, 2H).

### Example 37

### 1-(4-((1H-tetrazol-5-yl)methoxy)phenyl)ethanone O-((4'-(trifluoromethyl)-[1,1'-biphenyl]-4-yl)methyl) oxime.

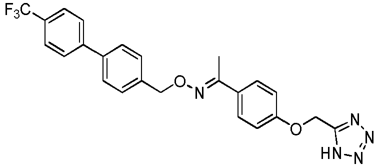

¹H NMR: DMSO-*d*₆, *δ* 2.21 (s, 3H), 5.23 (s, 2H), 5. 37 (s, 2H), 7.07 (d, *J* = 8.8 Hz, 2H), 7.53 (d, J = 8.0, 2H), 7.61 (d, *J* = 8.8 Hz, 2H), 7.74 (d, *J* =8.0 Hz, 2H), 7.81 (d, *J* = 8.0 Hz, 2H), 7.90 (d, *J* = 8.0 Hz, 2H).

### Example 38

### 1-(4-((1H-tetrazol-5-yl)methoxy)phenyl)ethanone O-(2,4,6-triisopropylbenzyl) oxime.

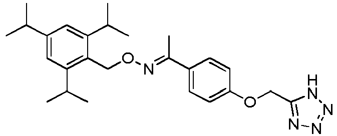

¹H NMR: DMSO-*d*₆, *δ* 1.20 (d, *J* = 6.8 Hz, 18H), 2.10 (s, 3H), 2.82-2.89 (m, 1H), 3.32-3.40 (m, 3H), 5.24 (s, 2H), 5.51 (s, 2H), 7.03 (s, 2H), 7.09 (d, *J* = 8.8 Hz, 2H), 7.65 (d, *J* = 8.8 Hz, 2H).

### Example 39

### 1-(4-((1H-tetrazol-5-yl)methoxy)phenyl)ethanone O-(4-(difluoromethyl)benzyl) oxime.

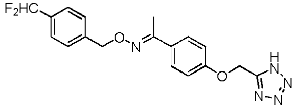

¹H NMR: DMSO-*d*₆, *δ* 2.20 (s, 3H), 5.22 (s, 2H), 5.23 (s, 2H), 6.88-7.16 (m, 3H), 7.52 (m, 6H).

### Example 40

### 1-(4-((1-Methyl-1H-tetrazol-5-yl)methoxy)phenyl)ethanone O-(2,4,6-triisopropylbenzyl) oxime.

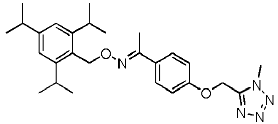

¹H NMR: CDCl₃, *δ* 1.26 (d, *J* = 6.8 Hz, 18H), 2.15 (s, 3H), 2.88-2.91 (m, 1H), 3.39-3.45 (m, 2H), 4.37 (s, 3H), 5.30 (s, 2H), 5.35 (s, 2H), 7.01-7.05 (m, 4H), 7.64 (d, *J* = 8.8 Hz, 2H).

### Example 41

### 1-(4-((2-Ethyl-2H-tetrazol-5-yl)methoxy)phenyl)ethanone O-(4-(trifluoromethyl)benzyl) oxime.

¹H NMR: DMSO-*d₆*, *δ* 1.52 (t, *J* = 7.2 Hz, 3H), 2.23 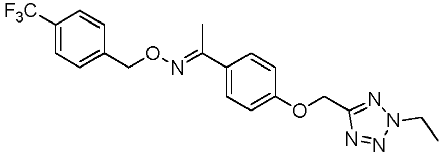 (s, 3H), 4.70 (q, *J=* 14.8 Hz, 2H), 5.27 (s, 2H), 5.39 (s, 2H), 7.07 (d, *J=* 7.6 Hz, 2H), 7.59-7.62 (m, 4H), 7.73 (d, *J* = 8.0 Hz, 2H).

### Example 42

### 1-(4-((1-Ethyl-2H-tetrazol-5-yl)methoxy)phenyl)ethanone O-(4-(trifluoromethyl)benzyl) oxime.

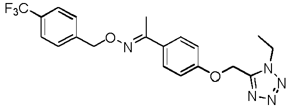

¹H NMR: DMSO-*d*₆, *δ* 1.44 (t, *J* = 7.2 Hz, 3H), 2.23 (s, 3H), 4.50 (q, *J* = 14.4 Hz, 2H), 5.27 (s, 2H), 5.57 (s, 2H), 7.09 (d, *J* = 6.8 Hz, 2H), 7.60-7.63 (m, 4H), 7.73 (d, *J=* 8.0 Hz, 2H).

### Example 43

### 1-(6-((1H-tetrazol-5-yl) methoxy) pyridin-3-yl) ethanone O-(4-(1, 1-difluoroethyl) benzyl) oxime.

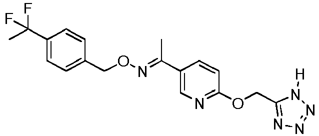

¹H NMR: DMSO-*d*₆, *δ* 1.95 (t, *J* = 18.8 Hz, 3H), 2.23 (s, 3H), 5.23 (s, 2H), 5.69 (s, 2H), 6.97 (d, *J* = 8.8 Hz, 1H), 7.50 (d, *J* = 8.4 Hz, 2H), 7.56 (d, *J* = 8.0 Hz, 2H), 8.03 (d, *J* = 8.4 Hz, 1H), 8.40 (d, *J=* 2.4 Hz, 1H).

### Example 44

### 1-(6-((2-Ethyl-2H-tetrazol-5-yl)methoxy)pyridin-3-yl)ethanone O-(4-(1,1-difluoroethyl) benzyl) oxime.

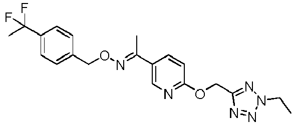

¹H NMR: DMSO-*d*₆, *δ* 1.50 (t, *J* = 7.2 Hz, 3H), 1.95 (t, *J* = 18.8 Hz, 3H), 2.22 (s, 3H), 4.70 (q, *J* = 14.6 Hz, 2H), 5.23 (s, 2H), 5.61 (s, 2H), 6.93 (d, *J* = 8.4 Hz, 1H), 7.50 (d, *J* = 8.4 Hz, 2H), 7.55 (d, *J* = 8.4 Hz, 2H), 8.00 (d, *J* = 8.8 Hz, 1H), 8.40 (d, *J* = 2.8 Hz, 1H).

### Example 45

### 1-(6-((1-Ethyl-2H-tetrazol-5-yl)methoxy)pyridin-3-yl)ethanone O-(4-(1,1-difluoroethyl) benzyl) oxime

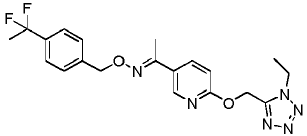

¹H NMR: DMSO-*d*₆, *δ* 1.45 (t, *J* = 7.2 Hz, 3H), 1.95 (t, *J* = 18.8 Hz, 3H), 2.22 (s, 3H), 4.51 (q, *J* = 14.4 Hz, 2H), 5.23 (s, 2H), 5.71 (s, 2H), 6.95 (d, *J* = 8.8 Hz, 1H), 7.50 (d, *J* = 8.4 Hz, 2H), 7.55 (d, *J* = 8.4 Hz, 2H), 8.02 (d, *J* = 8.8 Hz, 1H), 8.39 (d, *J* = 2.4 Hz, 1H).

### Example 46

### 1-(6-((2-Ethyl-2H-tetrazol-5-yl)methoxy)pyridin-3-yl)ethanone O-(4-(trifluoromethyl) benzyl) oxime.

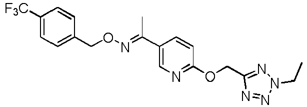

¹H NMR: DMSO-*d*₆, *δ* 1.48 (t, *J*= 7.2 Hz, 3H), 2.25 (s, 3H), 4.66 (q, *J* = 7.4 Hz, 2H), 5.28 (s, 2H), 5.61 (s, 2H), 6.91 (d, *J* = 9.2 Hz, 1H), 7.60 (d, *J* = 8.0 Hz, 2H), 7.72 (d, *J* = 8.0 Hz, 2H), 7.98 (dd, *J* = 8.8 & 2.4 Hz, 1H), 8.40 (d, *J* = 2.0 Hz, 1H).

### Example 47

### 1-(6-((1-Ethyl-2H-tetrazol-5-yl)methoxy)pyridin-3-yl)ethanone O-(4-(trifluoromethyl) benzyl) oxime.

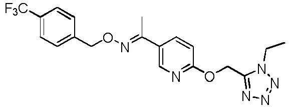

¹H NMR: DMSO-d₆: 1.43 (t, *J* = 7.4 Hz, 3H), 2.25 (s, 3H), 4.49 (q, *J* = 7.4 Hz, 2H), 5.28 (s, 2H), 5.71 (s, 2H), 6.95 (d, *J* = 8.8 Hz, 1H), 7.59 (d, *J* = 8.4 Hz, 2H), 7.72 (d, *J* = 8.4 Hz, 2H), 8.00 (dd, *J* = 8.8 & 2.4 Hz, 1H), 8.39 (d, *J* = 2.0 Hz, 1H).

### Example 48

### 1-(6-((2-isoPropyl-2H-tetrazol-5-yl)methoxy)pyridin-3-yl)ethanone O-(4-(trifluoromethyl) benzyl) oxime.

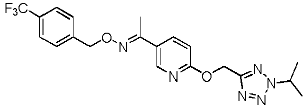

¹H NMR: DMSO-*d*₆, *δ* 1.54 (d, *J* = 6.4 Hz, 6H), 2.25 (s, 3H), 5.09-5.15 (m, 1H), 5.29 (s, 2H), 5.61 (s, 2H), 6.92 (d, *J* = 8.8 Hz, 1H), 7.60 (d, *J* = 9.2 Hz, 2H), 7.72 (d, *J* = 8.0 Hz, 2H), 7.99 (dd, *J* = 8.8 & 2.4 Hz, 1H), 8.41 (d, *J* = 2.0 Hz, 1H).

### Example 49

### 1-(6-((1-isoPropyl-2H-tetrazol-5-yl)methoxy)pyridin-3-yl)ethanone O-(4-(trifluoromethyl) benzyl) oxime.

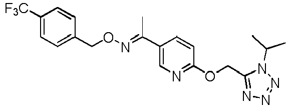

¹H NMR: DMSO-*d*₆, *δ* 1.50 (d, *J* = 6.4 Hz, 6H), 2.24 (s, 3H), 4.96-5.0 (m, 1H), 5.29 (s, 2H), 5.72 (s, 2H), 6.97 (d, *J* = 8.8 Hz, 1H), 7.60 (d, *J* = 8.0 Hz, 2H), 7.72 (d, *J* = 8.0 Hz, 2H), 8.00 (dd, *J* = 8.6 & 2.6 Hz, 1H), 8.41 (d, *J* = 2.0 Hz, 1H).

### Example 50

### 1-(6-((2-(Cyclopropylmethyl)-2H-tetrazol-5-yl)methoxy)pyridin-3-yl)ethanone O-(4-(trifluoromethyl)benzyl) oxime

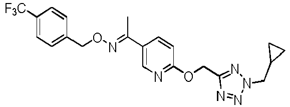

¹H NMR: DMSO-*d*₆, *δ* 0.50-0.53 (m, 2H), 0.67-0.72 (m, 2H), 1.43-1.50 (m, 1H), 2.28 (s, 3H), 4.46 (d, *J* = 7.2 Hz, 2H), 5.27 (s, 2H), 5.68 (s, 2H), 6.83 (dd, *J* = 8.8 & 0.4 Hz, 1H), 7.50 (d, *J* = 8.4 Hz, 2H), 7.62 (d, *J* = 8.0 Hz, 2H), 7.93 (dd, *J* = 8.8 & 2.4 Hz, 1H), 8.39 (dd, *J* = 2.4 & 0.4 Hz, 1H).

### Example 51

### 1-(6-((1-(Cyclopropylmethyl)-2H-tetrazol-5-yl)methoxy)pyridin-3-yl)ethanone O-(4-(trifluoromethyl)benzyl) oxime

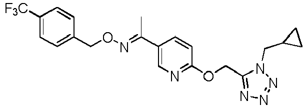

¹H NMR: DMSO-*d*₆, *δ* 0.46-0.50 (m, 2H), 0.66-0.71 (m, 2H), 1.30-1.41 (m, 1H), 2.28 (s, 3H), 4.34 (d, *J* = 7.2 Hz, 2H), 5.28 (s, 2H), 5.72 (s, 2H), 6.80 (d, *J* = 8.8 Hz, 1H), 7.50 (d, *J* = 8.0 Hz, 2H), 7.62 (d, *J* = 8.0 Hz, 2H), 7.96 (dd, *J* = 8.8 & 2.4 Hz, 1H), 8.37 (d, *J* = 2.0 Hz, 1H).

### Example 52

### 1-(6-((2-(Cyclohexylmethyl)-2H-tetrazol-5-yl)methoxy)pyridin-3-yl)ethanone O-(4-(trifluoromethyl)benzyl) oxime.

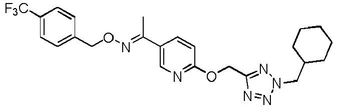

¹H NMR: DMSO-*d*₆, *δ* 1.095-1.096 (m, 2H), 1.23-1.30 (m, 2H), 1.59-1.60 (m, 2H), 1.63-1.64 (m, 2H), 1.67-1.76 (m, 2H), 2.03-2.10 (m, 1H), 2.27 (s, 3H), 4.45 (*d*, *J* = 7.6 Hz, 2H), 5.27 (s, 2H), 5.66 (s, 2H), 6.83 (dd, *J* = 8.6 & 0.6 Hz, 1H), 7.50 (d, *J* = 8.0 Hz, 2H), 7.62 (d, *J* = 8.4 Hz, 2H), 7.93 (dd, *J* = 8.8 & 2.4 Hz, 1H), 8.37 (d, *J* = 2.0 Hz, 1H).

### Example 53

### 1-(6-((1-(Cyclohexylmethyl)-2H-tetrazol-5-yl)methoxy)pyridin-3-yl)ethanone O-(4-(trifluoromethyl)benzyl) oxime.

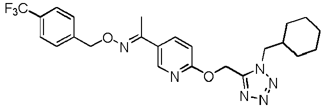

¹H NMR: DMSO-*d*₆, *δ* 1.01-1.06 (m, 2H), 1.14-1.26 (m, 2H), 1.59-1.60 (m, 2H), 1.63-1.76 (m, 4H), 1.94-2.01 (m, 1H), 2.28 (s, 3H), 4.27 (d, *J* = 7.2 Hz, 2H), 5.28 (s, 2H), 5.69 (s, 2H), 6.79 (d, *J* = 8.8 Hz, 1H), 7.50 (d, *J* = 8.0 Hz, 2H), 7.62 (d, *J* = 8.4 Hz, 2H), 7.96 (dd, *J* = 8.4 & 2.4 Hz, 1H), 8.37 (d, *J* = 2.0 Hz, 1H).

### Example 54

### 1-(6-((2-(2-Hydroxyethyl)-2H-tetrazol-5-yl)methoxy)pyridin-3-yl)ethanone O-(4-(trifluoromethyl)benzyl) oxime.

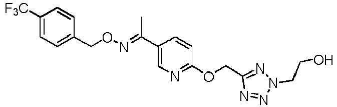

¹H NMR: DMSO-*d*₆, *δ* 2.26 (s, 3H), 4.11 (t, *J* = 4.8 Hz, 2H), 4.66 (t, *J* = 5.0 Hz, 2H), 5.27 (s, 2H), 5.75 (s, 2H), 6.83 (d, *J* = 8.8 Hz, 1H), 7.49 (d, *J* = 8.0 Hz, 2H), 7.64 (d, *J* = 8.0 Hz, 2H), 7.95 (dd, *J* = 8.4 & 2.4 Hz, 1H), 8.31 (d, *J* = 2.4 Hz, 1H).

### Example 55

### 2-(5-(((5-(1-(((4-(Trifluoromethyl)benzyl)oxy)imino)ethyl)pyridin-2-yl)oxy)methyl)-2H-tetrazol-2-yl)acetic acid.

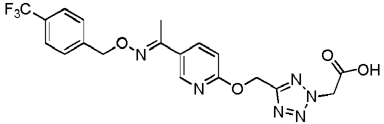

¹H NMR: DMSO-*d*₆, *δ* 2.25 (s, 3H), 5.29 (s, 2H), 5.65 (s, 2H), 5.67 (s, 2H), 6.92 (d, *J* = 8.8 Hz, 1H), 7.60 (d, *J* = 8.0 Hz, 2H), 7.74 (d, *J* = 8.0 Hz, 2H), 7.99 (dd, *J* = 8.8 & 2.4 Hz, 1H), 8.31 (dd, *J* = 2.4 & 0.4 Hz, 1H).

### Example 56

### 2-(5-(((5-(1-(((4-(Trifluoromethyl)benzyl)oxy)imino)ethyl)pyridin-2-yl)oxy)methyl)-1H-tetrazol-1-yl)acetic acid.

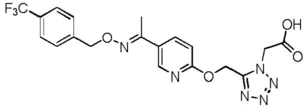

¹H NMR: DMSO-*d*₆, *δ* 2.24 (s, 3H), 5.28 (s, 2H), 5.48 (s, 2H), 5.67 (s, 2H), 6.88 (dd, *J* = 8.8 & 0.4 Hz, 1H), 7.60 (d, *J* = 8.0 Hz, 2H), 7.72 (d, *J* = 8.4 Hz, 2H), 8.00 (dd, *J* = 8.8 & 2.4 Hz, 1H), 8.38 (d, *J* = 2.4 Hz, 1H).

### Example 57

### 1-(6-((1H-tetrazol-5-yl)methoxy)-2-methoxypyridin-3-yl)ethanone O-(4-(trifluoromethyl) benzyl) oxime.

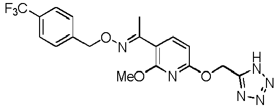

¹H NMR: DMSO-*d*₆, *δ* 2.14 (s, 3H), 3.78 (s, 3H), 5.23 (s, 2H), 5.69 (s, 2H), 6.49 (d, *J* = 8.0 Hz, 1H), 7.57-7.64 (m, 3H), 7.72 (d, *J* = 8.0 Hz, 2H).

### Example 58

### 1-(6-((2-isoPropyl-2H-tetrazol-5-yl)methoxy)pyridin-3-yl)ethanone O-(4-(1,1-difluoroethyl) benzyl) oxime.

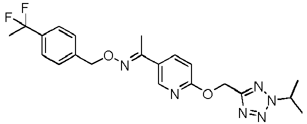

¹H NMR: DMSO-*d*₆, *δ* 1.55 (d, *J* = 6.8 Hz, 6H), 1.95 (t, *J* = 18.8 Hz, 3H), 2.23 (s, 3H), 5.11-5.14 (m, 1H), 5.23 (s, 2H), 5.61 (s, 2H), 6.92 (d, *J* = 8.8 Hz, 1H), 7.50 (d, *J* = 8.4 Hz, 2H), 7.56 (d, *J* = 8.4 Hz, 2H), 8.00 (d, *J* = 8.8 Hz, 1H), 8.41 (d, *J* = 2.8 Hz, 1H).

### Example 59

### 1-(6-((1-isoPropyl-2H-tetrazol-5-yl)methoxy)pyridin-3-yl)ethanone O-(4-(1,1-difluoroethyl) benzyl) oxime.

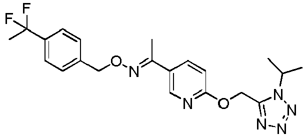

¹H NMR: DMSO-*d*₆, *δ* 1.52 (d, *J* = 6.4 Hz, 6H), 1.95 (t, *J* = 18.8 Hz, 3H), 2.22 (s, 3H), 4.98(t, *J* = 7.0 Hz, 1H), 5.23 (s, 2H), 5.72 (s, 2H), 6.96 (d, *J* = 8.8 Hz, 1H), 7.49 (d, *J* = 8.4 Hz, 2H), 7.55 (d, *J* = 8.4 Hz, 2H), 8.02 (d, *J* = 8.4 Hz, 1H), 8.38 (d, *J* = 2.8 Hz, 1H).

### Example 60

### 1-(6-((2-(Cyclopropylmethyl)-2H-tetrazol-5-yl)methoxy)pyridin-3-yl)ethanone O-(4-(1,1-difluoroethyl)benzyl) oxime.

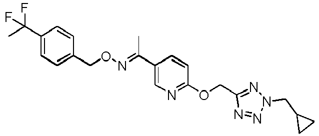

¹H NMR: DMSO-*d*₆, *δ* 0.50-0.53 (m, 2H), 0.67-0.72 (m, 2H), 1.43-1.50 (m, 1H), 1.93 (t, *J* = 18.2 Hz, 3H), 2.26 (s, 3H), 4.47 (d, *J* = 7.2 Hz, 2H), 5.24 (s, 2H), 5.67 (s, 2H), 6.84 (d, *J* = 8.4 Hz, 1H), 7.46 (d, *J* = 8.4 Hz, 2H), 7.51 (d, *J* = 8.4 Hz, 2H), 7.95 (d, *J* = 8.8 Hz, 1H), 8.39 (d, *J* = 2.4 Hz, 1H).

### Example 61

### 1-(6-((1-(Cyclopropylmethyl)-2H-tetrazol-5-yl)methoxy)pyridin-3-yl)ethanone O-(4-(1,1-difluoroethyl)benzyl) oxime.

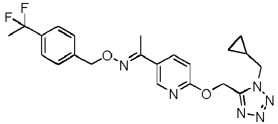

¹H NMR: DMSO-*d*₆, *δ* 0.47-0.50 (m, 2H), 0.66-0.69 (m, 2H), 1.53-1.54 (m, 1H), 1.93 (t, *J* = 18.2 Hz, 3H), 2.26 (s, 3H), 4.35 (d, *J* = 7.2 Hz, 2H), 5.25 (s, 2H), 5.72 (s, 2H), 6.81 (d, *J* = 8.8 Hz, 1H), 7.45 (d, *J* = 8.4 Hz, 2H), 7.52 (d, *J* = 8.8 Hz, 2H), 7.98 (d, *J* = 8.8 Hz, 1H), 8.37 (d, *J* = 2.4 Hz, 1H).

### Example 62

### 1-(6-((2-(2-Hydroxyethyl)-2H-tetrazol-5-yl)methoxy)pyridin-3-yl)ethanone O-(4-(1,1-difluoroethyl)benzyl) oxime.

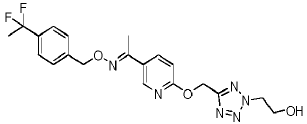

¹H NMR: DMSO-*d*₆, *δ* 1.95 (t, *J* = 19.0 Hz, 3H), 2.23 (s, 3H), 3.88 (q, *J* = 10.6 Hz, 2H), 4.69 (t, *J* = 5.2 Hz, 2H), 5.04 (t, *J* = 5.6 Hz, 1H), 5.23 (s, 2H), 5.62 (s, 2H), 6.92 (d, *J* = 8.8 Hz, 1H), 7.50 (d, *J* = 8.4 Hz, 2H), 7.56 (d, *J* = 8.4 Hz, 2H), 8.02 (d, *J* = 8.8 Hz, 1H), 8.41 (d, *J* = 2.4 Hz, 1H).

### Example 63

### 1-(6-((1-(2-Hydroxyethyl)-2H-tetrazol-5-yl)methoxy)pyridin-3-yl)ethanone O-(4-(1,1-difluoroethyl)benzyl) oxime.

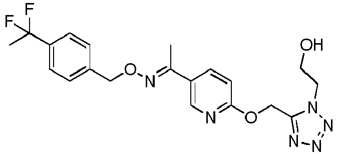

¹H NMR: DMSO-*d*₆, *δ* 1.95 (t, *J* = 18.8 Hz, 3H), 2.22 (s, 3H), 3.78 (m, 2H), 4.60 (t, *J* = 5.4 Hz, 2H), 5.23 (s, 2H), 5.69 (s, 2H), 6.96 (d, *J* = 8.4 Hz, 1H), 7.49 (d, *J* = 8.4 Hz, 2H), 7.55 (d, *J* = 8.4 Hz, 2H), 8.02 (d, *J* = 8.4 Hz, 1H), 8.39 (d, *J* = 2.0 Hz, 1H).

### Example 64

### 1-(4-((2-(2-Hydroxyethyl)-2H-tetrazol-5-yl)methoxy)phenyl)ethanone O-(4-(trifluoromethyl)benzyl) oxime.

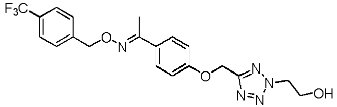

¹H NMR: DMSO-*d*₆, *δ* 2.22 (s, 3H), 3.89 (q, *J* = 10.4 Hz, 2H), 4.71 (t, *J* = 5.2 Hz, 2H), 5.05 (t, *J* = 5.6 Hz, 1H), 5.27 (s, 2H), 5.39 (s, 2H), 7.07 (d, *J* = 6.8 Hz, 2H), 7.58-7.61 (m, 4H), 7.73 (d, *J* = 8.0 Hz, 2H).

### Example 65

### 2-(5-((4-(1-(((4-(Trifluoromethyl)benzyl)oxy)imino)ethyl)phenoxy)methyl)-2H-tetrazol-2-yl)acetonitrile.

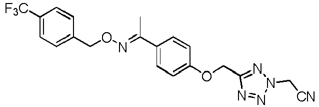

¹H NMR: DMSO-*d*₆, *δ* 2.22 (s, 3H), 5.27 (s, 2H), 5.47 (s, 2H), 6.26 (s, 2H), 7.07 (d, *J* = 6.8 Hz, 2H), 7.60 (d, *J* = 9.2 Hz, 4H), 7.73 (d, *J* = 8.0 Hz, 2H).

### Example 66

### 1-(4-((2-(2-Aminoethyl)-2H-tetrazol-5-yl)methoxy)phenyl)ethanone O-(4-(trifluoromethyl) benzyl) oxime.

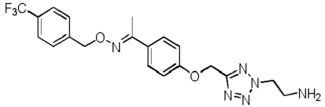

¹H NMR: DMSO-*d*₆, *δ* 2.22 (s, 3H), 3.06 (t, *J* = 6.0 Hz, 2H), 4.62 (t, *J* = 6.2 Hz, 2H), 5.27 (s, 2H), 5.38 (s, 2H), 7.07 (d, *J* = 7.2 Hz, 2H), 7.58-7.62 (m, 4H), 7.73 (d, *J* = 8.0 Hz, 2H).

### Example 67

### 1-(4-((1-((Tetrahydro-2H-pyran-4-yl)methyl)-1H-tetrazol-5-yl)methoxy)phenyl)ethanone O-(4-(trifluoromethyl)benzyl) oxime.

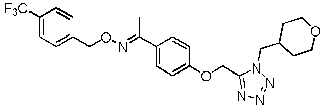

¹H NMR: DMSO-*d*₆, *δ* 1.24-1.39(m, 4H), 2.14-2.18 (m, 1H), 2.22 (s, 3H), 3.18-3.29 (m, 2H), 3.79-3.82 (m, 2H), 4.39 (d, *J* = 7.2 Hz, 2H), 5.27 (s, 2H), 5.56 (s, 2H), 7.08 (dd, *J* = 6.8 & 2.0 Hz, 2H), 7.59-7.63 (m, 4H), 7.72 (d, *J* = 8.0 Hz, 2H).

### Example 68

### 1-(4-((2-((Tetrahydro-2H-pyran-4-yl)methyl)-2H-tetrazol-5-yl)methoxy)phenyl)ethanone O-(4-(trifluoromethyl)benzyl) oxime.

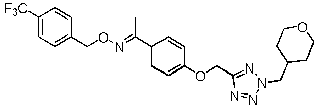

¹H NMR: DMSO-*d*₆, *δ* 1.24-1.31(m, 2H), 1.35-1.38 (m, 2H), 2.16-2.18 (m, 1H), 2.22 (s, 3H), 3.20-3.30 (m, 2H), 3.78 (dd, *J* = 7.4 & 2.4 Hz, 2H), 4.0 (d, *J* = 7.2 Hz, 2H), 5.27 (s, 2H), 5.40 (s, 2H), 7.04-7.07 (m, 2H), 7.57-7.61 (m, 4H), 7.71 (d, *J* = 8.4 Hz, 2H).

### Example 69

### 1-(6-((1-Benzyl-2H-tetrazol-5-yl)methoxy)pyridin-3-yl)ethanone O-(4-(trifluoromethyl) benzyl) oxime.

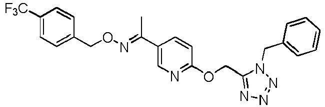

¹H NMR: DMSO-*d*₆, *δ* 2.23 (s, 3H), 5.28 (s, 2H), 5.71 (s, 2H), 5.78 (s, 2H), 6.8 (d, *J* = 8.8 Hz, 1H), 7.26-7.37 (m, 5H), 7.59 (d, *J* = 8.4 Hz, 2H), 7.72 (d, *J* = 8.0 Hz, 2H), 7.96 (dd, *J* = 8.8 & 2.4 Hz, 1H), 8.29 (d, *J* = 2.0 Hz, 1H).

### Example 70

### 1-(6-((2-Benzyl-2H-tetrazol-5-yl)methoxy)pyridin-3-yl)ethanone O-(4-(trifluoromethyl) benzyl) oxime.

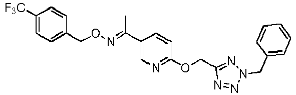

¹H NMR: DMSO-*d*₆, *δ* 2.24 (s, 3H), 5.28 (s, 2H), 5.61 (s, 2H), 5.93 (s, 2H), 6.90 (d, *J* = 8.8 Hz, 1H), 7.30-7.40 (m, 5H), 7.60 (d, *J* = 8.0 Hz, 2H), 7.72 (d, *J =* 8.0 Hz, 2H), 7.97 (dd, *J* = 8.6 & 2.6 Hz, 1H), 8.38 (d, *J* = 2.0 Hz, 1H).

### Example 71

### 1-(4-((1,3,4-Oxadiazol-2-yl)methoxy)phenyl)ethanone O-(4-(trifluoromethyl)benzyl)oxime.

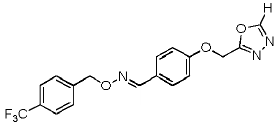

¹H NMR: CDCl₃, *δ* (s, 3H), 2.26 (s, 2H), 5.32 (s, 2H), 6.97-7.00 (m, 2H), 7.50 (d, *J* = 8.0 Hz, 2H), 7.58-7.62 (m, 4H), 8.45 (s, 1H).

### Example 72

### 1-(6-((5-Methyl-1,3,4-oxadiazol-2-yl)methoxy)pyridin-3-yl)ethanone O-(4-(trifluoromethyl) benzyl) oxime.

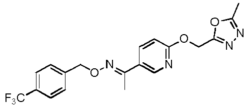

¹H NMR: DMSO-*d*₆, *δ* 2.27 (s, 3H), 2.56 (s, 3H), 5.27 (s, 2H), 5.58 (s, 2H), 6.83 (dd, *J* = 8.4 & 0.4 Hz, 1H), 7.50 (d, *J* = 8.0 Hz, 2H), 7.63 (d, *J* = 8.0 Hz, 2H), 7.96 (dd, *J* = 8.8 & 2.4 Hz, 1H), 8.36 (dd, *J* = 2.4 & 0.8, 1H).

### Example 73

### 1-(4-((2H-1,2,3-triazol-2-yl)methoxy)phenyl)ethanone O-(4-(trifluoromethyl)benzyl)oxime.

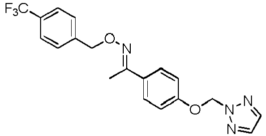

¹H NMR: CDCl₃, *δ* 2.23 (s, 3H), 5.25 (s, 2H), 6.29 (s, 2H), 7.00-7.04 (m, 2H), 7.50 (d *J* = 4.8 Hz, 2H), 7.55-7.62 (m, 4H), 7.74 (d, *J* = 0.8 Hz, 1H), 7.77 (d, *J* = 1.2 Hz, 1H).

### Example 74

### 1-(4-((1H-1,2,3-triazol-1-yl)methoxy)phenyl)ethanone O-(4-(trifluoromethyl)benzyl)oxime.

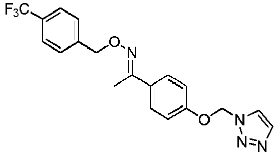

¹H NMR: CDCl₃, *δ* 2.24 (s, 3H), 5.24 (s, 2H), 6.26 (s, 2H), 7.10-7.14 (m, 2H), 7.50 (d, *J* = 4.8 Hz, 2H), 7.55-7.62 (m, 4H), 7.69 (s, 2H).

### Example 75

### 1-(4-(Thiophen-2-ylmethoxy)phenyl)ethanone O-(4-(trifluoromethyl)benzyl)oxime.

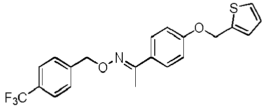

¹HNMR: CDCl₃, *δ* 2.25 (s, 3H), 5.23 (s, 2H), 5.26 (s, 2H), 6.93-6.97 (m, 2H), 7.00 (dd, *J* = 4.8 & 3.6 Hz, 1H), 7.11 (dd, *J* = 3.2 & 0.8 Hz, 1H), 7.32 (dd, *J* = 5.2 & 1.2 Hz, 1H), 7.50 (d, *J* = 8.4 Hz, 2H), 7.56-7.62 (m, 4H).

### Example 76

### 1-(4-(Furan-2-ylmethoxy) phenyl)ethanone O-(4-(trifluoromethyl)benzyl)oxime.

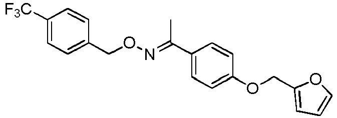

¹HNMR: DMSO-*d*₆, *δ* 2.22 (s, 3H), 5.08 (s, 2H), 5.27 (s, 2H), 6.43-6.47 (m, 1H), 6.59 (s, 1H), 7.03 (d, *J* = 8.8 Hz, 2H), 7.57-7.61 (m, 4H), 7.68-7.74 (m, 3H).

### Example 77

### 1-(4-(Furan-3-ylmethoxy)phenyl)ethanone O-(4-(trifluoromethyl)benzyl)oxime.

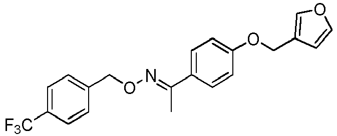

¹HNMR: CDCl₃, *δ* 2.26 (s, 3H), 4.95 (s, 2H), 5.26 (s, 2H), 6.48 (s, 1H), 6.91-6.95 (m, 2H), 7.43 (t, *J* = 1.6 Hz, 1H), 7.50 (d, *J* = 8.4 Hz, 3H), 7.56-7.62 (m, 4H).

### Example 78

### 3-((4-(1-(((4-(Trifluoromethyl) benzyl) oxy)immo)ethyl)phenoxy)methyl)-1H-1,2,4-triazol-5(4H)-one.

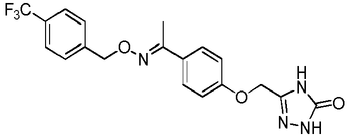

¹HNMR: DMSO-*d*₆, *δ* 2.22 (s, 3H), 4.87 (s, 2H), 5.27 (s, 2H), 7.02 (d, *J* = 8.8 Hz, 2H), 7.58-7.61 (m, 4H), 7.73 (d, *J* = 8.0 Hz, 2H), 11.41 (s, 1H), 11.66 (s, 1H).

### Example 79

### 1-(4-((2-Methylthiazol-4-yl)methoxy)phenyl)ethanone O-(4-(trifluoromethyl)benzyl)oxime.

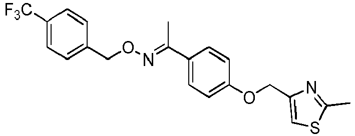

¹HNMR: DMSO-*d*₆, *δ* 2.22 (s, 3H), 2.65 (s, 3H), 5.12 (s, 2H), 5.27 (s, 2H), 7.03 (d, *J* = 8.4 Hz, 2H), 7.53-7.61 (m, 5H), 7.73 (d, *J* = 8.0 Hz, 2H).

### Example 80

### 5-((4-(1-(((3-methoxy-4-(Trifluoromethyl)benzyl)oxy)immo)ethyl)phenoxy)methyl) oxazolidine-2,4-dione.

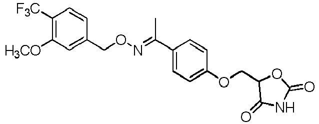

¹HNMR: DMSO-*d*₆, *δ* 2.24 (s, 3H), 3.91 (s, 3H), 4.46 (dd, *J* = 9.2 & 2.4 Hz, 2H), 5.13 (t, *J* = 2.8 Hz, 1H), 5.31 (s, 2H), 6.86-6.93 (m, 2H), 7.07 (d, *J* = 10 Hz, 1H), 7.23 (d, *J* = 8.0 Hz, 1H), 7.48 (d, *J* = 7.6 Hz, 1H), 7.60 (dd, *J* = 6.8 & 2.0 Hz, 2H).

### Example 81

### 1-(4-((1-Trityl-1H-imidazol-4-yl)methoxy)phenyl)ethanone O-(4-(trifluoromethyl)benzyl) oxime.

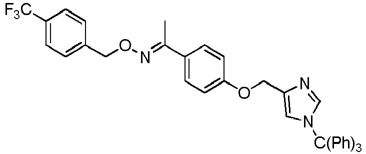

¹H NMR: DMSO-*d*₆, *δ* 2.23 (s, 3H), 4.95 (s, 2H), 5.28 (s, 2H), 6.98-7.02 (m,3H), 7.02-7.07 (m, 6H), 7.35-7.41 (m, 10H), 7.53 (d, *J* = 8.8 Hz, 2H), 7.60 (d, *J* = 8.0 Hz, 2H), 7.73 (d, *J* = 8.0 Hz, 2H).

### Example 82

### 1-(4-((1H-imidazol-4-yl)methoxy)phenyl)ethanone O-(4-(1,1-difluoroethyl)benzyl)oxime.

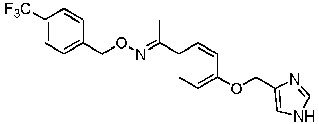

¹H NMR: DMSO-*d*₆, *δ* 2.22 (s, 3H), 4.97 (s, 2H), 5.27 (s, 2H), 7.02 (d, *J* = 8.8 Hz, 2H), 7.17 (s, 1H), 7.55-7.63 (m, 5H), 7.73 (d, *J* = 8.4 Hz, 2H).

### Example 83

### 5-((4-(1-(((4-(Trifluoromethyl)benzyl)oxy)imino)ethyl)phenoxy)methyl)isoxazol- 3(2H)-one.

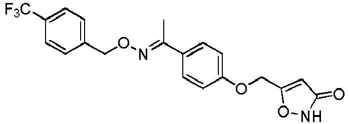

¹H NMR: CDCl₃, *δ* 2.26 (s, 3H), 5.05 (s, 2H), 5.26 (s, 2H), 6.02 (s, 1H), 6.90-6.94 (m, 2H), 7.49 (d, *J* = 8.0 Hz, 2H), 7.57-7.62 (m, 4H).

### Example 84

### 1-(4-((5-(Morpholinomethyl)isoxazol-3-yl)methoxy)phenyl)ethanone O-(4-(trifluoromethyl) benzyl) oxime.

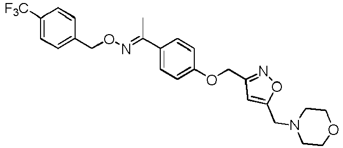

¹H NMR: DMSO-*d*₆, *δ* 2.23 (s, 3H), 2.39-2.42 (m, 4H), 3.55-3.57 (m, 4H), 3.68 (s, 2H), 5.20 (s, 2H), 5.27 (s, 2H), 6.51 (s, 1H), 7.03 (dd, *J* = 6.8 & 2.0 Hz, 2H), 7.58-7.61 (m, 4H), 7.73 (d, *J* = 8.0 Hz, 2H).

### Example 85, forming a disclosure of the application but not part of the invention

### 1-(4-((1H-benzo[d]imidazol-2-yl)methoxy)phenyl)ethanone O-(4-(trifluoromethyl)benzyl) oxime.

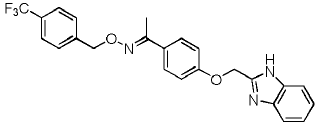

¹H NMR: DMSO-*d*₆, *δ* 2.21 (s, 3H), 5.26 (s, 2H), 5.33 (s, 2H), 7.10 (d, *J* = 8.8 Hz, 2H), 7.20 (m, 2H), 7.60 (d, *J* = 8.4 Hz, 1H), 7.72 (d, *J* = 8.0 Hz, 5H), 7.85 (d, *J* = 8.0 Hz, 2H). The following compounds can be prepared by procedure similar to those described above with appropriate variations of reactions, reaction conditions and quantities of reagents.

### Example 86

### 1-((4-(1-(((4-(trifluoromethyl)benzyl)oxy)imino)ethyl)phenoxy)methyl)pyrrolidine-2,5-dione.

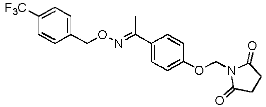

### Example 87

### 1-(((5-(1-(((4-(trifluoromethyl)benzyl)oxy)imino)ethyl)pyridin-2-yl)oxy)methyl) pyrrolidine -2,5-dione.

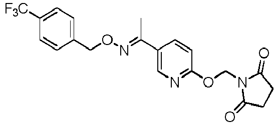

### Example 88

### 5-((4-(1-(((4-(trifluoromethyl)benzyl)oxy)imino)ethyl)phenoxy)methyl)thiazolidine-2,4 dione

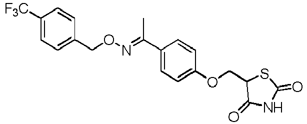

### Example 89

### 5-(((5-(1-(((4-(trifluoromethyl)benzyl)oxy)imino)ethyl)pyridin-2yl)oxy)methyl) thiazolidine -2,4-dione

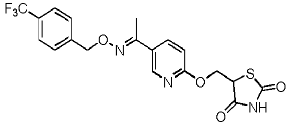

### Example 90

### 2-((4-(1-(((4-(trifluoromethyl)benzyl)oxy)imino)ethyl)phenoxy)methyl)cyclopentane-1,3-dione

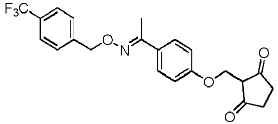

### Example 91

### 2-(((5-(1-(((4-(trifluoromethyl)benzyl)oxy)imino)ethyl)pyridin-2yl)oxy)methyl) cyclopentane-1,3-dione

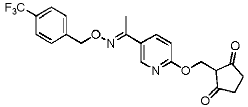

### Example 92

### 3-((4-(1-(((4-(trifluoromethyl)benzyl)oxy)imino)ethyl)phenoxy)methyl)pyrrolidine-2,5-dione

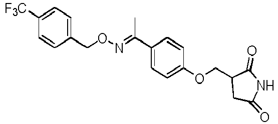

### Example 93

### 3-(((5-(1-(((4-(trifluoromethyl)benzyl)oxy)imino)ethyl)pyridin-2-yl)oxy)methyl)pyrrolidine-2,5-dione

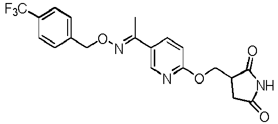

### Example 94

### 1-(4-((4,5-dihydroisoxazol-3-yl)methoxy)phenyl)ethanoneO-(4-(trifluoromethyl)benzyl) oxime

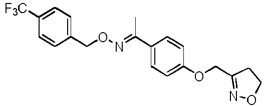

### Example 95

### 1-(6-((4,5-dihydroisoxazol-3-yl)methoxy)pyridin-3-yl)ethanoneO-(4-(trifluoromethyl)benzyl) oxime

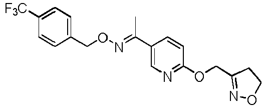

### Example 96

### 1-(4-(pyrrolidin-2-ylmethoxy)phenyl)ethanone O-(4-(trifluoromethyl)benzyl) oxime

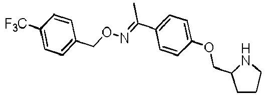

### Example 97

### 1-(6-(pyrrolidin-2-ylmethoxy)pyridin-3-yl)ethanoneO-(4-(trifluoromethyl)benzyl) oxime

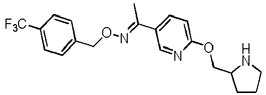

### Example 98

### 4-hydroxy-5,5-dimethyl-3-((4-(1-(((4-(trifluoromethyl)benzyl)oxy)imino)ethyl)phenoxy) methyl)furan-2(5H)-one

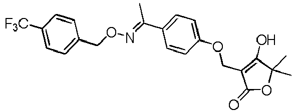

### Example 99

### 4-hydroxy-5,5-dimethyl-3-(((5-(1-(((4-(trifluoromethyl)benzyl)oxy)imino)ethyl)pyridin-2-yl)oxy)methyl)furan-2(5H)-one

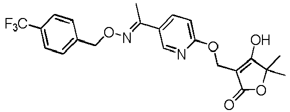

### Example 100

### 1-(4-(pyrrolidin-1-ylmethoxy)phenyl)ethanone O-(4-(trifluoromethyl)benzyl) oxime

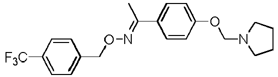

### Example 101

### 1-(6-(pyrrolidin-1-ylmethoxy)pyridin-3-yl)ethanoneO-(4-(trifluoromethyl)benzyl) oxime

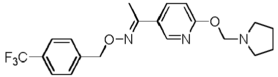

### Example 102

### 5-((4-(1-(((4-(trifluoromethyl)benzyl)oxy)imino)ethyl)phenoxy)methyl)dihydrofuran-2(3H)-one

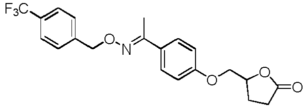

### Example 103

### 5-(((5-(1-(((4-(trinuoromethyl)benzyl)oxy)imino)ethyl)pyridin-2-yl)oxy)methyl) dihydrofuran-2(3H)-one

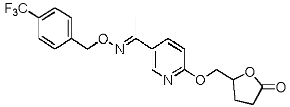

The compounds of the present invention lowered LDL cholesterol (LDL-c), and total cholesterol (TC). This was demonstrated by *in vivo* animal experiments.

### LDL-C lowering activity- in high fat diet C57 mice

The *in-vivo* LDL-c lowering for test compound was tested in C57 mice which were kept on high fat diet for 4 weeks and the blood was collected by retro-orbital sinus puncture method under light ether anesthesia on day 0 (pretreatment). Animal are grouped based on LDL-c levels, after that 4-6 week treatment with vehicle or test compound orally at a dose of 30 mpk dose once a day was given. On completion of treatment on day 28 of the treatment the blood was collected for LDL-c and TC levels measurement. The percent change in LDL-c and TC in test compound group Vs Vehicle group was calculated.

| **Example No** | **Dose** | **% Change Vs Vehicle Control** | |
|---|---|---|---|
| | | **LDL-c** | **TC** |
| 1 | 30mg | -50±4 | -24±4 |
| 2 | 30mg | -29±6 | -14±3 |
| 3 | 30mg | -5±8 | -1±3 |
| 4 | 30mg | -66±2 | -41±4 |
| 6 | 30mg | -30±5 | -11±2 |
| 7 | 30mg | -34±7 | -10±7 |
| 18 | 30mg | -23±6 | -11±3 |
| 20 | 30mg | -29±5 | -13±2 |
| 22 | 30 mg | -61±3 | -30±3 |
| 25 | 30mg | -54±3 | -21±2 |
| 27 | 30 mg | -50±5 | -25±3 |
| 29 | 30mg | -14±6 | -5±3 |
| 31 | 30mg | -26±10 | -7±4 |
| 36 | 30mg | -39±5 | -12±3 |
| 41 | 30mg | -36±6 | -11±5 |
| 42 | 30mg | -22±9 | 1±6 |
| 43 | 30 mg | -67±1 | -38±2 |
| 44 | 30mg | -50±5 | -17±3 |
| 46 | 30mg | -41±4 | -20±2 |
| 48 | 30mg | -47±7 | -17±3 |
| 50 | 30mg | -47±4 | -18±3 |
| 52 | 30mg | -18±11 | -2±5 |
| 56 | 30mg | -22±5 | -16±4 |
| 58 | 30mg | -36±6 | -7±2 |
| 60 | 30mg | -49±3 | -17±3 |
| 64 | 30mg | -41±3 | -10±6 |
| 66 | 30mg | -40±3 | -19±3 |
| 68 | 30mg | -20±4 | -12±2 |

In certain instances, it may be appropriate to administer at least one of the compounds described herein or a pharmaceutically acceptable salt, ester, or prodrug thereof in combination with another therapeutic agent. Several reasons can be attributed for using a combination therapy depending on the need of the patient. As an example, if one of the side effects experienced by a patient upon receiving one of the compounds herein is hypertension, then it may be appropriate to administer an anti-hypertensive agent in combination with the initial therapeutic agent. Or, by way of example only, the benefit experienced by a patient may be increased by administering one of the compounds described herein with another therapeutic agent (which also includes a therapeutic regimen) that also has therapeutic benefit. Several such instances are well known to a skilled person and the use of combination therapy may be envisaged for all such situations. In any case, regardless of the disease, disorder or condition being treated, the overall benefit experienced by the patient may simply be additive of the two therapeutic agents or the patient may experience a synergistic benefit.

Specific, non-limiting examples of possible combination therapies include use of certain compounds disclosed herein with agents found in the following pharmacotherapeutic classifications as indicated below. These lists should not be construed to be closed, but should instead serve as illustrative examples common to the relevant therapeutic area at present. Moreover, combination regimens may include a variety of routes of administration and should include oral, intravenous, intraocular, subcutaneous, dermal, and inhaled topical. For the treatment of metabolic disorders, compounds disclosed herein may be administered with an agent selected from the group comprising: insulin, insulin derivatives and mimetics, insulin secretagogues, insulin sensitizers, biguanide agents, alpha-glucosidase inhibitors, insulinotropic sulfonylurea receptor ligands, meglitinides, GLP-1 (glucagon like peptide-1), GLP-1 analogs, DPPIV (dipeptidyl peptidase IV) inhibitors, GPR-119 inhibitors, sodium-dependent glucose co-transporter (SGLT2) inhibitors, PPAR modulators, non-glitazone type PPAR.delta. agonist, HMG-CoA reductase inhibitors, cholesterol-lowering drugs, rennin inhibitors, anti-thrombotic and anti-platelet agents and anti-obesity agents.

For the treatment of metabolic disorders, compounds disclosed herein may be administered with an agent selected from the group comprising: insulin, metformin, Glipizide, glyburide, Amaryl, gliclazide, meglitinides, nateglinide, repaglinide, amylin mimetics (for example, pramlintide), acarbose, miglitol, voglibose, Exendin-4, , vildagliptin, Liraglutide, naliglutide, saxagliptin, pioglitazone, rosiglitazone, HMG-CoA reductase inhibitors (for example, rosuvastatin, atrovastatin, simvastatin, lovastatin, pravastatin, fluvastatin, cerivastatin, rosuvastatin, pitavastatin and like), cholesterol-lowering drugs (for example, fibrates which include: fenofibrate, benzafibrate, clofibrate, gemfibrozil and like; cholesterol absorption inhibitors such as Ezetimibe, eflucimibe etc.

## Claims

1. Compound of formula (I) their tautomeric forms, their stereoisomers, their pharmaceutically acceptable salts, and pharmaceutical compositions containing them wherein 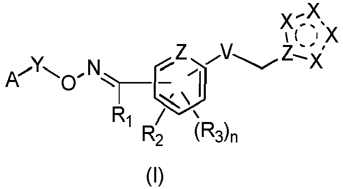
'A' represents an optionally substituted phenyl; 'Y' represents (C₁-C₆)alkyl; 'V' represents O; 'Z' at each occurrence independently represents C or N; 'X' at each occurrence independently represents substituted or unsubstituted C, N, O or S, wherein substitution on X at each occurrence is independently selected from hydrogen, hydroxyl, oxo, amino, cyano, or substituted or unsubstituted groups selected from alkyl, haloalkyl, alkoxy, haloalkoxy, cycloalkyl, heterocyclyl, heterocyclylakyl, acyl, monosubstituted or disubstituted amino, carboxylic acid and its derivatives such as esters and amides, hydroxyalkyl, aminoalkyl, alkoxyalkyl, hydroxyl amino; R₁ represents hydrogen or (C₁-C₃)alkyl
R₂ represents hydrogen or the groups selected from (C₁-C₆)alkyl, (C₁-C₆)alkoxy, hydroxy;
R₃ at each occurrence independently represents hydrogen, halogen, (C₁-C₃)alkyl, halo(C₁-C₃)alkyl, (C₁-C₃)alkoxy;
'n' represents integers from 0-3;

2. The compound as claimed in claim 1 selected from
1-(4-((1H-tetrazol-5-yl)methoxy)phenyl)ethanone O-(4-(trifluoromethyl)benzyl) oxime;
1-(4-((2-Methyl-2H-tetrazol-5-yl)methoxy)phenyl)ethanone O-(4-(trifluoromethyl)benzyl) oxime;
1-(4-((1-Methyl-1H-tetrazol-5-yl)methoxy)phenyl)ethanone O-(4-(trifluoromethyl)benzyl) oxime;
1-(6-((1H-tetrazol-5-yl)methoxy)pyridin-3-yl)ethanone O-(4-(trifluoromethyl)benzyl) oxime;
1-(4-((1H-1,2,4-triazol-1-yl)methoxy)phenyl)ethanone O-(4-(trifluoromethyl)benzyl) oxime;
1-(4-((1H-1,2,3-triazol-5-yl)methoxy)phenyl)ethanone O-(4-(trifluoromethyl)benzyl) oxime;
1-(4-((5-Methyl-1,3,4-oxadiazol-2-yl)methoxy)phenyl)ethanone O-(4-(trifluoromethyl)benzyl) oxime;
1-(4-((5-Ethyl-1,3,4-thiadiazol-2-yl)methoxy)phenyl)ethanone O-(4-(trifluoromethyl)benzyl) oxime;
1-(4-((1,3,4-Thiadiazol-2-yl)methoxy)phenyl)ethanone O-(4-(trifluoromethyl)benzyl) oxime;
3-((4-(1-(((4-(Trifluoromethyl)benzyl)oxy)imino)ethyl)phenoxy)methyl)-1,2,4-oxadiazol-5(4H)-one;
5-((4-(1-(((4-(Trifluoromethyl)benzyl)oxy)imino)ethyl)phenoxy)methyl)oxazolidine-2,4-dione;
1-(4-((2-(tert-Butyl)-5-methyloxazol-4-yl)methoxy)phenyl)ethanone O-(4-(trifluoromethyl)benzyl) oxime;
1-(4-((5-(Hydroxymethyl)furan-2-yl)methoxy)phenyl)ethanone O-(4-(trifluoromethyl)benzyl) oxime;
5-((4-(1-(((4-(Trifluoromethyl)benzyl)oxy)imino)ethyl)phenoxy)methyl)furan-2-carboxylic acid;
1-(4-((5-Methyl-4H-1,2,4-triazol-3-yl)methoxy)phenyl)ethanone O-(4-(trifluoromethyl)benzyl) oxime;
1-(6-((5-Methyl-4H-1,2,4-triazol-3-yl)methoxy)pyridin-3-yl)ethanone O-(4-(1,1-difluoroethyl)benzyl) oxime;
1-(6-((5-Methyl-4H-1,2,4-triazol-3-yl)methoxy)pyridin-3-yl)ethanone O-(4-(trifluoromethyl)benzyl) oxime;
1-(4-((3-Amino-1H-1,2,4-triazol-5-yl)methoxy)phenyl)ethanone O-(4-(trifluoromethyl)benzyl) oxime;
1-(4-((5-Amino-1,3,4-oxadiazol-2-yl)methoxy)phenyl)ethanone O-(4-(trifluoromethyl)benzyl) oxime;
N-(5-((4-(1-(((4-(Trifluoromethyl)benzyl)oxy)imino)ethyl)phenoxy)methyl)-1,3,4-oxadiazol-2-yl)methanesulfonamide;
1-(4-((1H-tetrazol-5-yl)methoxy)phenyl)ethanone O-(4-(1,1-difluoroethyl)benzyl)oxime;
1-(2-((1H-tetrazol-5-yl)methoxy)phenyl)ethanone O-(4-(trifluoromethyl)benzyl)oxime;
1-(4-((1H-tetrazol-5-yl)methoxy)phenyl)ethanone O-((6-(trifluoromethyl)pyridin-3-yl)methyl) oxime;
1-(4-((1H-tetrazol-5-yl)methoxy)phenyl)ethanone O-(3-methoxy-4-(trifluoromethyl)benzyl) oxime;
1-(6-((1H-tetrazol-5-yl)methoxy)pyridin-3-yl)ethanone O-(3-methoxy-4-(trifluoromethyl)benzyl) oxime;
1-(4-((1H-tetrazol-5-yl) methoxy)-2-hydroxyphenyl) ethanone O-(4-(trifluoromethyl) benzyl)oxime;
1-(2-Hydroxy-4-((1-methyl-1H-tetrazol-5-yl)methoxy)phenyl)ethanone O-(4-(trifluoromethyl)benzyl) oxime;
1-(4-((1H-tetrazol-5-yl)methoxy)phenyl)ethanone O-benzyl oxime;
1-(4-((1H-tetrazol-5-yl)methoxy)phenyl)ethanone O-(4-fluorobenzyl) oxime;
1-(6-((1H-tetrazol-5-yl)methoxy)pyridin-3-yl)ethanone O-(4-fluorobenzyl) oxime;
1-(6-((1H-tetrazol-5-yl)methoxy)pyridin-3-yl)ethanone O-(4-ethoxybenzyl) oxime;
1-(6-((1H-tetrazol-5-yl)methoxy)pyridin-3-yl)ethanone O-(4-propoxybenzyl) oxime;
1-(2-((1H-tetrazol-5-yl)methoxy)-6-chloropyridin-3-yl)ethanone O-(4-(trifluoromethyl)benzyl) oxime;
1-(6-((1H-tetrazol-5-yl)methoxy)-2-chloropyridin-3-yl)ethanone O-(4-(trifluoromethyl)benzyl) oxime;
1-(4-((1H-tetrazol-5-yl)methoxy)phenyl)ethanone O-((4'-(trifluoromethyl)-[1,1'-biphenyl]-4-yl)methyl) oxime.
1-(4-((1H-tetrazol-5-yl)methoxy)phenyl)ethanone O-(2,4,6-triisopropylbenzyl) oxime.
1-(4-((1H-tetrazol-5-yl)methoxy)phenyl)ethanone O-(4-(difluoromethyl)benzyl) oxime;
1-(4-((1-Methyl-1H-tetrazol-5-yl)methoxy)phenyl)ethanone O-(2,4,6-triisopropylbenzyl) oxime;
1-(4-((2-Ethyl-2H-tetrazol-5-yl)methoxy)phenyl)ethanone O-(4-(trifluoromethyl)benzyl)oxime;
1-(4-((1-Ethyl-2H-tetrazol-5-yl)methoxy)phenyl)ethanone O-(4-(trifluoromethyl)benzyl)oxime;
1-(6-((1H-tetrazol-5-yl) methoxy) pyridin-3-yl) ethanone O-(4-(1,1-difluoroethyl) benzyl) oxime.
1-(6-((2-Ethyl-2H-tetrazol-5-yl)methoxy)pyridin-3-yl)ethanone O-(4-(1,1-difluoroethyl)benzyl) oxime;
1-(6-((1-Ethyl-2H-tetrazol-5-yl)methoxy)pyridin-3-yl)ethanone O-(4-(1,1-difluoroethyl)benzyl) oxime;
1-(6-((2-Ethyl-2H-tetrazol-5-yl)methoxy)pyridin-3-yl)ethanone O-(4-(trifluoromethyl)benzyl) oxime;
1-(6-((1-Ethyl-2H-tetrazol-5-yl)methoxy)pyridin-3-yl)ethanone O-(4-(trifluoromethyl)benzyl) oxime;
1-(6-((2-*iso*Propyl-2H-tetrazol-5-yl)methoxy)pyridin-3-yl)ethanone O-(4-(trifluoromethyl)benzyl) oxime;
1-(6-((1-*iso*Propyl-2H-tetrazol-5-yl)methoxy)pyridin-3-yl)ethanone O-(4-(trifluoromethyl)benzyl) oxime;
1-(6-((2-(Cyclopropylmethyl)-2H-tetrazol-5-yl)methoxy)pyridin-3-yl)ethanone O-(4-(trifluoromethyl)benzyl) oxime;
1-(6-((1-(Cyclopropylmethyl)-2H-tetrazol-5-yl)methoxy)pyridin-3-yl)ethanone O-(4-(trifluoromethyl)benzyl) oxime;
1-(6-((2-(Cyclohexylmethyl)-2H-tetrazol-5-yl)methoxy)pyridin-3-yl)ethanone O-(4-(trifluoromethyl)benzyl) oxime;
1-(6-((1-(Cyclohexylmethyl)-2H-tetrazol-5-yl)methoxy)pyridin-3-yl)ethanone O-(4-(trifluoromethyl)benzyl) oxime;
1-(6-((2-(2-Hydroxyethyl)-2H-tetrazol-5-yl)methoxy)pyridin-3-yl)ethanone O-(4-(trifluoromethyl)benzyl) oxime;
2-(5-(((5-(1-(((4-(Trifluoromethyl)benzyl)oxy)imino)ethyl)pyridin-2-yl)oxy)methyl)-2H-tetrazol-2-yl)acetic acid;
2-(5-(((5-(1-(((4-(Trifluoromethyl)benzyl)oxy)imino)ethyl)pyridin-2-yl)oxy)methyl)-1H-tetrazol-1-yl)acetic acid;
3-(5-(((5-(1-(((4-(Trifluoromethyl)benzyl)oxy)imino)ethyl)pyridin-2-yl)oxy)methyl)-2H-tetrazol-2-yl)propanoic acid;
1-(6-((1H-tetrazol-5-yl)methoxy)-2-methoxypyridin-3-yl)ethanone O-(4-(trifluoromethyl)benzyl) oxime;
1-(6-((2-*iso*Propyl-2H-tetrazol-5-yl)methoxy)pyridin-3-yl)ethanone O-(4-(1,1-difluoroethyl)benzyl) oxime;
1-(6-((1-*iso*Propyl-2H-tetrazol-5-yl)methoxy)pyridin-3-yl)ethanone O-(4-(1,1-difluoroethyl)benzyl) oxime;
1-(6-((2-(Cyclopropylmethyl)-2H-tetrazol-5-yl)methoxy)pyridin-3-yl)ethanone O-(4-(1,1-difluoroethyl)benzyl) oxime;
1-(6-((1-(Cyclopropylmethyl)-2H-tetrazol-5-yl)methoxy)pyridin-3 -yl)ethanone O-(4-(1,1-difluoroethyl)benzyl) oxime;
1-(6-((2-(2-Hydroxyethyl)-2H-tetrazol-5-yl)methoxy)pyridin-3-yl)ethanone O-(4-(1,1-difluoroethyl)benzyl) oxime;
1-(6-((1-(2-Hydroxyethyl)-2H-tetrazol-5-yl)methoxy)pyridin-3-yl)ethanone O-(4-(1,1-difluoroethyl)benzyl) oxime;
1-(4-((2-(2-Hydroxyethyl)-2H-tetrazol-5-yl)methoxy)phenyl)ethanone O-(4-(trifluoromethyl)benzyl) oxime;
2-(5-((4-(1-(((4-(Trifluoromethyl)benzyl)oxy)imino)ethyl)phenoxy)methyl)-2H-tetrazol-2-yl)acetonitrile;
1-(4-((2-(2-Aminoethyl)-2H-tetrazol-5-yl)methoxy)phenyl)ethanone O-(4-(trifluoromethyl)benzyl) oxime;
1-(4-((1-((Tetrahydro-2H-pyran-4-yl)methyl)-1H-tetrazol-5-yl)methoxy)phenyl)ethanone O-(4-(trifluoromethyl)benzyl) oxime;
1-(4-((2-((Tetrahydro-2H-pyran-4-yl)methyl)-2H-tetrazol-5-yl)methoxy)phenyl)ethanone O-(4-(trifluoromethyl)benzyl) oxime;
1-(6-((1-Benzyl-2H-tetrazol-5-yl)methoxy)pyridin-3-yl)ethanone O-(4-(trifluoromethyl)benzyl) oxime;
1-(6-((2-Benzyl-2H-tetrazol-5-yl)methoxy)pyridin-3-yl)ethanone O-(4-(trifluoromethyl)benzyl) oxime;
1-(4-((1,3,4-Oxadiazol-2-yl)methoxy)phenyl)ethanone O-(4-(trifluoromethyl)benzyl) oxime;
1-(6-((5-Methyl-1,3,4-oxadiazol-2-yl)methoxy)pyridin-3-yl)ethanone O-(4-(trifluoromethyl)benzyl) oxime;
1-(4-((2H-1,2,3-triazol-2-yl)methoxy)phenyl)ethanone O-(4-(trifluoromethyl)benzyl)oxime;
1-(4-((1H-1,2,3-triazol-1-yl)methoxy)phenyl)ethanone O-(4-(trifluoromethyl)benzyl)oxime;
1-(4-(Thiophen-2-ylmethoxy)phenyl)ethanone O-(4-(trifluoromethyl)benzyl)oxime;
1-(4-(Furan-2-ylmethoxy) phenyl)ethanone O-(4-(trifluoromethyl)benzyl)oxime;
1-(4-(Furan-3-ylmethoxy)phenyl)ethanone O-(4-(trifluoromethyl)benzyl)oxime;
3-((4-(1-(((4-(Trifluoromethyl)benzyl)oxy)imino)ethyl)phenoxy)methyl)-1H-1,2,4-triazol-5(4H)-one;
1-(4-((2-Methylthiazol-4-yl)methoxy)phenyl)ethanone O-(4-(trifluoromethyl)benzyl)oxime;
5-((4-(1-(((3-methoxy-4-(Trifluoromethyl)benzyl)oxy)imino)ethyl)phenoxy)methyl)oxazolidine-2,4-dione;
1-(4-((1-Trityl-1H-imidazol-4-yl)methoxy)phenyl)ethanone O-(4-(trifluoromethyl)benzyl) oxime;
1-(4-((1H-imidazol-4-yl)methoxy)phenyl)ethanone O-(4-(1,1-difluoroethyl)benzyl)oxime;
5-((4-(1-(((4-(Trifluoromethyl)benzyl)oxy)imino)ethyl)phenoxy)methyl)isoxazol-3(2H)-one;
1-(4-((5-(Morpholinomethyl)isoxazol-3-yl)methoxy)phenyl)ethanone O-(4-(trifluoromethyl)benzyl)oxime;
1-(4-((1H-benzo[d]imidazol-2-yl)methoxy)phenyl)ethanone O-(4-(trifluoromethyl)benzyl) oxime;
1-((4-(1-(((4-(trifluoromethyl)benzyl)oxy)imino)ethyl)phenoxy)methyl)pyrrolidine-2,5-dione;
1-(((5-(1-(((4-(trifluoromethyl)benzyl)oxy)imino)ethyl)pyridin-2-yl)oxy)methyl) pyrrolidine-2,5-dione;
5-((4-(1-(((4-(trifluoromethyl)benzyl)oxy)imino)ethyl)phenoxy)methyl)thiazolidine-2,4-dione;
5-(((5-(1-(((4-(trifluoromethyl)benzyl)oxy)imino)ethyl)pyridin-2yl)oxy)methyl) thiazolidine-2,4-dione;
2-((4-(1-(((4-(trifluoromethyl)benzyl)oxy)imino)ethyl)phenoxy)methyl)cyclopentane-1,3-dione;
2-(((5-(1-(((4-(trifluoromethyl)benzyl)oxy)imino)ethyl)pyridin-2 yl)oxy)methyl)cyclopentane-1,3-dione;
3-((4-(1-(((4-(trifluoromethyl)benzyl)oxy)imino)ethyl)phenoxy)methyl)pyrrolidine-2,5 -dione;
3-(((5-(1-(((4-(trifluoromethyl)benzyl)oxy)imino)ethyl)pyridin-2-yl)oxy)methyl)pyrrolidine-2,5-dione;
1-(4-((4,5-dihydroisoxazol-3-yl)methoxy)phenyl)ethanone O-(4-(trifluoromethyl)benzyl)oxime;
1-(6-((4,5-dihydroisoxazol-3-yl)methoxy)pyridin-3-yl)ethanone O-(4-(trifluoromethyl)benzyl) oxime;
1-(4-(pyrrolidin-2-ylmethoxy)phenyl)ethanone O-(4-(trifluoromethyl)benzyl)oxime;
1-(6-(pyrrolidin-2-ylmethoxy)pyridin-3-yl)ethanone O-(4-(trifluoromethyl)benzyl)oxime;
4-hydroxy-5,5-dimethyl-3-((4-(1-(((4-(trifluoromethyl)benzyl)oxy)imino)ethyl)phenoxy)methyl)furan-2(5H)-one;
4-hydroxy-5,5-dimethyl-3-(((5-(1-(((4-(trifluoromethyl)benzyl)oxy)imino)ethyl)pyridin-2-yl)oxy)methyl)furan-2(5H)-one;
1-(4-(pyrrolidin-1-ylmethoxy)phenyl)ethanone O-(4-(trifluoromethyl)benzyl) oxime;
1-(6-(pyrrolidin-1-ylmethoxy)pyridin-3-yl)ethanone O-(4-(trifluoromethyl)benzyl) oxime;
5-((4-(1-(((4-(trifluoromethyl)benzyl)oxy)imino)ethyl)phenoxy)methyl)dihydrofuran-2(3H)-one;
5-(((5-(1-(((4-(trifluoromethyl)benzyl)oxy)imino)ethyl)pyridin-2-yl)oxy)methyl)dihydrofuran-2(3H)-one.

3. The compound as claimed in claim 1 to 2 suitably formulated into a suitable pharmaceutical composition.

4. The compound of formula (I) as claimed in claim 1 to 2 or its pharmaceutical composition as claimed in claim 3 for use in the treatment of hyperlipidemia or dyslipidemia.

5. A pharmaceutical composition comprising compounds of formula (I) as claimed in claim 1 to 2 in combination with one or more pharmaceutically active agents selected from group comprising insulin, insulin derivatives and mimetics, insulin secretagogues, insulin sensitizers, biguanide agents, alpha-glucosidase inhibitors, insulinotropic sulfonylurea receptor ligands, meglitinides, GLP-1 (glucagon like peptide-1), GLP-1 analogs, DPPIV (dipeptidyl peptidase IV) inhibitors, GPR-119 activators, sodium-dependent glucose co-transporter (SGLT2) inhibitors, PPAR modulators, non-glitazone type PPAR delta agonist, HMG-CoA reductase inhibitors, cholesterol-lowering drugs, rennin inhibitors, anti-thrombotic and anti-platelet agents and anti-obesity agents or pharmaceutically acceptable salts thereof.

6. The pharmaceutical composition as claimed in claim 3 for use in the treatment of dyslipidemia and hyperlipidemia.

## Patentansprüche

1. Verbindung der Formel (I), deren tautomere Formen, deren Stereoisomere, deren pharmazeutisch unbedenkliche Salze und diese enthaltende pharmazeutische Zusammensetzungen, wobei 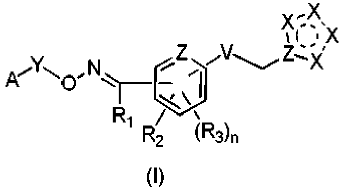
"A" für ein gegebenenfalls substituiertes Phenyl steht; "Y" für (C₁-C₆)-Alkyl steht; "V" für O steht; "Z" bei jedem Auftreten unabhängig für C oder N steht; "X" bei jedem Auftreten unabhängig für substituiertes oder unsubstituiertes C, N, O oder S steht, wobei eine Substitution an X bei jedem Auftreten unabhängig ausgewählt ist aus Wasserstoff, Hydroxyl, Oxo, Amino, Cyano, oder substituierten oder unsubstituierten Gruppen, die ausgewählt sind aus Alkyl, Halogenalkyl, Alkoxy, Halogenalkoxy, Cycloalkyl, Heterocyclyl, Heterocyclylakyl, Acyl, monosubstituiertem oder disubstituiertem Amino, Carbonsäure und ihren Derivaten, wie z. B. Estern und Amiden, Hydroxyalkyl, Aminoalkyl, Alkoxyalkyl, Hydroxylamino; R₁ für Wasserstoff oder (C₁-C₃)-Alkyl steht
R₂ für Wasserstoff oder die aus (C₁-C₆)-Alkyl, (C₁-C₆)-Alkoxy, Hydroxy ausgewählten Gruppen steht;
R₃ bei jedem Auftreten unabhängig für Wasserstoff, Halogen, (C₁-C₃)-Alkyl, Halogen(C₁-C₃)-alkyl, (C₁-C₃)-Alkoxy steht;
"n" für ganze Zahlen von 0-3 steht.

2. Verbindung nach Anspruch 1, ausgewählt aus
1-(4-((1H-Tetrazol-5-yl)methoxy)phenyl)ethanon-O-(4-(trifluormethyl)benzyl)oxim;
1-(4-((2-Methyl-2H-tetrazol-5-yl)methoxy)phenyl)ethanon-O-(4-(trifluormethyl)benzyl)oxim;
1-(4-((1-Methyl-1H-tetrazol-5-yl)methoxy)phenyl)ethanon-O-(4-(trifluormethyl)benzyl)oxim;
1-(6-((1H-tetrazol-5-yl)methoxy)pyridin-3-yl)ethanon-O-(4-(trifluormethyl)benzyl)oxim;
1-(4-((1H-1,2,4-Triazol-1-yl)methoxy)phenyl)ethanon-O-(4-(trifluormethyl)benzyl)oxim;
1-(4-((1H-1,2,3-Triazol-5-yl)methoxy)phenyl)ethanon-O-(4-(trifluormethyl)benzyl)oxim;
1-(4-((5-Methyl-1,3,4-oxadiazol-2-yl)methoxy)phenyl)ethanon-O-(4-(trifluormethyl)benzyl) oxim;
1-(4-((5-Ethyl-1,3,4-thiadiazol-2-yl)methoxy)phenyl)ethanon-O-(4-(trifluormethyl)benzyl)oxim;
1-(4-((1,3,4-Thiadiazol-2-yl)methoxy)phenyl)ethanon-O-(4-(trifluormethyl)benzyl)oxim;
3-((4-(1-(((4-(Trifluormethyl)benzyl)oxy)imino)ethyl)phenoxy)methyl)-1,2,4-oxadiazol-5(4H)-o n;
5-((4-(1-(((4-(Trifluormethyl)benzyl)oxy)imino)ethyl)phenoxy)methyl)oxazolidin-2,4-dion;
1-(4-((2-(tert-Butyl)-5-methyloxazol-4-yl)methoxy)phenyl)ethanon-O-(4-(trifluormethyl)benzyl) oxim;
1-(4-((5-(Hydroxymethyl)furan-2-yl)methoxy)phenyl)ethanon-O-(4-(trifluormethyl)benzyl)oxim;
5-((4-(1-(((4-(Trifluormethyl)benzyl)oxy)imino)ethyl)phenoxy)methyl)furan-2-carbonsäure;
1-(4-((5-Methyl-4H-1,2,4-triazol-3-yl)methoxy)phenyl)ethanon-O-(4-(trifluormethyl)benzyl)oxi m;
1-(6-((5-Methyl-4H-1,2,4-triazol-3-yl)methoxy)pyridin-3-yl)ethanon-O-(4-(1,1-difluorethyl)ben zyl)oxim;
1-(6-((5-Methyl-4H-1,2,4-triazol-3-yl)methoxy)pyridin-3-yl)ethanon-O-(4-(trifluormethyl)benzy l)oxim;
1-(4-((3-Amino-1H-1,2,4-triazol-5-yl)methoxy)phenyl)ethanon-O-(4-(trifluormethyl)benzyl)oxi m;
1-(4-((5-Amino-1,3,4-oxadiazol-2-yl)methoxy)phenyl)ethanon-O-(4-(trifluormethyl)benzyl)oxi m;
N-(5-((4-(1-(((4-(Trifluormethyl)benzyl)oxy)imino)ethyl)phenoxy)methyl)-1,3,4-oxadiazol-2-yl) methansulfonamid;
1-(4-((1H-Tetrazol-5-yl)methoxy)phenyl)ethanon-O-(4-(1,1-difluorethyl)benzyl)oxim;
1-(2-((1H-Tetrazol-5-yl)methoxy)phenyl)ethanon-O-(4-(trifluormethyl)benzyl)oxim;
1-(4-((1H-Tetrazol-5-yl)methoxy)phenyl)ethanone-O-((6-(trifluormethyl)pyridin-3-yl)methyl)ox im;
1-(4-((1H-Tetrazol-5-yl)methoxy)phenyl)ethanon-O-(3-methoxy-4-(trifluormethyl)benzyl)oxim;
1-(6-((1H-Tetrazol-5-yl)methoxy)pyridin-3-yl)ethanon-O-(3-methoxy-4-(trifluormethyl)benzyl) oxim;
1-(4-((1H-Tetrazol-5-yl)methoxy)-2-hydroxyphenyl)ethanon-O-(4-(trifluormethyl)benzyl)oxim;
1-(2-Hydroxy-4-((1-methyl-1H-tetrazol-5-yl)methoxy)phenyl)ethanon-O-(4-(trifluormethyl)benz yl)oxim;
1-(4-((1H-Tetrazol-5-yl)methoxy)phenyl)ethanon-O-benzyloxim;
1-(4-((1H-Tetrazol-5-yl)methoxy)phenyl)ethanon-O-(4-fluorbenzyl)oxim;
1-(6-((1H-Tetrazol-5-yl)methoxy)pyridin-3-yl)ethanon-O-(4-fluorbenzyl)oxim;
1-(6-((1H-Tetrazol-5-yl)methoxy)pyridin-3-yl)ethanon-O-(4-ethoxybenzyl)oxim;
1-(6-((1H-Tetrazol-5-yl)methoxy)pyridin-3-yl)ethanon-O-(4-propoxybenzyl)oxim;
1-(2-((1H-Tetrazol-5-yl)methoxy)-6-chlorpyridin-3-yl)ethanon-O-(4-(trifluormethyl)benzyl)oxi m;
1-(6-((1H-Tetrazol-5-yl)methoxy)-2-chlorpyridin-3-yl)ethanon-O-(4-(trifluormethyl)benzyl)oxi m;
1-(4-((1H-Tetrazol-5-yl)methoxy)phenyl)ethanon-O-((4'-(trifluormethyl)-[1,1'-biphenyl]-4-yl)m ethyl)oxim;
1-(4-((1H-Tetrazol-5-yl)methoxy)phenyl)ethanon-O-(2,4,6-triisopropylbenzyl)oxim;
1-(4-((1H-Tetrazol-5-yl)methoxy)phenyl)ethanon-O-(4-(difluormethyl)benzyl)oxim;
1-(4-((1-Methyl-1H-tetrazol-5-yl)methoxy)phenyl)ethanon-O-(2,4,6-triisopropylbenzyl)oxim;
1-(4-((2-Ethyl-2H-tetrazol-5-yl)methoxy)phenyl)ethanon-O-(4-(trifluormethyl)benzyl)oxim;
1-(4-((1-Ethyl-2H-tetrazol-5-yl)methoxy)phenyl)ethanon-O-(4-(trifluormethyl)benzyl)oxim;
1-(6-((1H-Tetrazol-5-yl)methoxy)pyridin-3-yl)ethanon-O-(4-(1,1-difluorethyl)benzyl)oxim;
1-(6-((2-Ethyl-2H-tetrazol-5-yl)methoxy)pyridin-3-yl)ethanon-O-(4-(1,1-difluorethyl)benzyl)oxi m;
1-(6-((1-Ethyl-2H-tetrazol-5-yl)methoxy)pyridin-3-yl)ethanon-O-(4-(1,1-difluorethyl)benzyl)oxi m;
1-(6-((2-Ethyl-2H-tetrazol-5-yl)methoxy)pyridin-3-yl)ethanon-O-(4-(trifluormethyl)benzyl)oxim; ;
1-(6-((1-Ethyl-2H-tetrazol-5-yl)methoxy)pyridin-3-yl)ethanon-O-(4-(trifluormethyl)benzyl)oxim;
1-(6-((2-Isopropyl-2H-tetrazol-5-yl)methoxy)pyridin-3-yl)ethanon-O-(4-(trifluormethyl)benzyl) oxim;
1-(6-((1-Isopropyl-2H-tetrazol-5-yl)methoxy)pyridin-3-yl)ethanon-O-(4-(trifluormethyl)benzyl) oxim;
1-(6-((2-(Cyclopropylmethyl)-2H-tetrazol-5-yl)methoxy)pyridin-3-yl)ethanon-O-(4-(trifluormet hyl)benzyl)oxim;
1-(6-((1-(Cyclopropylmethyl)-2H-tetrazol-5-yl)methoxy)pyridin-3-yl)ethanon-O-(4-(trifluormet hyl)benzyl)oxim;
1-(6-((2-(Cyclohexylmethyl)-2H-tetrazol-5-yl)methoxy)pyridin-3-yl)ethanon-O-(4-(trifluormeth yl)benzyl)oxim;
1-(6-((1-(Cyclohexylmethyl)-2H-tetrazol-5-yl)methoxy)pyridin-3-yl)ethanon-O-(4-(trifluormeth yl)benzyl)oxim;
1-(6-((2-(2-Hydroxyethyl)-2H-tetrazol-5-yl)methoxy)pyridin-3-yl)ethanon-O-(4-(trifluormethyl) benzyl)oxim;
2-(5-(((5-(1-(((4-(Trifluormethyl)benzyl)oxy)imino)ethyl)pyridin-2-yl)oxy)methyl)-2H-tetrazol-2-yl)essigsäure;
2-(5-(((5-(1-(((4-(Trifluormethyl)benzyl)oxy)imino)ethyl)pyridin-2-yl)oxy)methyl)-1H-tetrazol-1-yl)essigsäure;
3-(5-(((5-(1-(((4-(Trifluormethyl)benzyl)oxy)imino)ethyl)pyridin-2-yl)oxy)methyl)-2H-tetrazol-2-yl)propansäure;
1-(6-((1H-Tetrazol-5-yl)methoxy)-2-methoxypyridin-3-yl)ethanon-O-(4-(trifluormethyl)benzyl) oxim;
1-(6-((2-Isopropyl-2H-tetrazol-5-yl)methoxy)pyridin-3-yl)ethanon-O-(4-(1,1-difluorethyl)benzyl )oxim;
1-(6-((1-Isopropyl-2H-tetrazol-5-yl)methoxy)pyridin-3-yl)ethanon-O-(4-(1,1-difluorethyl)benzyl )oxim;
1-(6-((2-(Cyclopropylmethyl)-2H-tetrazol-5-yl)methoxy)pyridin-3-yl)ethanon-O-(4-(1,1-difluore thyl)benzyl)oxim;
1-(6-((1-(Cyclopropylmethyl)-2H-tetrazol-5-yl)methoxy)pyridin-3-yl)ethanon-O-(4-(1,1-difluorethyl)benzyl)oxim;
1-(6-((2-(2-Hydroxyethyl)-2H-tetrazol-5-yl)methoxy)pyridin-3-yl)ethanon-o-(4-(1,1-difluorethyl)benzyl)oxim;
1-(6-((1-(2-Hydroxyethyl)-2H-tetrazol-5-yl)methoxy)pyridin-3-yl)ethanon-o-(4-(1,1-difluorethyl)benzyl)oxim;
1-(4-((2-(2-Hydroxyethyl)-2H-tetrazol-5-yl)methoxy)phenyl)ethanon-O-(4-(trifluormethyl)benzyl)oxim;
2-(5-((4-(1-(((4-(Trifluormethyl)benzyl)oxy)imino)ethyl)phenoxy)methyl)-2H-tetrazol-2-yl)acetonitril;
1-(4-((2-(2-Aminoethyl)-2H-tetrazol-5-yl)methoxy)phenyl)ethanon-O-(4-(trifluormethyl)benzyl)oxim;
1-(4-((1-((Tetrahydro-2H-pyran-4-yl)methyl)-1H-tetrazol-5-yl)methoxy)phenyl)ethanon-O-(4-(trifluormethyl)benzyl)oxim;
1-(4-((2-((Tetrahydro-2H-pyran-4-yl)methyl)-2H-tetrazol-5-yl)methoxy)phenyl)ethanon-O-(4-(trifluormethyl)benzyl)oxim;
1-(6-((1-Benzyl-2H-tetrazol-5-yl)methoxy)pyridin-3-yl)ethanon-O-(4-(trifluormethyl)benzyl)oxim;
1-(6-((2-Benzyl-2H-tetrazol-5-yl)methoxy)pyridin-3-yl)ethanon-O-(4-(trifluormethyl)benzyl)oxim;
1-(4-((1,3,4-Oxadiazol-2-yl)methoxy)phenyl)ethanon-O-(4-(trifluormethyl)benzyl)oxim;
1-(6-((5-Methyl-1,3,4-oxadiazol-2-yl)methoxy)pyridin-3-yl)ethanon-O-(4-(trifluormethyl)benzyl)oxim;
1-(4-((2H-1,2,3-Triazol-2-yl)methoxy)phenyl)ethanon-O-(4-(trifluormethyl)benzyl)oxim;
1-(4-((1H-1,2,3-Triazol-1-yl)methoxy)phenyl)ethanon-O-(4-(trifluormethyl)benzyl)oxim;
1-(4-(Thiophen-2-ylmethoxy)phenyl)ethanon-O-(4-(trifluormethyl)benzyl)oxim;
1-(4-(Furan-2-ylmethoxy)phenyl)ethanon-O-(4-(trifluormethyl)benzyl)oxim;
1-(4-(Furan-3-ylmethoxy)phenyl)ethanon-O-(4-(trifluormethyl)benzyl)oxim;
3-((4-(1-(((4-(Trifluormethyl)benzyl)oxy)imino)ethyl)phenoxy)methyl)-1H-1,2,4-triazol-5(4H)-on;
1-(4-((2-Methylthiazol-4-yl)methoxy)phenyl)ethanon-O-(4-(trifluormethyl)benzyl)oxim;
5-((4-(1-(((3-Methoxy-4-(trifluormethyl)benzyl)oxy)imino)ethyl)phenoxy)methyl)oxazolidin-2,4-dion;
1-(4-((1-Trityl-1H-imidazol-4-yl)methoxy)phenyl)ethanon-O-(4-(trifluormethyl)benzyl)oxim;
1-(4-((1H-Imidazol-4-yl)methoxy)phenyl)ethanon-O-(4-(1,1-difluorethyl)benzyl)oxim;
5-((4-(1-(((4-(Trifluormethyl)benzyl)oxy)imino)ethyl)phenoxy)methyl)isoxazol-3(2H)-on;
1-(4-((5-(Morpholinomethyl)isoxazol-3-yl)methoxy)phenyl)ethanon-O-(4-(trifluormethyl)benzyl)oxim;
1-(4-((1H-Benzo[d]imidazol-2-yl)methoxy)phenyl)ethanon-O-(4-(trifluormethyl)benzyl)oxim;
1-((4-(1-(((4-(Trifluormethyl)benzyl)oxy)imino)ethyl)phenoxy)methyl)pyrrolidin-2,5-dion;
1-(((5-(1-(((4-(Trifluormethyl)benzyl)oxy)imino)ethyl)pyridin-2-yl)oxy)methyl)pyrrolidin-2,5-dion;
5-((4-(1-(((4-(Trifluormethyl)benzyl)oxy)imino)ethyl)phenoxy)methyl)thiazolidin-2,4-dion;
5-(((5-(1-(((4-(Trifluormethyl)benzyl)oxy)imino)ethyl)pyridin-2yl)oxy)methyl)thiazolidin-2,4-dion;
2-((4-(1-(((4-(Trifluormethyl)benzyl)oxy)imino)ethyl)phenoxy)methyl)cyclopentan-1,3-dion;
2-(((5-(1-(((4-(Trifluormethyl)benzyl)oxy)imino)ethyl)pyridin-2-yl)oxy)methyl)cyclopentan-1,3-dion;
3-((4-(1-(((4-(Trifluormethyl)benzyl)oxy)imino)ethyl)phenoxy)methyl)pyrrolidin-2,5-dion;
3-(((5-(1-(((4-(Trifluormethyl)benzyl)oxy)immo)ethyl)pyridin-2-yl)oxy)methyl)pyrrolidin-2,5-dion;
1-(4-((4,5-Dihydroisoxazol-3-yl)methoxy)phenyl)ethanon-O-(4-(trifluormethyl)benzyl)oxim;
1-(6-((4,5-Dihydroisoxazol-3-yl)methoxy)pyridin-3-yl)ethanon-O-(4-(trifluormethyl)benzyl)oxim;
1-(4-(Pyrrolidin-2-ylmethoxy)phenyl)ethanon-O-(4-(trifluormethyl)benzyl)oxim;
1-(6-(Pyrrolidin-2-ylmethoxy)pyridin-3-yl)ethanon-O-(4-(trifluormethyl)benzyl)oxim;
4-Hydroxy-5,5-dimethyl-3-((4-(1-(((4-(trifluormethyl)benzyl)oxy)imino)ethyl)phenoxy)methyl)furan-2(5H)-on;
4-Hydroxy-5,5-dimethyl-3-(((5-(1-(((4-(trifluormethyl)benzyl)oxy)imino)ethyl)pyridin-2-yl)oxy)methyl)furan-2(5H)-on;
1-(4-(Pyrrolidin-1-ylmethoxy)phenyl)ethanon-O-(4-(trifluormethyl)benzyl)oxim;
1-(6-(Pyrrolidin-1-ylmethoxy)pyridin-3-yl)ethanon-O-(4-(trifluormethyl)benzyl)oxim;
5-((4-(1-(((4-(Trifluormethyl)benzyl)oxy)imino)ethyl)phenoxy)methyl)dihydrofuran-2(3H)-on;
5-(((5-(1-(((4-(Trifluormethyl)benzyl)oxy)imino)ethyl)pyridin-2-yl)oxy)methyl)dihydrofuran-2(3H)-on.

3. Verbindung nach Anspruch 1 bis 2, die in geeigneter Weise zu einer geeigneten pharmazeutischen Zusammensetzung formuliert ist.

4. Verbindung der Formel (I) nach Anspruch 1 bis 2 oder deren pharmazeutische Zusammensetzung nach Anspruch 3 zur Verwendung bei der Behandlung von Hyperlipidämie oder Dyslipidämie.

5. Pharmazeutische Zusammensetzung, umfassend Verbindungen der Formel (I) nach Anspruch 1 bis 2 in Kombination mit einem oder mehreren pharmazeutischen Wirkstoffen, ausgewählt aus der Gruppe, umfassend Insulin, Insulinderivate und -mimetika, Insulin-Sekretagoga, Insulin-Sensibilisatoren, Biguanidmittel, alpha-Glucosidase-Inhibitoren, insulinotrope Sulfonylharnstoffrezeptorliganden, Meglitinide, GLP-1 (Glucagon-like Peptide-1), GLP-1-Analoga, DPPIV (Dipeptidylpeptidase IV)-Inhibitoren, GPR-119-Aktivatoren, Natrium/Glucose-Cotransporter(SGLT2)-Inhibitoren, PPAR-Modulatoren, PPAR-delta-Agonist von Nicht-Glitazon-Typ, HMG-CoA-Reduktase-Inhibitoren, cholesterinsenkende Arzneimittel, Rennininhibitoren, Antithrombotika und Thrombozytenaggregationshemmer und Antiadiposita oder pharmazeutisch unbedenkliche Salze davon.

6. Pharmazeutische Zusammensetzung nach Anspruch 3 zur Verwendung bei der Behandlung von Dyslipidämie und Hyperlipidämie.

## Revendications

1. Composé de formule (I), ses formes tautomères, ses stéréoisomères, ses sels pharmaceutiquement acceptables, et compositions pharmaceutiques les contenant, dans laquelle 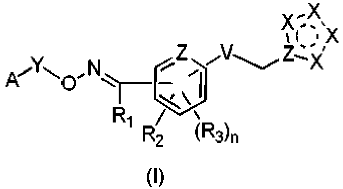
« A » représente un groupe phényle éventuellement substitué ; « Y » représente un groupe (C₁ à C₆)alkyle ; « V » représente un atome O ; « Z » à chaque occurrence représente indépendamment un atome C ou N ; « X » à chaque occurrence représente indépendamment un atome C, N, O ou S substitué ou non substitué, la substitution sur X étant indépendamment choisie parmi un atome d'hydrogène, un groupe hydroxy, oxo, amino, cyano, ou des groupes substitués ou non substitués choisis parmi un groupe alkyle, halogénoalkyle, alcoxy, halogénoalcoxy, cycloalkyle, hétérocyclyle, hétérocyclylalkyle, acyle, un groupe amino monosubstitué ou disubstitué, un groupe acide carboxylique et ses dérivés tels que des groupes ester ou des groupes amide, un groupe hydroxyalkyle, aminoalkyle, alcoxyalkyle, hydroxyamino ; R₁ représente un atome d'hydrogène ou un groupe (C₁ à C₃)alkyle ;
R₂ représente un atome d'hydrogène ou les groupes choisis parmi un groupe (C₁ à C₆)alkyle, (C₁ à C₆)alcoxy, hydroxy ;
R₃ à chaque occurrence représente indépendamment un atome d'hydrogène, un atome d'halogène, un groupe (C₁ à C₃)alkyle, halogéno(C₁ à C₃)alkyle, (C₁ à C₃)alcoxy ;
« n » représente un entier valant de 0 à 3.

2. Composé selon la revendication 1 choisi parmi
1-(4-((1H-tétrazol-5-yl)méthoxy)phényl)éthanone O-(4-(trifluorométhyl)benzyl)oxime ;
1-(4-((2-méthyl-2H-tétrazol-5-yl)méthoxy)phényl)éthanone O-(4-(trifluorométhyl)benzyl)oxime ;
1-(4-((1-méthyl-1H-tétrazol-5-yl)méthoxy)phényl)éthanone O-(4-(trifluorométhyl)benzyl)oxime ;
1-(6-((1H-tétrazol-5-yl)méthoxy)pyridin-3-yl)éthanone O-(4-(trifluorométhyl)benzyl)oxime ;
1-(4-((1H-1,2,4-triazol-1-yl)méthoxy)phényl)éthanone O-(4-(trifluorométhyl)benzyl)oxime ;
1-(4-((1H-1,2,3-triazol-5-yl)méthoxy)phényl)éthanone O-(4-(trifluorométhyl)benzyl)oxime ;
1-(4-((5-méthyl-1,3,4-oxadiazol-2-yl)méthoxy)phényl)éthanone O-(4-(trifluorométhyl)benzyl)oxime ;
1-(4-((5-éthyl-1,3,4-thiadiazol-2-yl)méthoxy)phényl)éthanone O-(4-(trifluorométhyl)benzyl)oxime ;
1-(4-((1,3,4-thiadiazol-2-yl)méthoxy)phényl)éthanone O-(4-(trifluorométhyl)benzyl)oxime ;
3-((4-(1-(((4-(trifluorométhyl)benzyl)oxy)imino)éthyl)phénoxy)méthyl)-1,2,4-oxadiazol-5(4H)-o ne ;
5-((4-(1-(((4-(trifluorométhyl)benzyl)oxy)imino)éthyl)phenoxy)méthyl)oxazolidine-2,4-dione ;
1-(4-((2-(tert-butyl)-5-méthyloxazol-4-yl)méthoxy)phényl)éthanone O-(4-(trifluorométhyl)benzyl)oxime ;
1-(4-((5-(hydroxyméthyl)furan-2-yl)méthoxy)phényl)éthanone O-(4-(trifluorométhyl)benzyl)oxime ;
acide 5-((4-(1-(((4-(trifluorométhyl)benzyl)oxy)imino)éthyl)phénoxy)méthyl)furan-2-carboxylique ;
1-(4-((5-méthyl-4H-1,2,4-triazol-3-yl)méthoxy)phényl)éthanone 0-(4-(trifluorométhyl)benzyl)oxime ;
1-(6-((5-méthyl-4H-1,2,4-triazol-3-yl)méthoxy)pyridin-3-yl)éthanone O-(4-(1,1-difluoroéthyl)benzyl)oxime ;
1-(6-((5-méthyl-4H-1,2,4-triazol-3-yl)méthoxy)pyridin-3-yl)éthanone O-(4-(trifluorométhyl)benzyl)oxime ;
1-(4-((3-amino-1H-1,2,4-triazol-5-yl)méthoxy)phényl)éthanone O-(4-(trifluorométhyl)benzyl)oxime ;
1-(4-((5-amino-1,3,4-oxadiazol-2-yl)méthoxy)phényl)éthanone O-(4-(trifluorométhyl)benzyl)oxime ;
N-(5-((4-(1-(((4-(trifluorométhyl)benzyl)oxy)imino)éthyl)phénoxy)méthyl)-1,3,4-oxadiazol-2-yl )méthanesulfonamide ;
1-(4-((1H-tétrazol-5-yl)méthoxy)phényl)éthanone O-(4-(1,1-difluoroéthyl)benzyl)oxime ;
1-(2-((1H-tetrazol-5-yl)méthoxy)phenyl)ethanone O-(4-(trifluorométhyl)benzyl)oxime ; 1-(4-((1H-tétrazol-5-yl)méthoxy)phényl)éthanone
O-((6-(trifluorométhyl)pyridin-3-yl)méthyl)oxime ; 1-(4-((1H-tétrazol-5-yl)méthoxy)phényl)éthanone
O-(3-méthoxy-4-(trifluorométhyl)benzyl)oxime ; 1-(6-((1H-tétrazol-5-yl)méthoxy)pyridin-3-yl)éthanone
O-(3-méthoxy-4-(trifluorométhyl)benzyl)oxime ; 1-(4-((1H-tétrazol-5-yl)méthoxy)-2-hydroxyphényl)éthanone
O-(4-(trifluorométhyl)benzyl)oxime ; 1-(2-hydroxy-4-((1-méthyl-1H-tétrazol-5-yl)méthoxy)phényl)éthanone 0-(4-(trifluorométhyl)benzyl)oxime ;
1-(4-((1H-tétrazol-5-yl)méthoxy)phényl)éthanone O-benzyloxime ;
1-(4-((1H-tétrazol-5-yl)méthoxy)phényl)éthanone O-(4-fluorobonzyl)oxime ;
1-(6-((1H-tétrazol-5-yl)méthoxy)pyridin-3-yl)éthanone O-(4-fluorobenzyl)oxime ;
1-(6-((1H-tétrazol-5-yl)méthoxy)pyridin-3-yl)éthanone O-(4-éthoxybenzyl)oxime ;
1-(6-((1H-tétrazol-5-yl)méthoxy)pyridin-3-yl)éthanone O-(4-propoxybenzyl)oxime ;
1-(2-((1H-tétrazol-5-yl)méthoxy)-6-chloropyridin-3-yl)éthanone O-(4-(trifluorométhyl)benzyl)oxime ;
1-(6-((1H-tétrazol-5-yl)méthoxy)-2-chloropyridin-3-yl)éthanone O-(4-(trifluorométhyl)benzyl)oxime ;
1-(4-((1H-tétrazol-5-yl)méthoxy)phényl)éthanone O-((4'-(trifluorométhyl)-[1,1'-biphényl]-4-yl)méthyl)oxime ;
1-(4-((1H-tétrazol-5-yl)méthoxy)phényl)éthanone O-(2,4,6-triisopropylbenzyl)oxime ;
1-(4-((1H-tétrazol-5-yl)méthoxy)phényl)éthanone O-(4-(difluorométhyl)benzyl)oxime ;
1-(4-((1-méthyl-1H-tetrazol-5-yl)méthoxy)phényl)éthanone O-(2,4,6-triisopropylbenzyl)oxime ;
1-(4-((2-éthyl-2H-tétrazol-5-yl)méthoxy)phényl)éthanone O-(4-(trifluorométhyl)benzyl)oxime ;
1-(4-((1-éthyl-2H-tétrazol-5-yl)méthoxy)phényl)éthanone O-(4-(trifluorométhyl)benzyl)oxime ;
1-(6-((1H-tétrazol-5-yl)méthoxy)pyridin-3-yl)éthanone O-(4-(1,1-difluoroéthyl)benzyl)oxime ;
1-(6-((2-éthyl-2H-tétrazol-5-yl)méthoxy)pyridin-3-yl)éthanone O-(4-(1,1-difluoroéthyl)benzyl)oxime ;
1 -(6-((1-éthyl-2H-tétrazol-5-yl)méthoxy)pyridin-3 -yl)éthanone O-(4-(1,1-difluoroéthyl)benzyl)oxime ;
1-(6-((2-éthyl-2H-tétrazol-5-yl)méthoxy)pyridin-3-yl)éthanone O-(4-(trifluorométhyl)benzyl)oxime ;
1 -(6-((1-éthyl-2H-tétrazol-5-yl)méthoxy)pyridin-3-yl)éthanone 0-(4-(trifluorométhyl)benzyl)oxime ;
1-(6-((2-isopropyl-2H-tétrazol-5-yl)méthoxy)pyridin-3-yl)éthanone O-(4-(trifluorométhyl)benzyl)oxime ;
1 -(6-((1-isopropyl-2H-tétrazol-5-yl)méthoxy)pyridin-3-yl)éthanone O-(4-(trifluorométhyl)benzyl)oxime ;
1-(6-((2-(cyclopropylméthyl)-2H-tétrazol-5-yl)méthoxy)pyridin-3-yl)éthanone O-(4-(trifluorométhyl)benzyl)oxime ;
1-(6-((1-(cyclopropylméthyl)-2H-tetrazol-5-yl)methoxy)pyridin-3-yl)éthanone O-(4-(trifluorométhyl)benzyl)oxime ;
1-(6-((2-(cyclohexylméthyl)-2H-tétrazol-5-yl)méthoxy)pyridin-3-yl)éthanone 0-(4-(trifluorométhyl)benzyl)oxime ;
1-(6-((1-(cyclohexylméthyl)-2H-tétrazol-5-yl)méthoxy)pyridin-3-yl)éthanone O-(4-(trifluorométhyl)benzyl)oxime;
1-(6-((2-(2-hydroxyéthyl)-2H-tétrazol-5-yl)méthoxy)pyridin-3-yl)éthanone O-(4-(trifluorométhyl)benzyl)oxime;
acide 2-(5-(((5-(1-(((4-(trifluorométhyl)benzyl)oxy)imino)éthyl)pyridin-2-yl)oxy)méthyl)-2H-tétrazol-2-yl)acétique;
acide 2-(5-(((5-(1-(((4-(trifluorométhyl)benzyl)oxy)imino)éthyl)pyridin-2-yl)oxy)méthyl)-1H-tétrazol-1-yl)acétique ;
acide 3-(5-(((5-(1-(((4-(trifluorométhyl)benzyl)oxy)imino)éthyl)pyridin-2-yl)oxy)méthyl)-2H-tétrazol-2-yl)propanoïque;
1-(6-((1H-tétrazol-5-yl)méthoxy)-2-méthoxypyridin-3-yl)éthanone O-(4-(trifluorométhyl)benzyl)oxime;
1-(6-((2-isopropyl-2H-tétrazol-5-yl)méthoxy)pyridin-3-yl)éthanone O-(4-(1,1-difluoroéthyl)benzyl)oxime ;
1--(6-((1-*iso*propyl-2H-tétrazol-5-yl)méthoxy)pyridin-3-yl)éthanone O-(4-(1,1-difluoroéthyl)benzyl)oxime ;
1-(6-((2-(cyclopropylméthyl)-2H-tétrazol-5-yl)méthoxy)pyridin-3-yl)éthanone O-(4-(1,1-difluoroéthyl)benzyl)oxime ;
1-(6-((1-(cyclopropylméthyl)-2H-tétrazol-5-yl)méthoxy)pyridin-3-yl)éthanone O-(4-(1,1-difluoroéthyl)benzyl)oxime ;
1-(6-((2-(2-hydroxyéthyl)-2H-tétrazol-5-yl)méthoxy)pyridin-3-yl)éthanone O-(4-(1,1-difluoroéthyl)benzyl)oxime ;
1-(6-((1-(2-hydroxyéthyl)-2H-tétrazol-5-yl)méthoxy)pyridin-3-yl)éthanone O-(4-(1,1-difluoroéthyl)benzyl)oxime;
1-(4-((2-(2-hydroxyéthyl)-2H-tétrazol-5-yl)méthoxy)phényl)éthanone O-(4-(trifluorométhyl)benzyl)oxime;
2-(5-((4-(1-(((4-(trifluorométhyl)benzyl)oxy)imino)éthyl)phénoxy)méthyl)-2H-tétrazol-2-yl)acét onitrile ;
1-(4-((2-(2-aminoéthyl)-2H-tétrazol-5-yl)méthoxy)phényl)éthanone O-(4-(trifluorométhyl)benzyl)oxime;
1-(4-((1-((tétrahydro-2H-pyran-4-yl)méthyl)-1H-tétrazol-5-yl)méthoxy)phényl)éthanone 0-(4-(trifluorométhyl)benzyl)oxime ;
1-(4-((2-((tétrahydro-2H-pyran-4-yl)méthyl)-2H-tétrazol-5-yl)méthoxy)phényl)éthanone O-(4-(trifluorométhyl)benzyl)oxime;
1-(6-((1-benzyl-2H-tétrazol-5-yl)méthoxy)pyridin-3-yl)éthanone O-(4-(trifluorométhyl)benzyl)oxime;
1-(6-((2-benzyl-2H-tétrazol-5-yl)méthoxy)pyridin-3-yl)éthanone O-(4-(trifluorométhyl)benzyl)oxime;
1--(4-((1,3,4-oxadiazol-2-yl)méthoxy)phényl)éthanone O-(4-(trifluorométhyl)benzyl)oxime;
1-(6-((5-méthyl-1,3,4-oxadiazol-2-yl)méthoxy)pyridin-3-yl)éthanone O-(4-(trifluorométhyl)benzyl)oxime;
1-(4-((2H-1,2,3-triazol-2-yl)méthoxy)phényl)éthanone 0-(4-(trifluorométhyl)benzyl)oxime ;
1-(4-((1H-1,2,3-triazol-1-yl)méthoxy)phényl)éthanone O-(4-(trifluorométhyl)benzyl)oxime;
1-(4-(thiophén-2-ylméthoxy)phényl)éthanone O-(4-(trifluorométhyl)benzyl)oxime ;
1-(4-(furan-2-ylméthoxy)phényl)éthanone O-(4-(trifluorométhyl)benzyl)oxime;
1-(4-(furan-3-ylméthoxy)phényl)éthanone O-(4-(trifluorométhyl)benzyl)oxime;
3-((4-(1-(((4-(trifluorométhyl)benzyl)oxy)imino)éthyl)phénoxy)méthyl)-1H-1,2,4-triazol-5(4H)-one ;
1-(4-((2-méthylthiazol-4-yl)méthoxy)phényl)éthanone O-(4-(trifluorométhyl)benzyl)oxime;
5-((4-(1-(((3-méthoxy-4-(trifluorométhyl)benzyl)oxy)imino)éthyl)phénoxy)méthyl)oxazolidine-2,4-dione ;
1 -(4-((1-trityl-1H-imidazol-4-yl)méthoxy)phényl)éthanone O-(4-(trifluorométhyl)benzyl)oxime;
1-(4-((1H-imidazol-4-yl)méthoxy)phényl)éthanone O-(4-(1,1-difluoroéthyl)benzyl)oxime;
5-((4-(1-(((4-(trifluorométhyl)benzyl)oxy)imino)éthyl)phénoxy)méthyl)isoxazol-3(2H)-one ;
1-(4-((5-(morpholinométhyl)isoxazol-3-yl)méthoxy)phényl)éthanone O-(4-(trifluorométhyl)benzyl)oxime;
1-(4-((1H-benzo[d]imidazol-2-yl)méthoxy)phényl)éthanone O-(4-(trifluorométhyl)benzyl)oxime;
1-((4-(1-(((4-(trifluorométhyl)benzyl)oxy)imino)éthyl)phénoxy)méthyl)pyrrolidine-2,5-dione ;
1-(((5-(1-(((4-(trifluorométhyl)benzyl)oxy)imino)éthyl)pyridin-2-yl)oxy)méthyl)pyrrolidine-2,5-dione ;
5-((4-(1-(((4-(trifluorométhyl)benzyl)oxy)imino)éthyl)phénoxy)méthyl)thiazolidine-2,4-dione ;
5-(((5-(1-(((4-(trifluorométhyl)benzyl)oxy)imino)éthyl)pyridin-2-yl)oxy)méthyl)thiazolidine-2,4 -dione ;
2-((4-(1-(((4-(trifluorométhyl)benzyl)oxy)imino)éthyl)phénoxy)méthyl)cyclopentane-1,3-dione ;
2-(((5-(1-(((4-(trifluorométhyl)benzyl)oxy)imino)éthyl)pyridin-2 yl)oxy)méthyl)cyclopentane-1, 3-dione ;
3-((4-(1-(((4-(trifluorométhyl)benzyl)oxy)imino)éthyl)phénoxy)méthyl)pyrrolidine-2,5-dione ;
3-(((5-(1-(((4-(trifluorométhyl)benzyl)oxy)imino)éthyl)pyridin-2-yl)oxy)méthyl)pyrrolidine-2,5-dione ;
1-(4-((4,5-dihydroisoxazol-3-yl)méthoxy)phényl)éthanone O-(4-(trifluorométhyl)benzyl)oxime;
1-(6-((4,5-dihydroisoxazol-3-yl)méthoxy)pyridin-3-yl)éthanone O-(4-(trifluorométhyl)benzyl)oxime;
1-(4-(pyrrolidin-2-ylméthoxy)phényl)éthanone 0-(4-(trifluorométhyl)benzyl)oxime ;
1-(6-(pyrrolidin-2-ylméthoxy)pyridin-3-yl)éthanone O-(4-(trifluorométhyl)benzyl)oxime ;
4-hydroxy-5,5-diméthyl-3-((4-(1-(((4-(trifluorométhyl)benzyl)oxy)imino)éthyl)phénoxy)méthyl) furan-2(5H)-one ;
4-hydroxy-5,5-diméthyl-3-(((5-(1-(((4-(trifluorométhyl)benzyl)oxy)imino)éthyl)pyridin-2-yl)oxy )méthyl)furan-2(5H)-one ;
1-(4-(pyrrolidin-1-ylméthoxy)phényl)éthanone 0-(4-(trifluorométhyl)benzyl)oxime ;
1-(6-(pyrrolidin-1-ylméthoxy)pyridin-3-yl)éthanone O-(4-(trifluorométhyl)benzyl)oxime ;
5-((4-(1-(((4-(trifhrorométhyl)benzyl)oxy)imino)éthyl)phénoxy)méthyl)dihydrofuran-2(3H)-one ;
5-(((5-(1-(((4-(trifluorométhyl)benzyl)oxy)imino)éthyl)pyridin-2-yl)oxy)méthyl)dihydrofuran-2( 3H)-one.

3. Composé selon les revendications 1 à 2 formulé de manière appropriée dans une composition pharmaceutique acceptable.

4. Composé de formule (I) selon les revendications 1 à 2 ou sa composition pharmaceutique selon la revendication 3 pour utilisation dans le traitement de l'hyperlipidémie ou de la dyslipidémie.

5. Composition pharmaceutique comprenant des composés de formule (I) selon les revendications 1 à 2 en combinaison avec un ou plusieurs agents pharmaceutiquement actifs choisis dans le groupe comprenant l'insuline, les dérivés et mimétiques de l'insuline, les sécrétagogues d'insuline, les sensibilisants à l'insuline, les agents biguanides, les inhibiteurs de l'alpha-glucosidase, les ligands de récepteurs de sulfonylurée insulinotropes, les méglitinides, le GLP-1 (peptide-1 analogue au glucagon), les analogues de GLP-1, les inhibiteurs de la DPPIV (dipeptidylpeptidase IV), les activateurs de GPR-119, les inhibiteurs du co-transporteur de glucose dépendant du sodium (SGLT2), les modulateurs de PPAR, un agoniste PPAR-delta non du type glitazone, les inhibiteurs de la HMG-CoA réductase, les médicaments hypocholestérolémiants, les inhibiteurs de la rénine, les agents antithrombotiques et anti-plaquettes et les agents anti-obésité ou les sels pharmaceutiquement acceptables de ceux-ci.

6. Composition pharmaceutique selon la revendication 3 pour utilisation dans le traitement de la dyslipidémie ou de l'hyperlipidémie.
